(19) Europäisches Patentamt
European Patent Office
Office européen des brevets

(11) EP 4 331 612 A1

(12) **EUROPEAN PATENT APPLICATION**
published in accordance with Art. 153(4) EPC

(43) Date of publication:
**06.03.2024 Bulletin 2024/10**

(21) Application number: **22794964.1**

(22) Date of filing: **28.04.2022**

(51) International Patent Classification (IPC):
***A61K 47/68*** *(2017.01)* ***A61K 47/54*** *(2017.01)*
***C07K 5/062*** *(2006.01)* ***A61P 35/00*** *(2006.01)*

(52) Cooperative Patent Classification (CPC):
**A61K 47/54; A61K 47/68; A61P 35/00; C07K 5/06017**

(86) International application number:
**PCT/CN2022/089725**

(87) International publication number:
**WO 2022/228494 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.04.2021 CN 202110475315**

(71) Applicant: **Hyslink Therapeutics**
**Shanghai 201499 (CN)**

(72) Inventors:
- **ZHANG, Hui**
**Shanghai 201499 (CN)**
- **MENG, Xun**
**Shanghai 201499 (CN)**

(74) Representative: **Weickmann & Weickmann PartmbB**
**Postfach 860 820**
**81635 München (DE)**

Remarks:
The complete document including Reference Table(s) and the Sequence Listing(s) can be downloaded from the EPO website

# (54) PREPARATION METHOD FOR AND APPLICATION OF ANTIBODY CONJUGATED DRUG

(57) The present application relates to a preparation method for and an application of an antibody conjugated drug, in particular to a compound, or a tautomer, mesomer, racemate, enantiomer, diastereomer, or mixture thereof, or a pharmaceutically acceptable salt, prodrug, or solvate thereof, preparation methods of the compound and related antibody conjugated drug, and uses thereof in the preparation of drugs for treating cancers.

EP 4 331 612 A1

## Description

### FIELD OF THE INVENTION

The present application relates to the field of biomedicine, in particular to a method for preparing an antibody drug conjugate and an application thereof.

### BACKGROUND OF THE INVENTION

As a novel targeted therapy drug, an antibody drug conjugate (ADC) delivers a drug to the diseased site through an appropriate carrier. However, due to the constraints of the technology of antibodies and highly active cytotoxic drugs, a limited number of antibody drug conjugates have been approved for marketing, and the application of antibody drug conjugates in the field of tumor therapy is in urgent need of rapid development.

Cytotoxins are essential for ADC drugs to play a role. The application of camptothecins is very promising, and the marketed ADC drugs Trodelvy and Enhertu use camptothecins SN38 and DX-8951f as warhead molecules, respectively. However, all the ADCs finally prepared by camptothecins have greatly changed the properties of monoclonal antibodies, with a large degree of decay in stability and half-life. Although in the DESTINY-Breast03 clinical study, the confirmed objective response rate (ORR) of Enhertu reached 79.7%, according to the DS8201-A-J101 clinical study, for breast cancer with low HER2 expression, the confirmed objective response rate (ORRs) of Enhertu was 37.0%, and the treatment efficiency was significantly lower than that of breast cancer with high or medium expression. In addition, ADC drugs showed new symptoms of toxic side effects, for example, the drug Enhertu showed side effects such as pneumonia and interstitial lung disease. WO 2020233174 A1 discloses a class of camptothecin compounds containing a valine-citrulline (Val-Cit)-PAB linker. However, this molecule cannot be coupled with an antibody to obtain a qualified ADC molecule (the aggregate content in a qualified ADC product needs to be less than 5%). Therefore, there is an urgent need in the field to provide a more suitable antibody drug conjugate based on camptothecins (such as exatecan, belotecan, etc.) to achieve efficient, simple and practical chemical preparation and conjugation, and improve the pharmaceutical properties, metabolic properties, efficacy and safety of the existing antibody drug conjugate, such as improving the stability of ADC molecules, improving the therapeutic window, or the like.

### SUMMARY OF THE INVENTION

The present application provides an antibody drug conjugate, an intermediate, a preparation method and an application thereof. The antibody drug conjugate of this application can realize the wide application of cytotoxic drugs in the field of ADC to treat tumor diseases. The main technical effect of this application is that the provided novel linker can conjugate highly hydrophobic anti-tumor drugs, such as exatecan, belotecan, and other topoisomerase inhibitors, with antibodies through specific chemical methods, to obtain a conjugate with high hydrophilicity and stability. Compared with traditional ADC linkers, the linker provided in the present application is less likely to produce aggregates in the antibody conjugate when the drug load is high; compared with ADC drugs of the same type, the released toxin molecules in the present application are the intact form of the topoisomerase inhibitors exatecan and belotecan. It is shown by testing that the drug molecules have better biological activities, safety, and other drug-related properties than the drug derivatives released by ADC drugs of the same type. Therefore, the present application can improve the in vivo half-life of the drug and the concentration of the drug in tumor tissues, thereby improving the anti-tumor activity of the drug and/or improving the overall therapeutic window.

In one aspect, the present application provides a compound, or a tautomer, mesomer, racemate, enantiomer, and diastereomer thereof, or a mixture thereof, or a pharmaceutically acceptable salt, prodrug or solvate thereof, wherein the compound comprises a structure represented by formula (C-HER2):

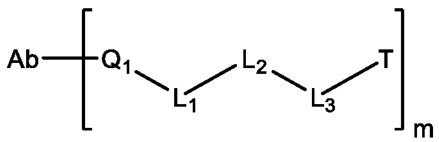

(C-HER2)

wherein, $Q_1$ comprises a linker,

$L_1$ comprises $-L_{1a}-C(=O)-$,

wherein, $L_{1a}$ is selected from the group consisting of optionally substituted alkylene groups, optionally substituted

polyethylene glycol groups, optionally substituted alkenylene groups, optionally substituted alkynylene groups, optionally substituted aliphatic cyclylene groups, optionally substituted aliphatic heterocyclylene groups, optionally substituted arylene groups, and optionally substituted heteroarylene groups;

$L_2$ comprises an optionally substituted polypeptide residue,

$L_3$ comprises an optionally substituted spacer group,

wherein, $L_2$ and/or $L_3$ comprises optionally substituted polysarcosine residues,

T comprises a drug unit,

Ab is a ligand capable of binding to HER2, and m is a number from 1 to 8.

In another aspect, the present application provides a compound, or a tautomer, mesomer, racemate, enantiomer, and diastereomer thereof, or a mixture thereof, or a pharmaceutically acceptable salt, prodrug or solvate thereof, wherein, the compound comprises a structure selected from the group consisting of

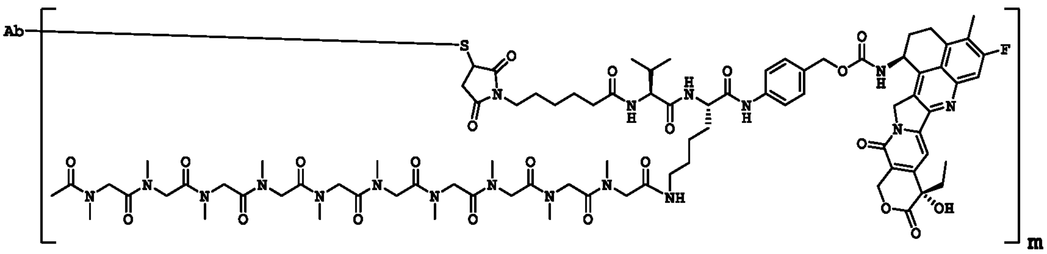

(C-HER2-1),

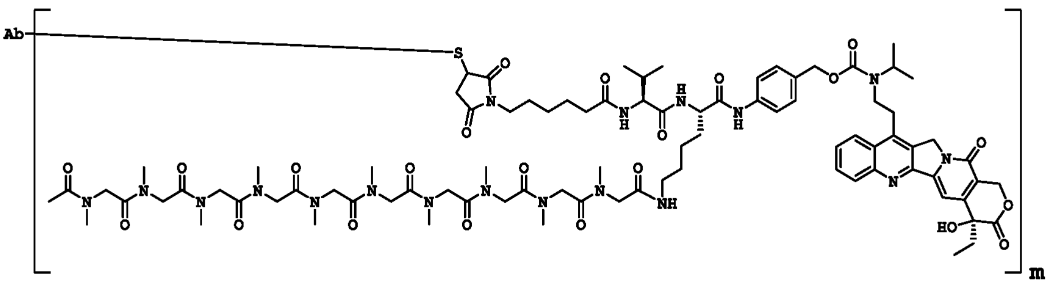

(C-HER2-2),

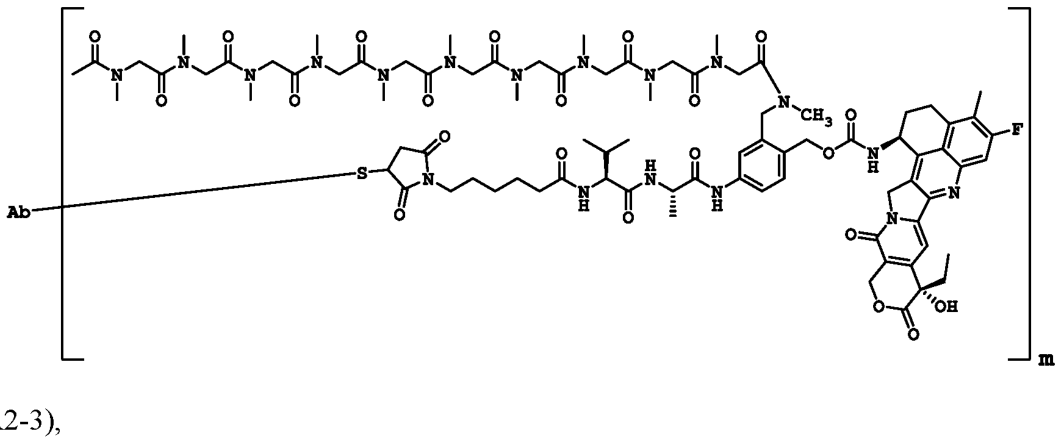

(C-HER2-3),

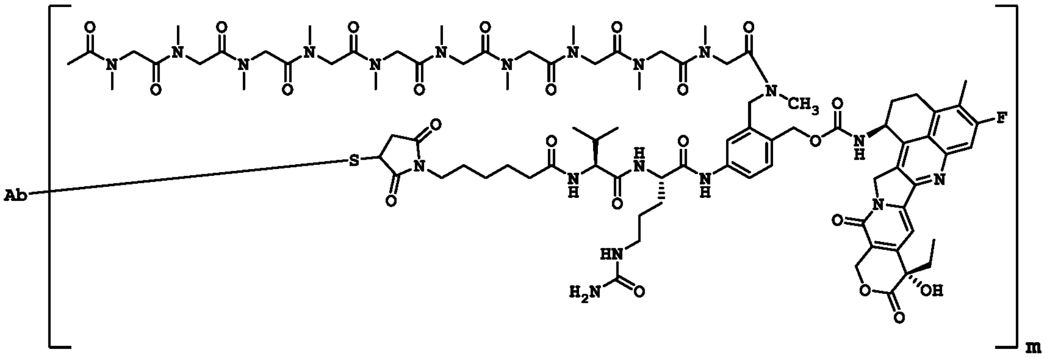

(C-HER2-4),

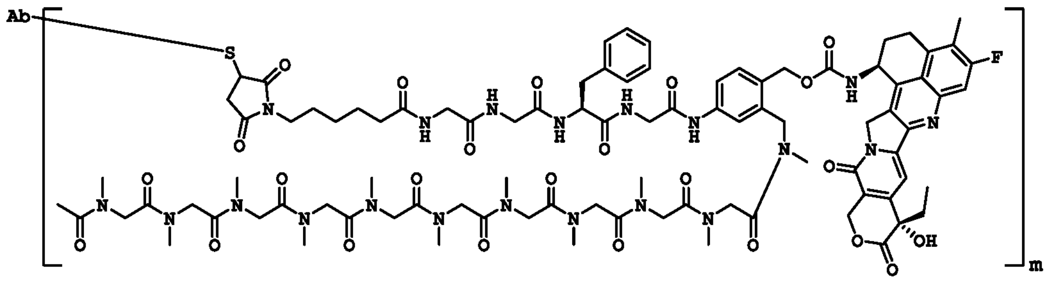

(C-HER2-5),

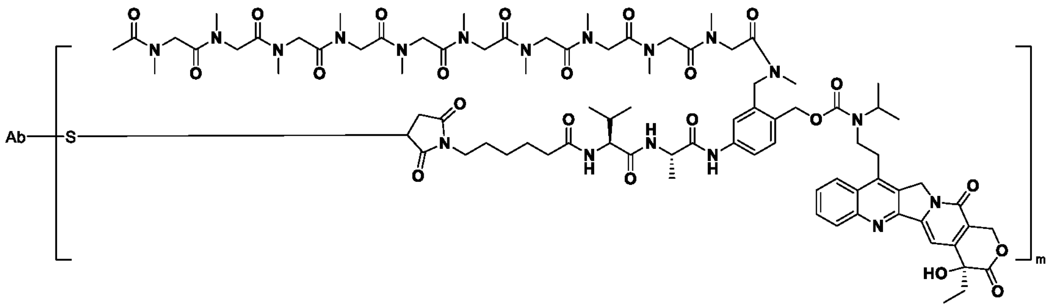

(C-HER2-6),

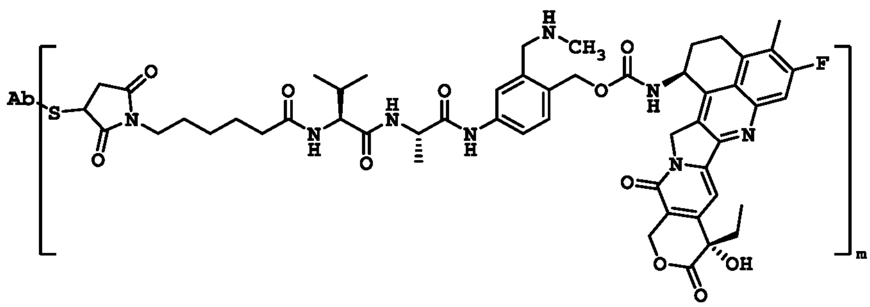

(C-HER2-7),

and

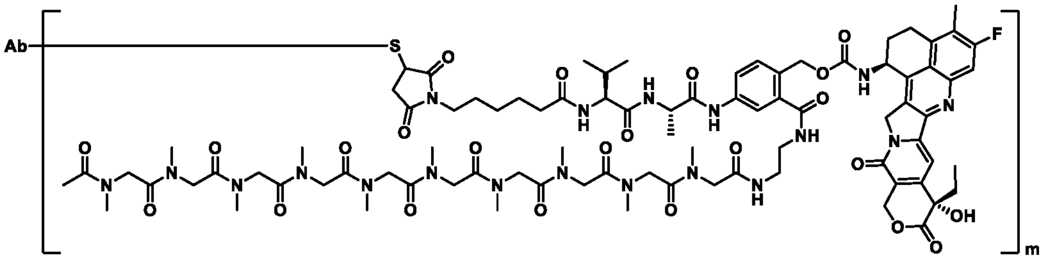 (C-HER2-8),

Ab is a ligand capable of binding to HER2, and m is a number from 1 to 8.

In another aspect, the present application provides a pharmaceutical composition comprising the compound of any one of this application, or a tautomer, mesomer, racemate, enantiomer, and diastereomer thereof, or a mixture thereof, or a pharmaceutically acceptable salt, prodrug or solvate thereof, and optionally a pharmaceutically acceptable carrier.

In another aspect, the present application provides use of the compound of any one of this application, or a tautomer, mesomer, racemate, enantiomer, and diastereomer thereof, or a mixture thereof, or a pharmaceutically acceptable salt, prodrug or solvate thereof, and/or the pharmaceutical composition of this application in the preparation of a drug for treating and/or preventing a tumor.

In another aspect, the present application provides an antibody-drug conjugate, which is formed by conjugating the compound of this application with an antibody.

In one embodiment, there are one or more drug components covalently attached to the conjugate of this application.

In one embodiment, the antibodies and drugs in this application are conjugated covalently (e.g., they can be covalently attached to a linker, respectively).

In another aspect, the present application provides a method for preparing an antibody-drug conjugate by reducing the disulfide chains in the hinge region of the antibody or a fragment thereof to generate a pair of cysteine residues, and subjecting the mercapto group in the cysteine residues to a substitution reaction with the linking group of the compound in this application, such as maleimide group, so as to link the compound of this application to the cysteine mercapto group of the antibody or the fragment thereof, thus obtaining the antibody-drug conjugate, wherein the drug-to-antibody ratio DAR (e.g., m in the present application) is controlled according to the reaction conditions, for example, it is usually between 2 and 8.

In another aspect, m in the present application represents the molar ratio of cytotoxic drug molecules to Ab (also known as DAR, i.e., drug-to-antibody ratio), which can be an integer or a decimal number, and can be understood as: the average value of molar ratios of the drug molecules to the monoclonal antibody molecules in the antibody drug conjugate obtained after conjugating a single monoclonal antibody molecule with a cytotoxic drug. It can be generally determined by Hydrophobic-Interaction Chromatography (HIC), Reverse phase HPLC (RP-HPLC), polyacrylamide-SDS gel electrophoresis (SDS PAGE, electrophoresis), liquid chromatograph-mass spectrometer (LC-MS), ultraviolet/visible spectrometry (UV/Vis), or the like.

In another aspect, the present application provides a method for preparing the compound of this application, comprising the steps of:

contacting an amino acid active ester having an amino protecting group N1 with an amino acid to obtain an intermediate M1; contacting the intermediate M1 with substituted/unsubstituted p-aminobenzyl alcohol in the presence of a condensing agent such as EEDQ (2-ethoxy-1-ethoxycarbonyl-1,2-dihydroquinoline) to obtain an intermediate M2; removing the N1 of the intermediate M2 to obtain an intermediate M3; contacting the intermediate M3 with a compound containing an maleimide group to obtain an intermediate M4; contacting the intermediate M4 with bis(4-nitrophenyl)carbonate to obtain an intermediate M5; and contacting the intermediate M5 with a drug unit.

In one embodiment, the N1 comprises fluorenylmethoxycarbonyl.

In one embodiment, when preparing the compound in which the $L_2$ is substituted with a structure comprising a polysarcosine residue, the intermediate comprising the amino protecting group N2 is contacted with trifluoroacetic acid, followed by contacting with acetylated polysarcosine.

In one embodiment, the N2 comprises tert-butoxycarbonyl.

In another aspect, the present application provides a method for preparing the compound of this application, comprising the steps of contacting a ligand with the compound of this application under a condition suitable for forming a bond between the ligand and the compound.

In one embodiment, the ligand is contacted with the compound of this application in a mixture of a buffer and an organic solvent.

In one embodiment, the ligand is contacted with the compound of this application at about 0 to about 37°C.

In one embodiment, the following step is included before contacting the ligand with the compound of this application: reacting the ligand with a reducing agent in a buffer to obtain a reduced ligand.

In one embodiment, a step of removing the reducing agent is included after obtaining the reduced ligand, and before contacting the ligand with the compound of this application.

In one embodiment, the removing the reducing agent includes subjecting the reaction product to a desalting column and/or ultrafiltration.

In one embodiment, the reducing agent is selected from the group consisting of tris(2-carboxyethyl)phosphine hydrochloride (TCEP), beta-mercaptoethanol, beta-mercaptoethylamine hydrochloride, and dithiothreitol (DTT).

In one embodiment, the buffer is selected from the group consisting of potassium dihydrogen phosphate-sodium hydroxide ($KH_2PO_4$-NaOH)/sodium chloride (NaCl)/diethyltriaminepentaacetic acid (DTPA) buffer, disodium hydrogen phosphate-citric acid/sodium chloride (NaCl)/diethyltriaminepentaacetic acid (DTPA), boric acid-borax/sodium chloride (NaCl)/diethyltriaminepentaacetic acid (DTPA), histidine-sodium hydroxide/sodium chloride (NaCl)/diethyltriaminepentaacetic acid (DTPA) and PBS/diethyltriaminepentaacetic acid (DTPA).

In one embodiment, the organic solvent is selected from the group consisting of acetonitrile (ACN), dimethylformamide (DMF), dimethylacetamide (DMA) and dimethylsulfoxide (DMSO).

In one embodiment, the volume ratio of the organic solvent in the mixture of the buffer and the organic solvent does not exceed 30%.

It is found in the present application that, compared with traditional antibody-drug conjugates, in the antibody-drug conjugate in this application, due to the excessive hydrophobicity of exatecan, belotecan and related drugs, the introduction of polysarcosine can greatly increase the hydrophilicity of the conjugate, thereby making the obtained antibody-drug conjugate more stable and less prone to form aggregation as a whole. In addition, the present application provides carbamate, which can undergo rapid 1,6-elimination after enzyme digestion to release the drug and has better stability and biological activity in vitro and in vivo. Based on the above findings, the present application has been completed.

Other aspects and advantages of the present application can be readily perceived by those skilled in the art from the following detailed description. In the following detailed description, only exemplary embodiments of the present application are shown and described. As will be recognized by those skilled in the art, the content of the present application enables those skilled in the art to make changes to the disclosed specific embodiments without departing from the spirit and scope of the invention involved in the present application. Correspondingly, the drawings and description in the specification of the present application are merely exemplary, rather than restrictive.

## BRIEF DESCRIPTION OF THE DRAWING

The specific features of the invention involved in the present application are as shown in the appended claims. The characteristics and advantages of the invention involved in the present application can be better understood by referring to the exemplary embodiments described in detail below and the accompanying drawings. A brief description of the drawings is as below:

FIG. 1 shows the size exclusion chromatography (SEC-HPLC) chromatogram of the antibody conjugate 1 of this application;

FIG. 2 shows the size exclusion chromatography (SEC-HPLC) chromatogram of the antibody conjugate 2 of this application;

FIG. 3 shows the hydrophobic interaction high performance liquid chromatogram (HIC-HPLC) of the antibody conjugate 2 of this application;

FIG. 4 shows the size exclusion chromatography (SEC-HPLC) chromatogram of the antibody conjugate 3 of this application;

FIG. 5 shows the size exclusion chromatography (SEC-HPLC) chromatogram of the antibody conjugate 4 of this application;

FIG. 6 shows the hydrophobic interaction high performance liquid chromatogram (HIC-HPLC) of the antibody conjugate 4 of this application;

FIG. 7 shows the size exclusion chromatography (SEC-HPLC) chromatogram of the antibody conjugate 5 of this application;

FIG. 8 shows the hydrophobic interaction high performance liquid chromatogram (HIC-HPLC) of the antibody conjugate 5 of this application;

FIG. 9 shows the size exclusion chromatography (SEC-HPLC) chromatogram of the antibody conjugate 6 of this application;

FIG. 10 shows the hydrophobic interaction high performance liquid chromatogram (HIC-HPLC) of the antibody conjugate 6 of this application;

FIG. 11 shows the size exclusion chromatography (SEC-HPLC) chromatogram of the antibody conjugate 7 of this application;

FIG. 12 shows the hydrophobic interaction high performance liquid chromatogram (HIC-HPLC) of the antibody conjugate 7 of this application;

FIG. 13 shows the size exclusion chromatography (SEC-HPLC) chromatogram of the antibody conjugate 8 of this application;

FIG. 14 shows the hydrophobic interaction high performance liquid chromatogram (HIC-HPLC) of the antibody conjugate 8 of this application;

FIG. 15 shows the size exclusion chromatography (SEC-HPLC) chromatogram of the antibody conjugate 9 of this application;

FIG. 16 shows the hydrophobic interaction high performance liquid chromatogram (HIC-HPLC) of the antibody conjugate 9 of this application;

FIG. 17 shows the size exclusion chromatography (SEC-HPLC) chromatogram of the antibody conjugate 10 of this application;

FIG. 18 shows the hydrophobic interaction high performance liquid chromatogram (HIC-HPLC) of the antibody conjugate 10 of this application;

FIG. 19 shows the size exclusion chromatography (SEC-HPLC) chromatogram of the antibody conjugate 11 of this application;

FIG. 20 shows the hydrophobic interaction high performance liquid chromatogram (HIC-HPLC) of the antibody conjugate 11 of this application;

FIG. 21 shows the size exclusion chromatography (SEC-HPLC) chromatogram of the antibody conjugate 12 of this application;

FIG. 22 shows the hydrophobic interaction high performance liquid chromatogram (HIC-HPLC) of the antibody conjugate 12 of this application;

FIG. 23 shows a graph of the concentration change in the accelerated stability experiment of some antibody conjugates of this application;

FIG. 24 shows a graph of the increase of aggregates in the accelerated stability experiment of some antibody conjugates of this application;

FIG. 25 shows the activity testing results of some antibody conjugates of this application on inhibiting the proliferation of NCI-N87 cells (human gastric cancer cells) in vitro;

FIG. 26 shows the activity testing results of some antibody conjugates of this application on inhibiting the proliferation of OV-CAR3 cells (human ovarian adenocarcinoma cells) in vitro;

FIG. 27 shows the activity testing results of some antibody conjugates of this application on inhibiting the proliferation

of NCI-N87 cells (human gastric cancer cells) in vitro;

FIG. 28 shows the activity testing results of some antibody conjugates of this application on inhibiting the proliferation of SK-BR-3 cells (human breast cancer cells) in vitro;

FIG. 29 shows the in vivo efficacy results of some anti-HER2 antibody conjugates of this application in COLO205 human colon cancer;

FIG. 30 shows the in vivo efficacy results of some anti-HER2 antibody conjugates of this application in HCC1954 human breast cancer;

FIG. 31 shows the size exclusion chromatography (SEC-HPLC) chromatogram of the antibody conjugate 13 of this application;

FIG. 32 shows the size exclusion chromatography (SEC-HPLC) chromatogram of the antibody conjugate 14 of this application;

FIG. 33 shows the size exclusion chromatography (SEC-HPLC) chromatogram of the antibody conjugate 15 of this application;

FIG. 34 shows the mass spectrum of Trastuzumab naked antibody after reduction;

FIG. 35 shows the mass spectra corresponding to the light and heavy chains of the antibody conjugate 5 of this application after reduction;

FIG. 36 shows the mass spectra corresponding to the light and heavy chains of the antibody conjugate 6 of this application after reduction.

## DETAILED DESCRIPTION

The implementation of the present application will be illustrated below by specific examples, and other advantages and effects of the present application will be easily known by those familiar with the art from the disclosure of the specification.

## Definition of terms

In the present application, the term "ligand" generally refers to a macromolecular compound capable of recognizing and binding to an antigen or receptor associated with a target cell. The role of a ligand can be to present a drug to a target cell population that binds to the ligand, and the ligand includes but not limited to protein hormones, lectin, growth factors, antibodies, or other molecules capable of binding to cells, receptors and/or antigens. In the present application, the ligand can be represented as Ab, and the antigen of a ligand forms a linker bond with a linking unit through a heteroatom on the ligand. The ligand can be an antibody or an antigen-binding fragment thereof, and the antibody can be selected from chimeric antibodies, humanized antibodies, fully human antibodies, or murine antibodies; and the antibody can be a monoclonal antibody. For example, the antibody can be an antibody or an antigen-binding fragment thereof targeting a target selected from the group consisting of HER2.

In the present application, the term "alkyl" generally refers to a residue derived by removing a hydrogen atom from an alkane. Alkyl can be substituted or unsubstituted, replaced or unreplaced. The term "alkyl" generally refers to a saturated linear or branched aliphatic hydrocarbon group having a residue derived by removing a hydrogen atom from the same carbon atom or two different carbon atoms of the parent alkane. It can be a linear or branched group comprising from 1 to 20 carbon atoms, for example alkyl containing from 1 to 12 carbon atoms, for example containing from 1 to 6 carbon atoms. Non-limiting examples of alkyl include, but are not limited to, methyl, ethyl, propyl, propyl, butyl, etc. Alkyl can be substituted or unsubstituted, replaced or unreplaced. For example, when alkyl is substituted, the substituent can be substituted at any available point of attachment, and the substituent can be independently and optionally substituted with one or more substituents selected from alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo. For example, the substituent can be hydrogen, protium, deuterium, tritium, halogen, $-NO_2$, -CN, -OH, -SH, $-NH_2$, -C(O)H, $-CO_2H$, -C(O)C(O)H, $-C(O)CH_2C(O)H$, - S(O)H, $-S(O)_2H$, $-C(O)NH_2$, $-SO_2NH_2$, -OC(O)H, $-N(H)SO_2H$, or a $C_{1-6}$ aliphatic group.

In the present application, the term "alkylene" generally refers to a saturated linear or branched aliphatic hydrocarbon group having two residues derived by removing two hydrogen atoms from the same carbon atom or two different carbon atoms of the parent alkane. It can be a linear or branched group comprising from 1 to 20 carbon atoms. For example, the term "methylene" can refer to a residue derived by removing two hydrogen atoms from a group of 1 carbon atom. Methylene can be substituted or unsubstituted, replaced or unreplaced; for example, alkylene containing from 1 to 12 carbon atoms, for example, containing from 1 to 6 carbon atoms. Non-limiting examples of alkylene include, but are not limited to, methylene ($-CH_2-$), 1,1-ethylene ($-CH(CH_3)-$), 1,2-ethylene ($-CH_2CH_2$)-, 1,1-propylene ($-CH(CH_2CH_3)-$), 1,2-propylene ($-CH_2CH(CH_3)-$), 1,3-propylene ($-CH_2CH_2CH_2-$), 1,4- butylene ($-CH_2CH_2CH_2CH_2-$) and 1,5-butylene ($-CH_2CH_2CH_2CH_2CH_2-$), etc. Alkylene can be substituted or unsubstituted, replaced or unreplaced. For example, when alkylene is substituted, the substituent can be substituted at any available point of attachment, and the substituent can be independently and optionally substituted with one or more substituents selected from alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo. For example, the substituent can be hydrogen, protium, deuterium, tritium, halogen, $-NO_2$, -CN, -OH, - SH, $-NH_2$, -C(O)H, $-CO_2H$, -C(O)C(O)H, $-C(O)CH_2C(O)H$, -S(O)H, $-S(O)_2H$, $-C(O)NH_2$, $-SO_2NH_2$, -OC(O)H, $-N(H)SO_2H$, or a $C_{1-6}$ aliphatic group. Methylene or alkylene can be substituted or unsubstituted.

In the present application, the term "alkenyl" generally refers to a linear or branched hydrocarbon group containing one or more double bonds. Exemplary examples of alkenyl include allyl, homoallyl, vinyl, crotyl, butenyl, pentenyl, hexenyl, etc. Exemplary examples of $C_{2-6}$ alkenyl having more than one double bond include butadienyl, pentadienyl, hexadienyl, and hexatrienyl and branched forms thereof. The position of the unsaturated bond (double bond) can be at any position in the carbon chain. Alkenyl can be substituted or unsubstituted.

In the present application, the term "alkenylene" generally refers to a residue derived by removing two hydrogen atoms from the carbon atoms of an alkene. For example, it can be allylene, vinylene, butenylene, pentenylene, hexenylene, etc. Alkenylene can be substituted or unsubstituted.

In the present application, the term "alkynyl" generally refers to an unsaturated linear or branched alkynyl, e.g., ethynyl, 1-propynyl, propargyl, butynyl, etc. Alkynyl can be substituted or unsubstituted.

In the present application, the term "alkynylene" generally refers to a residue derived by removing two hydrogen atoms from the carbon atoms of an alkyne. For example, it can be ethynylene, propynylene, propargylene, butynylene, etc. Alkynylene can be substituted or unsubstituted.

In the present application, the term "aryl" generally refers to a residue derived by removing one hydrogen atom from an aromatic ring. The term "aromatic ring" can refer to a 6- to 14-membered all-carbon monocyclic or fused polycyclic ring (i.e., rings that share adjacent pairs of carbon atoms) having a conjugated $\pi$ electron system, and can be 6 to 10 membered, e.g., benzene and naphthalene. The aromatic ring can be fused to a heteroaryl, heterocyclyl or cycloalkyl ring, wherein the ring attached to the parent structure is an aryl ring. Aryl can be substituted or unsubstituted. When aryl is substituted, the substituent can be one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, and heterocycloalkylthio. Aryl can be substituted or unsubstituted.

In the present application, the term "arylene" generally refers to a residue derived by removing two hydrogen atoms from carbon atoms of an aromatic ring. For example, it can be phenylene and naphthylene. Arylene can be substituted or unsubstituted.

In the present application, the term "heteroaryl" generally refers to a residue derived by removing one hydrogen atom from a carbon atom of a heteroaromatic ring. The term "heteroaromatic ring" refers to a heteroaromatic system comprising 1 to 4 heteroatoms and 5 to 14 ring atoms, wherein the heteroatoms may be selected from the group consisting of oxygen, sulfur and nitrogen. Heteroaryl can be 5 to 10 membered, or it can be 5 membered or 6 membered, e.g., furanyl, thienyl, pyridyl, pyrrolyl, N-alkylpyrrolyl, pyrimidinyl, pyrazinyl, imidazolyl, tetrazolyl, etc. The heteroaryl ring can be fused to an aryl, heterocyclyl or cycloalkyl ring, wherein the ring attached to the parent structure is a heteroaryl ring. Heteroaryl can be optionally substituted or unsubstituted. When heteroaryl is substituted, the substituent can be one or more groups independently selected from the group consisting of alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, and heterocycloalkylthio. Heteroaryl can be substituted or unsubstituted.

In the present application, the term "heteroarylene" generally refers to a residue derived by removing two hydrogen atoms from carbon atoms of a heteroaromatic ring. For example, it can be furanylene, thienylene, pyridylene, pyrrolylene, pyrimidinylene, pyrazinylene, imidazolylene, tetrazolylene, etc. Heteroarylene can be substituted or unsubstituted.

In the present application, the term "aliphatic cyclyl" generally refers to a residue derived by removing hydrogen atoms from the same carbon atom or multiple different carbon atoms of an aliphatic ring. The term "cycloalkane" generally refers to a saturated or partially unsaturated monocyclic or polycyclic cyclic hydrocarbon. A carbon ring comprises 3 to

20 carbon atoms, and it may comprise 3 to 12 carbon atoms, 3 to 10 carbon atoms, or 3 to 8 carbon atoms. Non-limiting examples of aliphatic cyclyl include cyclopropanyl, cyclobutanyl, cyclopentanyl, cyclopentenyl, cyclohexanyl, cyclohexenyl, cyclohexadienyl, cycloheptanyl, cycloheptatrienyl, cyclooctanyl, etc.; and a polycyclic carbon ring can include spiro, fused, and bridged carbon rings. Aliphatic cyclyl can be substituted or unsubstituted. In the present application, the term "carbocyclyl" generally refers to a residue derived by removing one hydrogen atom from a carbon atom of a carbon ring. The term "carbon ring" generally refers to a saturated or partially unsaturated monocyclic or polycyclic cyclic hydrocarbon. The carbon ring comprises 3 to 20 carbon atoms, and it may comprise 3 to 12 carbon atoms, 3 to 10 carbon atoms, or 3 to 8 carbon atoms. Non-limiting examples of a monocyclic carbon ring include cyclopropane, cyclobutane, cyclopentane, cyclopentene, cyclohexane, cyclohexene, cyclohexadiene, cycloheptane, cycloheptatriene, cyclooctane, etc.; and a polycyclic carbon ring can include spiro, fused and bridged carbon rings. Carbocyclyl can be substituted or unsubstituted. In certain cases, an aliphatic ring and a carbon ring can be used interchangeably.

In the present application, the term "partially unsaturated" generally refers to a cyclic structure containing at least one double bond or triple bond between the ring molecules. The term "partially unsaturated" encompasses cyclic structures with multiple unsaturations, but it is not intended to include aromatic or heteroaromatic rings as defined in the present application. The term "unsaturated" means that the moiety has one or more degrees of unsaturation.

In the present application, the term "aliphatic cyclylene" generally refers to a residue derived by removing two hydrogen atoms from carbon atoms of an aliphatic ring. For example, it can be cyclopropanylene, cyclobutanylene, cyclopentanylene, cyclopentenylene, cyclohexanylene, cyclohexenylene, cyclohexadienylene, cycloheptanylene, cycloheptatrienylene, cyclooctanylene, etc.; and a polycyclic carbon ring can include spiro, fused, and bridged carbon rings. Aliphatic cyclylene can be substituted or unsubstituted.

In the present application, the term "aliphatic heterocyclyl" generally refers to a stable non-aromatic 3- to 7-membered monocyclic carbocyclic structure, a fused 7- to 10-membered bicyclic heterocyclic structure or a bridged 6- to 10-membered bicyclic heterocyclic structure. These cyclic structures can be either saturated or partially saturated. In addition to carbon atoms, these cyclic structures also contain one or more heteroatoms which can be selected from the group consisting of oxygen, sulfur and nitrogen. For example, these cyclic structures contain 1-4 heteroatoms as defined above. The term "nitrogen" when used to refer to an atom on an aliphatic heterocyclic cyclic structure can include nitrogen that has subjected to a substitution reaction. For example, aliphatic heterocyclyl can include "heterocycloalkyl", and heterocycloalkyl can refer to a stable non-aromatic 3- to 7-membered monocyclic alkane structure, a fused 7- to 10-membered bicyclic heterocyclic structure, or a bridged 6- to 10-membered bicyclic heterocyclic structure. In addition to carbon atoms, these cyclic structures also contain one or more heteroatoms which can be selected from the group consisting of oxygen, sulfur and nitrogen. For example, these cyclic structures contain 1-4 heteroatoms as defined above. Heterocycloalkyl can be substituted or unsubstituted. Aliphatic heterocyclyl can be substituted or unsubstituted.

In the present application, the term "aliphatic heterocyclylene" generally refers to a residue derived by removing two hydrogen atoms from carbon atoms of an aliphatic heterocyclic ring. Aliphatic heterocyclylene can be substituted or unsubstituted.

In the present application, the terms "optional" or "optionally" generally mean that the event or circumstance subsequently described can but need not occur, and that the description includes instances where the event or circumstance occurs or does not occur. For example, "a heterocyclic group optionally substituted with an alkyl group" means that the alkyl group can but need not be present, and the description can include instances where the heterocyclic group is substituted with an alkyl group and where the heterocyclic group is not substituted with an alkyl group.

In the present application, the term "substituted" generally means that one or more hydrogen atoms in a group, e.g., up to 5, e.g., 1 to 3 hydrogen atoms, independently of each other, are substituted with corresponding number of substituents. Substituents are only in their possible chemical positions, and those skilled in the art can determine (either experimentally or theoretically) possible or impossible substitutions without undue efforts. For example, amino or hydroxyl groups with free hydrogens may be unstable when bound with carbon atoms with unsaturated (e.g., olefinic) bonds.

In the present application, as known to those skilled in the art, terms such as "alkyl", "alkenyl", "cycloalkyl", etc. can have names preceded by an identification to indicate the number of atoms present in the group in a particular instance, e.g., $C_1$-$C_4$ alkyl, $C_3$-$C_7$ cycloalkoxy, $C_1$-$C_4$ alkylcarbonylamino, etc., and the subscript number following "C" indicates the number of carbon atoms present in the group. For example, $C_3$ alkyl refers to an alkyl group having three carbon atoms (e.g., n-propyl, isopropyl); and in $C_{1-10}$, members of the group can have any number of carbons atoms falling within the range of 1-10.

One or more hydrogen atoms in a group, e.g., up to 5, e.g., 1 to 3 hydrogen atoms, independently of each other, are substituted with corresponding number of substituents. Substituents are only in their possible chemical positions, and those skilled in the art can determine (either experimentally or theoretically) possible or impossible substitutions without undue efforts. For example, amino or hydroxyl groups with free hydrogens may be unstable when bound with carbon atoms with unsaturated (e.g., olefinic) bonds.

In the present application, the term "compound" generally refers to a substance having two or more different elements. For example, the compound of this application may be an organic compound. For example, the compound

of this application can be a compound with a molecular weight of less than 500, can be a compound with a molecular weight of less than 1000, can also be a compound with a molecular weight of more than 1000, and can also be a compound with a molecular weight of more than 10000 or more than 100000. In the present application, a compound can also refer to a compound linked through chemical bonds, for example, it can be a compound in which one or more molecules with a molecular weight of less than 1000 are linked to a biological macromolecule through chemical bonds, and the biological macromolecule can be a polysaccharide, a protein, a nucleic acid, a peptide, etc. For example, the compound of this application can include a compound in which a protein is linked to one or more molecules with a molecular weight of less than 1000. The compound of this application can be a compound including a protein linked to one or more molecules with a molecular weight of less than 10000, and can be a compound including a protein linked to one or more molecules with a molecular weight of less than 100000.

In the present application, the term "comprise" generally refers to the inclusion of explicitly specified features, but not excluding other elements. The terms "more than" and "less than" generally refer to the instance that comprises the number itself.

In the present application, the term "about" generally refers to variation in the range of 0.5%-10% above or below the specified value, for example, variation in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the compound of this application comprises a tautomer, mesomer, racemate, enantiomer, and/or diastereomer thereof. In the present application, the term "diastereomer" generally refers to stereoisomers that have two or more chiral centers and whose molecules are not mirror images of each other. Diastereomers can have different physical properties, e.g., melting points, boiling points, spectral properties, and reactivities. In the present application, the terms "tautomer" or "tautomeric form" are used interchangeably and generally refer to structural isomers of different energies that can be interconverted through a low energy barrier. For example, protontautomers (also known as prototropic tautomers) include interconversions by migration of protons, such as keto-enol isomerization and imine-enamine isomerization. Valence tautomers include interconversion by recombination of some of the bonding electrons. In the present application, the term "mesomer" generally refers to a molecule containing asymmetric atoms, but having a symmetry factor such that the total intramolecular optical rotation is zero. The term "racemate" or "racemic mixture" refers to a composition composed of two enantiomeric species in equimolar amounts.

In the present application, certain atoms of the compounds of this application may occur in more than one isotopic form. For example, hydrogen can exist as protium ($^1H$), deuterium ($^2H$), and tritium ($^3H$), and carbon can exist naturally in three different isotopes ($^{12}C$, $^{13}C$, and $^{14}C$). Examples of isotopes that can be incorporated into the compound of this application also include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Thus, the compound of this application may be enriched in one or more of these isotopes relative to their natural abundances. Such isotopically enriched compounds can be used for a variety of uses, as known to those skilled in the art. For example, replacement with heavy isotopes such as deuterium ($^2H$) can offer certain therapeutic advantages, which can be due to a higher metabolic stability. For example, the natural abundance of deuterium ($^2H$) is about 0.015%. Therefore, for about every 6500 hydrogen atoms in nature, there is one deuterium atom. Accordingly, the deuterium-containing compound of this application has a deuterium abundance greater than 0.015% at one or more positions (as appropriate). Unless otherwise indicated, the structure described in the present application can also include compounds that differ only in the presence or absence of one or more isotopically enriched atoms. For example, compounds whose rest parts are consistent with the structures of this application except that the hydrogen atoms are replaced by deuterium or tritium, or the carbon atoms are replaced by carbon 13 or carbon 14 are all within the scope of the present application.

In the present application, the term "pharmaceutical composition" generally refers to a mixture containing one or more of the compounds described in this application, or physiologically/pharmaceutically acceptable salts or prodrugs thereof, and other chemical components, as well as other components, e.g., physiological/pharmaceutically acceptable carriers and excipients. A pharmaceutical composition can facilitate the administration to an organism, and facilitate the absorption of an active ingredient, thereby exerting its biological activity. The preparation of conventional pharmaceutical compositions can be found in the techniques commonly used in the art.

In the present application, the term "pharmaceutically acceptable salt" generally refers to a salt of the compound or ligand-drug conjugate of this application, or a salt of the compound described in this application, which salts can be of safety and/or efficacy when used in mammals, and can have desired biological activity. The antibody-antibody drug conjugate compound of this application can form salts with acids, and non-limiting examples of pharmaceutically acceptable salts include: hydrochloride, hydrobromide, hydroiodide, sulfate, bisulfate, citrate, acetate, succinate, ascorbate, oxalate, nitrate, pearate, hydrogen phosphate, dihydrogen phosphate, salicylate, hydrogen citrate, tartrate, maleate, fumarate, formate, benzoate, mesylate, esilate, benzenesulfonate, or p-toluenesulfonate.

In the present application, the term "conjugate" generally refers to a compound prepared by one or more chemical reactions of the compound of this application, or by linking the compounds of this application to each other through one or more linking structures such as bridges, spacers, or linking moieties, etc.

In the present application, the term "pharmaceutically acceptable carrier" generally refers to a carrier for administration of therapeutic agents, e.g., antibodies or polypeptides, genes and other therapeutic agents. The term refers to any pharmaceutical carrier that does not induce the production of antibodies detrimental to the individual receiving the composition itself and can be administered without undue toxicity. For example, a pharmaceutically acceptable carrier can be distinguished from a nucleic acid vector used in genetic engineering to contain a gene of interest. Suitable carriers can be large, slowly metabolized macromolecules, e.g., proteins, polysaccharides, polylactic acid, polyglycolic acid, polyamino acids, amino acid copolymers, lipid aggregates, and inactivated viral particles. These carriers are well-known by those skilled in the art. Pharmaceutically acceptable carriers in therapeutic compositions can include liquid, e.g., water, saline, glycerin and ethanol. Auxiliary substances, e.g., wetting or emulsifying agents, pH buffering substances, etc., can also be present in these carriers.

In the present application, the terms "Trop2", "TROP2" generally refer to a single-pass transmembrane type I cell membrane protein. In the present application, the term "Trop2" can also encompass homologues, variants and isoforms of Trop 2, including spliced isoforms. The term "Trop" also includes proteins having one or more sequences of Trop 2 homologues, variants and isoforms, as well as fragments of such sequences, as long as it is the variant protein (including isoforms). Trop2 can be human Trop2. For example, Uniprot Accession No. P09758 provides the description of Trop2 and the sequences.

In the present application, the term "HER2" generally refers to human epidermal growth factor receptor 2 (HER2). For example, the term "HER2" refers to any native HER2 from any human source. The term also encompasses "full-length" and unprocessed HER2 as well as any form of HER2 derived from processing in a cell (e.g., a mature protein). The term also encompasses naturally occurring variants and isoforms of HER2, e.g., spliced variants or allelic variants. For example, Uniprot Accession No. P04626 provides the description of HER2 and the sequences.

In the present application, the term "Nectin-4" generally refers to adhesion molecule 4. For example, the term "Nectin-4" refers to any native Nectin-4 from any human source. The term also encompasses "full-length" and unprocessed Nectin-4 as well as any form of Nectin-4 derived from processing in a cell (e.g., a mature protein). The term also encompasses naturally occurring variants and isoforms of Nectin-4, e.g., spliced variants or allelic variants. For example, Uniprot Accession No. Q96NY8 provides the description of Nectin-4 and the sequences.

In the present application, the term "chimeric antibody" generally refers to an antibody formed by fusing a variable region of a murine antibody with a constant region of a human antibody, which can alleviate the immune response induced by the murine antibody. To establish a chimeric antibody, a hybridoma that secretes a murine-specific monoclonal antibody can be established, and then the variable region gene can be cloned from the murine hybridoma cell. The constant region gene of human antibodies can be cloned as required, and the murine variable region gene can be linked to the human constant region gene to form chimeric gene, which can be subsequently inserted into an expression vector so as to express the chimeric antibody molecule in a eukaryotic system or a prokaryotic system.

In the present application, the term "humanized antibody", also known as CDR-grafted antibody, generally refers to antibodies produced by grafting murine CDR sequences into human antibody variable region frameworks, i.e., different types of human lineage antibody framework sequences. The heterologous reaction induced by chimeric antibodies as a result of the large amount of murine protein components carried can be overcome. Such framework sequences can be obtained from public DNA databases or published references that include lineage antibody gene sequences. For example, lineage DNA sequences of human heavy and light chain variable region genes can be found in the "VBase" human lineage sequence database.

In the present application, with regard to the term "fully human antibody", also known as "fully human monoclonal antibody", the variable region and constant region of the antibody can be both of human origin, with the immunogenicity and toxic side effects removed. The development of monoclonal antibodies has gone through four stages, namely: murine monoclonal antibodies, chimeric monoclonal antibodies, humanized monoclonal antibodies and fully human monoclonal antibodies. The antibodies or ligands described in this application can be fully human monoclonal antibodies. Related technologies for preparing fully human antibodies can be: human hybridoma technology, EBV transformation technology of B lymphocyte, phage display technology, transgenic mouse antibody preparation technology and single B cell antibody preparation technology, etc.

In the present application, the term "CDR" generally refers to one of the 6 hypervariable regions within the variable domain of an antibody that primarily contribute to antigen binding. One of the most commonly used definitions of the 6 CDRs is provided by Kabat E.A. et al., Chothia et al. and MacCallum et al. As used in this application, the Kabat definition of CDRs can be applied to CDR1, CDR2 and CDR3 of light chain variable domains (CDR L1, CDR L2 and CDR L3, or L1, L2 and L3), as well as CDR1, CDR2 and CDR3 of heavy chain variable domains (CDR H1, CDR H2 and CDR H3, or H1, H2 and H3).

In the present application, the term "group capable of coupling with mercapto" generally means that the compound A has mercapto, and the compound B has a group capable of coupling with mercapto. The linkage between the compound A and the compound B can be achieved by reacting the group of the compound B capable of coupling with mercapto with the mercapto group of the compound A.

In the present application, the term "linker" generally refers to a chemical structural fragment or bond with one

end attached to one group and the other end attached to another group. It can also be attached to other linkers and then attached to drugs and/or ligands. The direct or indirect attachment of ligands can refer to that the group is directly attached to a ligand through a covalent bond, and it can also be attached to the ligand through a linker. For example, the linker can be the structure shown in $Q_1$ as described in this application. For example, chemical structural fragments comprising acid-labile linker structures (e.g., hydrazones), protease-sensitive (e.g., peptidase-sensitive) linker structures, photolabile linker structures, dimethyl linker structures, or disulfide-containing linker structures, or bonds can be used as linkers.

In the present application, the term "linking group" generally refers to a group capable of linking to another group. For example, for a compound with a linking group, the attachment between the compound and another group can be achieved through a coupling reaction between the linking group and the another group. For example, a maleimide group can serve as a linking group.

In the present application, the term "drug unit" generally refers to a chemical moiety that directly or indirectly conjugates with an antibody or an antigen-binding fragment to form an immunoconjugate. For example, a "drug unit" includes, but is not limited to, compounds described herein with anti-tumor activity. For example, the drug unit includes a topoisomerase inhibitor.

In the present application, the term "compound with anti-tumor activity" generally refers to a compound capable of reducing the proliferation rate, viability or metastatic activity of tumor cells. For example, anti-tumor activity can be indicated by reduction in the growth rate of abnormal cells, or stabilization or reduction of tumor size during treatment, or longer survival due to treatment compared to a control in the absence of treatment. Anti-tumor activity can be assessed using recognized in vitro or in vivo tumor models, e.g., xenograft models.

In some embodiments of the present invention, the biologically active molecules in the conjugate are compounds with anti-tumor activity, specifically, for example radioisotopes, e.g., $At^{211}$, $I^{131}$, $I^{125}$, $Y^{90}$, $Re^{186,}$ $Re^{188}$, $Sm^{153}$, $Bi^{212}$, $P^{32}$, $Pb^{212}$ or radioisotopes of Lu; metal complexes, e.g., metal platinum complexes (such as oxaliplatin) or metal gold complexes; glycopeptide antibiotics, e.g., bleomycin or pingyangmycin; topoisomerase inhibitors; drugs that interfere with DNA synthesis, e.g., methotrexate, 5-fluorouracil, cytarabine, gemcitabine, mercaptopurine, pentostatin, fludarabine, cladribine, or nerabine, etc.; drugs that act on structural proteins, e.g., tubulin inhibitors (such as vinca alkaloids, vincristine, vinblastine, paclitaxels, maytansines, auristatins, Tubulysin B, or eribulin, etc.) ; tumor signaling pathway inhibitors, e.g., serine/threonine kinase inhibitors, tyrosine kinase inhibitors, aspartic acid kinase inhibitors or histidine kinase inhibitors, etc.; proteasome inhibitors; epigenetically related target inhibitors; tumor angiogenesis inhibitors; cyclin inhibitors;

In the present application, the term "topoisomerase inhibitor" generally refers to compounds or derivatives thereof that include topoisomerase I inhibitors and topoisomerase II inhibitors. Examples of topoisomerase I inhibitors include, but are not limited to, camptothecin and its analogs; topoisomerase II inhibitors (such as actinomycin D, adriamycin, doxorubicin, docarmycin, daunorubicin, mitoxantrone, podophyllotoxin or etoposide, etc.). A topoisomerase can refer to an enzyme that corrects the linking number of DNA by cleaving phosphodiester bonds in one or two strands of DNA, followed by rewinding and sealing.

In the present application, the term "camptothecin analog" generally refers to a compound that is structurally similar to or derived from camptothecin. For example, the structure of camptothecin can be described in CAS Accession No. 7689-03-4. For example, a camptothecin analog can refer to Exatecan (CAS Accession No. 171335-80-1) or Belotecan (CAS Accession No. 256411-32-2). The term "non-camptothecin topoisomerase I inhibitor" generally refers to heterocyclic molecules having topoisomerase I inhibitory activity including indolecarbazole, indenoisoquinolinone, benzophenanthridine, and dibenzonaphthyridinone, primarily Genz-644282 (CAS Accession No. 529488-28-6).

In the present application, the term disease "associated with the expression" of a target generally means that the occurrence and/or progression of the disease is associated with the expression level of the target. For example, when the expression level of a certain target in cells from a disease area, such as within a particular tissue or organ of a patient, is increased relative to the expression level in normal cells from a tissue or an organ, that is, the target is highly expressed. Alternatively, for example, when the expression level of a certain target in cells from a disease area, such as within a particular tissue or organ of a patient, is decreased relative to the expression level in normal cells from a tissue or an organ, that is, the target is under-expressed. Alternatively, for example, when cells from a disease area, such as within a particular tissue or organ of a patient, express a certain target, that is, the cells are positive. Alternatively, for example, when cells from a disease area, such as within a particular tissue or organ of a patient, don't express a certain target, i.e., the cells are negative. For example, target expression can be characterized by standard assays known in the art.

In the present application, the term "effective amount" generally refers to an amount of a therapeutic agent that treats, ameliorates, or prevents a target disease or condition, or exhibits a measurable therapeutic or prophylactic effect. The precise effective amount for a subject depends on the size and health condition of the subject, the nature and extent of the disorder, as well as the therapeutic agent and/or combination of therapeutic agents selected for administration. Therefore, it is useless to pre-specify the exact effective amount. However, for a given condition, routine experiments can be used to determine the effective amount, as can be judged by a clinician.

Unless specifically stated, all compounds appearing in this application are intended to include all possible optical isomers, such as single chiral compounds, or mixtures of various different chiral compounds (i.e., racemates). In all compounds of this application, each chiral carbon atom can optionally be in R configuration or S configuration, or a mixture of R configuration and S configuration.

As used herein, the term "compound of this application" refers to the compound of the present application. The term also includes various crystalline forms, pharmaceutically acceptable salts, hydrates or solvates of the compound of this application.

When a trade name is used herein, the trade name is intended to include the preparation of the trade name product, the corresponding generic drug thereof, and the active pharmaceutical ingredient of the trade name product.

As used herein, "antibody" is used in its broadest sense and specifically covers monoclonal antibodies, polyclonal antibodies, dimers, multimers, multispecific antibodies (e.g., bispecific antibodies), and antibody fragments, as long as they exhibit the desired biological activity. Antibodies can be murine, human, humanized, chimeric antibodies, or derived from other species. Antibodies are proteins produced by an immune system capable of recognizing and binding specific antigens. Target antigens generally have a large number of binding sites, also referred to as epitopes, recognized by CDRs of various antibodies. Each antibody that specifically binds to a different epitope has a different structure. Thus, an antigen can have more than one corresponding antibodies. Antibodies include full-length immunoglobulin molecules, or immunologically active portions of full-length immunoglobulin molecules, i.e., molecules containing an antigen or portion thereof that specifically binds a target of interest. Such targets include, but not limited to, cancer cells or cells that produce autoimmune antibodies associated with autoimmune diseases. Immunoglobulins described in this application can be of any type (e.g., IgG, IgE, IgM, IgD, and IgA), class (e.g., IgG1, IgG2, IgG3, IgG4, IgA1, and IgA2), or subclass of the immunoglobulin molecules. Immunoglobulins can be derived from any species. However, in one aspect, Immunoglobulins are derived from human, murine or rabbits. An "antibody fragment" can comprise a portion of a full-length antibody, which generally is an antigen-binding region or a variable region of the full-length antibody. Examples of antibody fragments include Fab, Fab', F(ab')2 and Fv fragments; diabodies; linear antibodies; minibodies; fragments prepared from Fab expression library; anti-idiotypic (anti-Id) antibodies; CDRs (complementarity determining regions); and any of the above epitope-binding fragments that immunospecifically bind to cancer cell antigens, viral antigens, or microbial antigens; single-chain antibody molecules; and multispecific antibodies formed from antibody fragments. The antibody constituting the antibody-drug conjugate in this application can maintain the antigen-binding ability in its original wild state. Thus, the antibody of this application can, for example, specifically bind to an antigen. Antigens involved include, for example, tumor-associated antigens (TAAs), cell surface receptor proteins and other cell surface molecules, regulators of cell survival, regulators of cell proliferation, molecules associated with tissue growth and differentiation (such as those known or predicted to be functional), lymphokines, cytokines, molecules involved in the regulation of the cell cycle, molecules involved in angiogenesis, as well as angiogenesis-related molecules (for example, the antigens to which the known antibodies bind can be one or a subset of the above categories, while other subsets contain other molecules/antigens with specific properties (compared to the target antigen)). Antibodies for use in the antibody drug conjugates include, but not limited to, antibodies directed against cell surface receptors and tumor-associated antigens. Such tumor-associated antigens are well known in the art and can be prepared by methods and information well known in the art for preparing antibodies. These targets can be specifically expressed on the surface of one or more cancer cells with little or no expression on the surface of one or more non-cancer cells. In general, such tumor-associated polypeptides can be more overexpressed on the surface of cancer cells than on the surface of non-cancer cells.

In the present application, the term "Enfortumab" generally refers to an antibody targeting Nectin-4. For example, Enfortumab can be described in WO2017042210A1. In the present application, Enfortumab can refer to any antibodies or antigen-binding fragments comprising the heavy chain variable regions CDR1-3 and light chain variable regions CDR1-3 of Enrozumab. In the present application, Ennozumab can refer to any antibodies or antigen-binding fragments comprising the heavy chain variable regions and light chain variable regions of Enrozumab.

In the present application, the term "Pertuzumab" generally refers to an antibody targeting HER2. For example, Pertuzumab can be described in WO2014172371A2. In the present application, Pertuzumab can refer to any antibodies or antigen-binding fragments comprising the heavy chain variable regions CDR1-3 and light chain variable regions CDR1-3 of Pertuzumab. In the present application, Pertuzumab can refer to any antibodies or antigen-binding fragments comprising the heavy chain variable regions and light chain variable regions of Pertuzumab.

In the present application, the term "Trastuzumab" generally refers to an antibody targeting HER2. For example, Trastuzumab can be described in US20060275305A1. In the present application, Trastuzumab can refer to any antibodies or antigen-binding fragments comprising the heavy chain variable regions CDR1-3 and light chain variable regions CDR1-3 of Trastuzumab. In the present application, Trastuzumabcan refer to any antibodies or antigen-binding fragments comprising the heavy chain variable regions and light chain variable regions of Trastuzumab.

In the present application, the term "Sacituzumab" generally refers to an antibody targeting TROP2. For example, Sacituzumab (hRS7) can be described in WO2003074566. In the present application, Sacituzumab can refer to any antibodies or antigen-binding fragments comprising the heavy chain variable regions CDR1-3 and light chain variable

regions CDR1-3 of Sacituzumab. In the present application, Sacituzumab refer to any antibodies or antigen-binding fragments comprising the heavy chain variable regions and light chain variable regions of Sacituzumab.

In the present application, the term "Patritumab" generally refers to an antibody targeting HER3. For example, Patritumab can be described in CN102174105B. In the present application, Patritumab can refer to any antibodies or antigen-binding fragments comprising the heavy chain variable regions CDR1-3 and light chain variable regions CDR1-3 of Patritumab. In the present application, Patritumab can refer to any antibodies or antigen-binding fragments comprising the heavy chain variable regions and light chain variable regions of Patritumab.

In the present application, the term "antibody H01L02" generally refers to an antibody targeting CDH6. For example, the monoclonal antibody H01L02 can be described in WO2018212136, US20200171163A1. For example, the H01L02 monoclonal antibody can be the monoclonal antibody used in the drug DS6000. In the present application, the H01L02 monoclonal antibody can refer to any antibodies or antigen-binding fragments comprising the heavy chain variable regions CDR1-3 and light chain variable regions CDR1-3 of the H01L02 monoclonal antibody. In the present application, the H01L02 monoclonal antibody can refer to any antibodies or antigen-binding fragments comprising the heavy chain variable regions and light chain variable regions of the H01L02 monoclonal antibody.

In the present application, the term "polypeptide residue" generally refers to a residue comprising one or more amino acid residues linked together. For example, one or more amino acids in a polypeptide residue can be optionally substituted. For example, the polypeptide residues of this application can be selected from the group consisting of phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine-alanine (Ala-Ala-Ala), alanine-alanine-asparagine (Ala-Ala-Asn), and glycine-glycine-phenylalanine-glycine (Gly-Gly-Phe-Gly).

In the present application, the term "polyethylene glycol group" generally refers to a residue comprising one or more ethylene glycol residues linked together. For example, a polyethylene glycol group can comprise $-(CH_2CH_2O)_p-$, where p is a number of at least 1. For example, the polyethylene glycol group in this application can be optionally substituted.

In the present application, the term "glycol group" generally refers to a polyethylene glycol group. For example, the glycol group in this application may be optionally substituted. For example, the number preceding a glycol group can indicate the number of ethylene glycol units in the glycol group. For example, a diethylene glycol group can refer to a residue polymerized from two ethylene glycols.

In the present application, the term "polysarcosine residue" generally refers to a residue comprising one or more sarcosine residues linked together. For example, a polysarcosine residue can comprise $-(COCH_2N(CH_3))_q-$, where p is a number of at least 1. For example, the polysarcosine residue in this application can be optionally substituted. For example, the structure comprising a polysarcosine residue can be

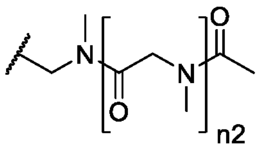
,

wherein n2 is a number from 4 to 18.

In the present application, the term "sodium dodecyl sulfate polyacrylamide gel electrophoresis" generally refers to an analytical characterization technique for substances. For example, sodium dodecyl sulfate polyacrylamide gel electrophoresis (SDS-PAGE) can detect the molecular weight of a substance.

In the present application, the term "Hydrophobic-Interaction Chromatography" generally refers to an analytical technique based on differences in hydrophobicity of substances.

In the present application, the term "liquid chromatograph-mass spectrometer" generally refers to an analytical method for identifying constituents of a substance. For example, liquid chromatograph-mass spectrometer can analyze the molecular weight of the substance to be tested by liquid chromatography combined with mass spectrometer.

In the present application, the term "tumor" generally refers to any new pathological tissue proliferation. With regard to the present application, angiogenesis is a part of the tumor profile. A tumor can be benign, or can be malignant. The term "tumor" is generally used to refer to a benign or malignant tumor, while the term "cancer" is generally used to refer to a malignant tumor, which can be a metastatic or non-metastatic cancer. Tumors that can be diagnosed using the methods of the present application are selected from the group consisting of breast cancer, ovarian cancer, non-Hodgkin lymphoma, Hodgkin lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal cancer, gastric cancer, neuroglioma and mesothelioma. For research, these tissues can be isolated from readily available sources by

methods well known to those skilled in the art.

**Detailed description of the invention**

In one aspect, the present application provides a compound, or a tautomer, mesomer, racemate, enantiomer, and diastereomer thereof, or a mixture thereof, or a pharmaceutically acceptable salt, prodrug or solvate thereof, wherein the compound includes a structure represented by formula (C-HER2):

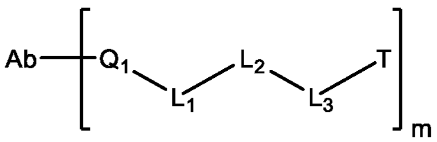 (C-HER2)

wherein, $Q_1$ can be a linking group,

$L_1$ can include $-L_{1a}-C(=O)-$,

wherein, $L_{1a}$ can be selected from the group consisting of optionally substituted alkylene groups, optionally substituted polyethylene glycol groups, optionally substituted alkenylene groups, optionally substituted alkynylene groups, optionally substituted aliphatic cyclylene groups, optionally substituted aliphatic heterocyclylene groups, optionally substituted arylene groups, and optionally substituted heteroarylene groups;

$L_2$ can include an optionally substituted polypeptide residue,

$L_3$ can include an optionally substituted spacer group. For example, the spacer group of this application may have self-degrading ability. For example, the spacer group of this application can include optionally substituted

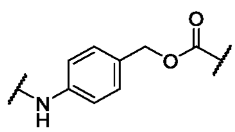,

or optionally substituted

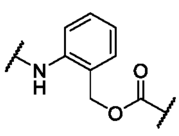,

wherein, $L_2$ and/or $L_3$ can include optionally substituted polysarcosine residues,

T can include a drug unit,

Ab is a ligand capable of binding to HER2, and m is a number from 1 to 8.

In another aspect, the present application also provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C-M-HER2):

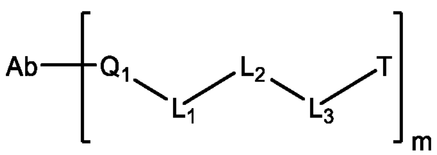 (C-M-HER2)

wherein, $Q_1$ can include a linker,
$L_1$ can include $-L_{1a}-C(=O)-$,
wherein, $L_{1a}$ can be selected from the group consisting of optionally substituted alkylene groups, optionally substituted polyethylene glycol groups, optionally substituted alkenylene groups, optionally substituted alkynylene groups, optionally substituted aliphatic cyclylene groups, optionally substituted aliphatic heterocyclylene groups, optionally substituted arylene groups, and optionally substituted heteroarylene groups;
$L_2$ can include an optionally substituted polypeptide residue,
$L_3$ can include an optionally substituted spacer group. For example, the spacer group of this application may have self-degrading ability. For example, the spacer group of this application can include optionally substituted

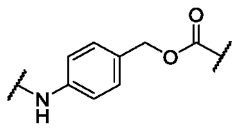

,

or optionally substituted

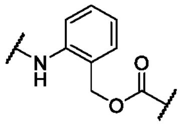

,

wherein, $L_2$, and/or $L_3$ can include an optionally substituted structural unit -X,
T can include a drug unit,
Ab is a ligand capable of binding to HER2, and m can be a number from 1 to 8.

For example, wherein, $L_3$ is selected from the group consisting of optionally substituted

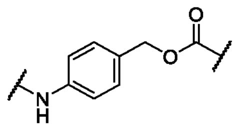

,

and optionally substituted

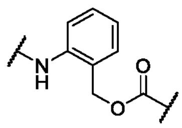

.

For example, wherein, the benzene ring of $L_3$ can be substituted with the optionally substituted structural unit -X. For example, the structural unit -X can be selected from the group consisting of optionally substituted

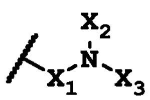

,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

For example, wherein, the benzene ring of $L_3$ can be substituted with the optionally substituted structural unit -X. For example, the structural unit -X can include optionally substituted

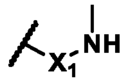,

wherein $X_1$ is selected from the group consisting of $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S, and the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

For example, wherein, the structural unit -X is optionally substituted

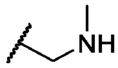.

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (CM2):

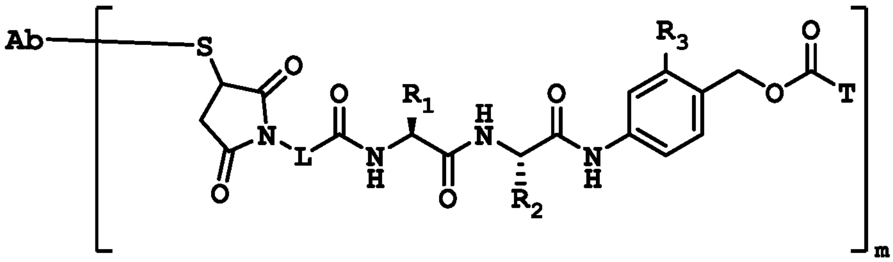 (C-M2),

wherein, L can contain an optionally substituted alkylene group, an optionally substituted polyethylene glycol group, and an optionally substituted aliphatic cyclylene groups; $R_1$ can be optionally substituted isopropyl or optionally substituted benzyl; $R_2$ can be optionally substituted methyl, optionally substituted

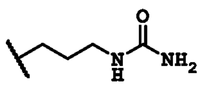

or optionally substituted

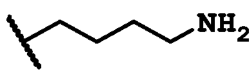;

$R_3$ can be hydrogen or $R_5$; wherein, $R_5$ can be optionally substituted

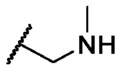,

and T can include Exatecan (CAS Accession No. 171335-80-1), and/or Belotecan (CAS Accession No. 256411-32-2),

and/or Genz-644282 (CAS Accession No. 529488-28-6).

For example, wherein, $Q_1$ can include a linker coupled with mercapto.

For example, wherein, $Q_1$ can be selected from the group consisting of optionally substituted

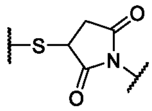

,

optionally substituted

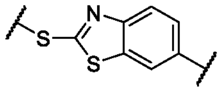

,

optionally substituted

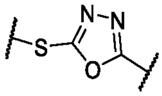

,

and optionally substituted

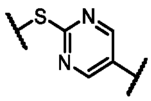

.

For example, wherein, $L_{1a}$ can be selected from the group consisting of optionally substituted $C_1$-$C_7$ alkylene groups, optionally substituted diethylene glycol to octaethylene glycol groups, optionally substituted $C_3$-$C_6$ aliphatic cyclylene groups, optionally substituted arylene groups, and optionally substituted heteroarylene groups.

For example, wherein, $L_{1a}$ can be selected from the group consisting of optionally substituted methylene groups, optionally substituted ethylene groups, optionally substituted propylene groups, optionally substituted butylene groups, optionally substituted pentylene groups, optionally substituted diethylene glycol groups, optionally substituted tetraethylene glycol groups, optionally substituted hexaethylene glycol groups, optionally substituted octaethylene glycol groups, and optionally substituted cyclohexylene groups.

For example, wherein, $L_{1a}$ can be selected from the group consisting of optionally substituted methylene groups, optionally substituted ethylene groups, optionally substituted propylene groups, optionally substituted butylene groups, and optionally substituted pentylene groups.

For example, wherein, $L_{1a}$ can be selected from the group consisting of optionally substituted diethylene glycol groups, optionally substituted triethylene glycol groups, optionally substituted tetraethylene glycol groups, optionally substituted pentaethylene glycol groups, optionally substituted hexaethylene glycol groups, optionally substituted heptaethylene glycol groups, and optionally substituted octaethylene glycol groups.

For example, wherein, $L_{1a}$ can be selected from the group consisting of optionally substituted cyclopropylene groups, optionally substituted cyclobutylene groups, and optionally substituted cyclohexylene groups.

For example, wherein, $L_2$ can include optionally substituted polypeptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

For example, wherein, $L_2$ can include optionally substituted polypeptide residues composed of amino acids selected from the group consisting of glycine, phenylalanine, valine, alanine, arginine, citrulline, aspartic acid, asparagine, and lysine.

For example, wherein, $L_2$ can include optionally substituted polypeptide residues selected from the group consisting of phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine-alanine (Ala-Ala-Ala), alanine-alanine-asparagine (Ala-Ala-Asn), and glycine-glycine-phenylalanine-glycine (Gly-Gly-Phe-Gly).

For example, wherein, $L_2$ can include optionally substituted polypeptide residues selected from the group consisting of phenylalanine-lysine (Phe-Lys), valine-alanine (Val-Ala), valine-citrulline (Val-Cit), and valine-lysine (Val-Lys).

For example, wherein, when $L_2$ includes a lysine residue, the lysine residue can be substituted with a structure $R_1$ comprising a polysarcosine residue.

For example, any H included in $L_2$ can be substituted with $R_1$.

For example, wherein, the $R_1$ can be optionally substituted

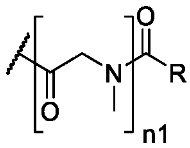
,

wherein n1 is a number from 4 to 18, and R is selected from the group consisting of $C_1$-$C_6$ alkyl, $C_1$-$C_6$ cycloalkyl, and $C_1$-$C_6$ alkoxy.

For example, wherein, n1 can be 4 to 18, 8 to 18, 4 to 12 or 8 to 12. For example, wherein, n1 can be 4, 5, 6, 7, 8, 9, 10, 11, 12, 13, 14, 15, 16, 17 or 18.

For example, wherein, $L_3$ is selected from the group consisting of optionally substituted

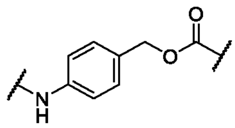
,

and optionally substituted

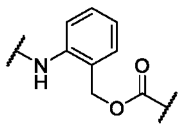
.

For example, wherein, the benzene ring of $L_3$ can be substituted with a structure $R_2$ comprising a polysarcosine residue.

For example, any H included in the benzene ring of $L_3$ can be substituted with $R_2$.

For example, wherein, the benzene ring of $L_3$ is linked to the optionally substituted polysarcosine residues through a structural unit -X-, and the structural unit -X- is selected from the group consisting of optionally substituted

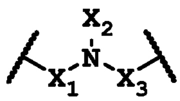
,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

For example, the benzene ring of $L_3$ is linked to the optionally substituted polysarcosine residues through a structural unit -X-, and the structural unit -X- is selected from, but not limited to,

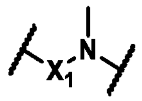, 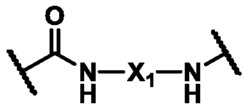,

wherein $X_1$ is selected from the group consisting of $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S, and the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

For example, wherein, the structural unit -X- is optionally substituted

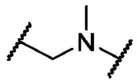,

and the optionally substituted polysarcosine residue comprises

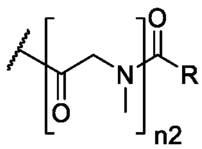,

wherein n2 is a number from 4 to 18, and R is selected from the group consisting of $C_1$-$C_6$ alkyl, $C_1$-$C_6$ cycloalkyl, and $C_1$-$C_6$ alkoxy.

For example, wherein, the structural unit -X- is selected from the group consisting of optionally substituted

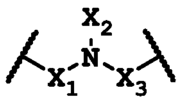,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl, and the optionally substituted polysarcosine residue comprises

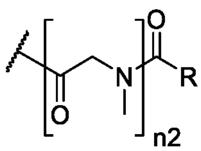,

wherein n2 is a number from 4 to 18, and R is selected from the group consisting of $C_1$-$C_6$ alkyl, $C_1$-$C_6$ cycloalkyl, and $C_1$-$C_6$ alkoxy.

For example, wherein, the structural unit -X- is optionally substituted

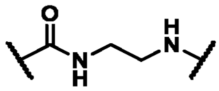,

and the optionally substituted polysarcosine residue comprises

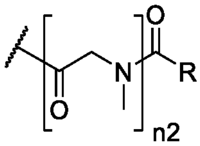,

wherein n2 is a number from 4 to 18, and R is selected from the group consisting of $C_1$-$C_6$ alkyl, $C_1$-$C_6$ cycloalkyl, and $C_1$-$C_6$ alkoxy.

For example, wherein, the structural unit -X- is selected from the group consisting of optionally substituted

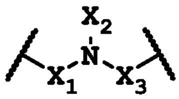,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl, and the optionally substituted polysarcosine residue comprises

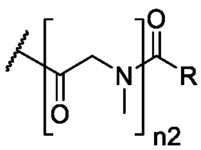,

wherein n2 is a number from 4 to 18, and R is selected from the group consisting of $C_1$-$C_6$ alkyl, $C_1$-$C_6$ cycloalkyl, and $C_1$-$C_6$ alkoxy.

For example, wherein, n2 can be 4 to 18, 8 to 18, 4 to 12 or 8 to 12. For example, wherein, n2 can be 4, 5, 6, 7, 8, 9, 10, 11, 12, 13, 14, 15, 16, 17 or 18.

For example, wherein, T can include a compound with anti-tumor activity.

For example, wherein, T can include a topoisomerase inhibitor.

For example, wherein, T can include camptothecin and non-camptothecin topoisomerase I inhibitors.

For example, wherein, T can include Exatecan (CAS Accession No. 171335-80-1), and/or Belotecan (CAS Accession No. 256411-32-2), and/or Genz-644282(CAS Accession No. 529488-28-6).

For example, wherein, T can be a structure selected from the group consisting of

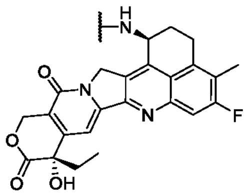 , 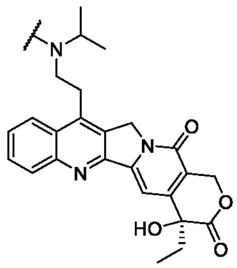 , and 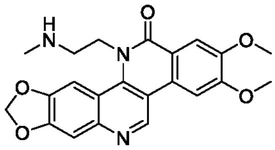 .

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C-HER2):

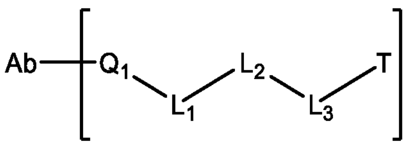 (C-HER2)

wherein, $Q_1$ can include a linker coupled with mercapto,

$L_1$ can include $-L_{1a}-C(=O)-$, wherein, $L_{1a}$ can be selected from the group consisting of optionally substituted $C_1$-$C_7$ alkylene groups, optionally substituted diethylene glycol to octaethylene glycol groups, optionally substituted $C_3$-$C_6$ aliphatic cyclylene groups, optionally substituted arylene groups, and optionally substituted heteroarylene groups;

$L_2$ can include optionally substituted polypeptide residues composed of amino acids selected from the group consisting of glycine, phenylalanine, valine, alanine, arginine, citrulline, aspartic acid, asparagine, and lysine,

$L_3$ can include optionally substituted

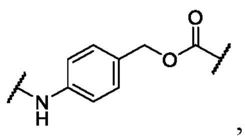 , 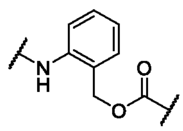 ,

wherein, $L_2$ can include polysarcosine residues,

T can include a topoisomerase inhibitor.

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C-HER2):

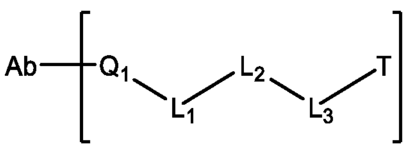 (C-HER2)

wherein, $Q_1$ can include a linker coupled with mercapto,

$L_1$ can include $-L_{1a}-C(=O)-$, wherein, $L_{1a}$ can be selected from the group consisting of optionally substituted $C_1$-$C_7$ alkylene groups, optionally substituted diethylene glycol to octaethylene glycol groups, optionally substituted $C_3$-$C_6$ aliphatic cyclylene groups, optionally substituted arylene groups, and optionally substituted heteroarylene groups;

$L_2$ can include optionally substituted polypeptide residues composed of amino acids selected from the group consisting of glycine, phenylalanine, valine, alanine, arginine, citrulline, aspartic acid, asparagine, and lysine,

$L_3$ can include optionally substituted

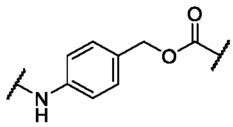 , 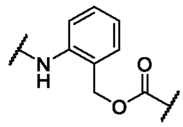 ,

wherein, $L_3$ can include polysarcosine residues,

T can include a topoisomerase inhibitor.

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C-HER2):

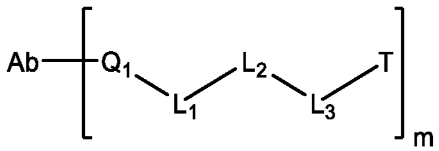 (C-HER2)

wherein, $Q_1$ can include optionally substituted

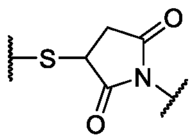 ,

$L_1$ can include $-L_{1a}-C(=O)-$, wherein, $L_{1a}$ can include optionally substituted $C_1$-$C_7$ alkylene groups;

$L_2$ can include a structure selected from the group consisting of phenylalanine-lysine (Phe-Lys), valine-alanine (Val-Ala), valine-citrulline (Val-Cit), and valine-lysine (Val-Lys),

$L_3$ can include optionally substituted

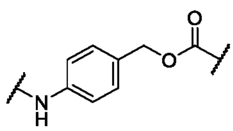 , 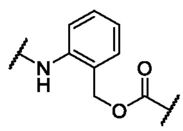 ,

wherein, $L_2$ can include a lysine residue, and the lysine residue can be substituted with optionally substituted

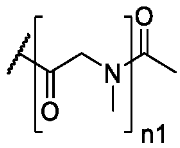 ,

wherein n1 is a number from 4 to 18,

T can include Exatecan (CAS Accession No. 171335-80-1), and/or Belotecan (CAS Accession No. 256411-32-2), and/or Genz-644282(CAS Accession No. 529488-28-6).

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C-HER2):

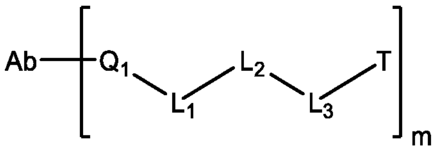 (C-HER2)

wherein, $Q_1$ can be selected from the group consisting of optionally substituted

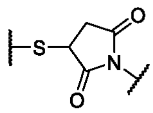,

optionally substituted

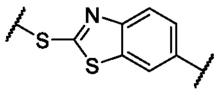,

optionally substituted

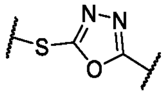,

and optionally substituted

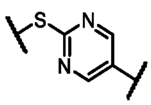,

$L_1$ can include $-L_{1a}-C(=O)-$, wherein, $L_{1a}$ can include optionally substituted $C_1$-$C_7$ alkylene groups;
$L_2$ can include a structure selected from the group consisting of phenylalanine-lysine (Phe-Lys), valine-alanine (Val-Ala), valine-citrulline (Val-Cit), and valine-lysine (Val-Lys),
$L_3$ can include optionally substituted

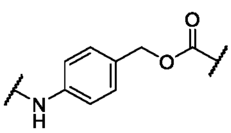, 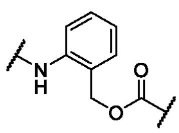,

wherein, $L_3$ can be substituted with optionally substituted

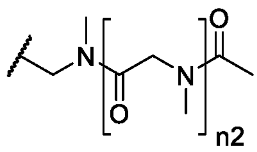,

wherein n2 is a number from 4 to 18,
T can include Exatecan (CAS Accession No. 171335-80-1), and/or Belotecan (CAS Accession No. 256411-32-2), and/or Genz-644282(CAS Accession No. 529488-28-6).

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C2-HER2):

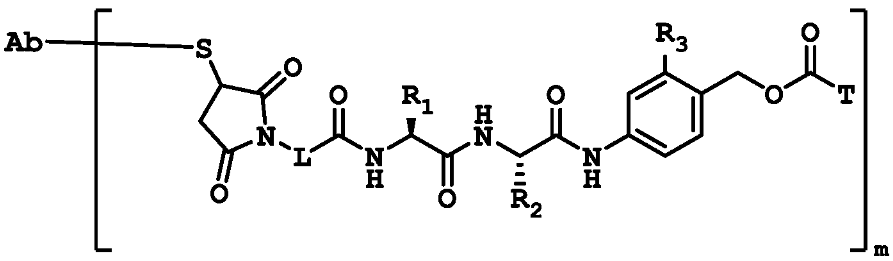 (C2-HER2),

wherein, L can contain an optionally substituted alkylene group, an optionally substituted polyethylene glycol group, and an optionally substituted aliphatic cyclylene group; $R_1$ can be optionally substituted isopropyl or optionally substituted benzyl; $R_2$ can be optionally substituted methyl, optionally substituted

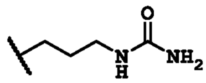,

or optionally substituted

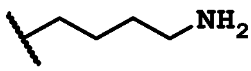;

$R_3$ can be hydrogen or optionally substituted methyl; wherein, $R_2$ can be substituted with optionally substituted

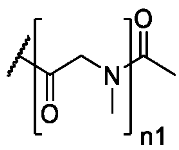,

wherein n1 is a number from 4 to 18, and T can include Exatecan (CAS Accession No. 171335-80-1), and/or Belotecan (CAS Accession No. 256411-32-2), and/or Genz-644282(CAS Accession No. 529488-28-6).

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C2-HER2):

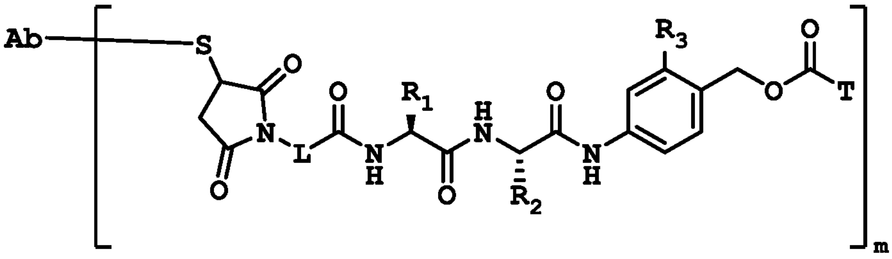

(C2-HER2),

wherein, L can contain an optionally substituted alkylene group, an optionally substituted polyethylene glycol group, and an optionally substituted aliphatic cyclylene group; $R_1$ can be optionally substituted isopropyl or optionally substituted benzyl; $R_2$ can be optionally substituted methyl, optionally substituted

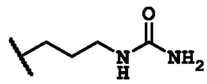

,

or optionally substituted

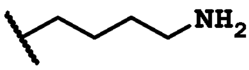

;

$R_3$ can be hydrogen or optionally substituted methyl; wherein, $R_2$ can be substituted with optionally substituted

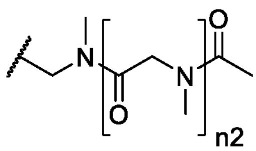

,

wherein n2 is a number from 4 to 18, and T can include Exatecan (CAS Accession No. 171335-80-1), and/or Belotecan (CAS Accession No. 256411-32-2), and/or Genz-644282(CAS Accession No. 529488-28-6).

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C2-HER2):

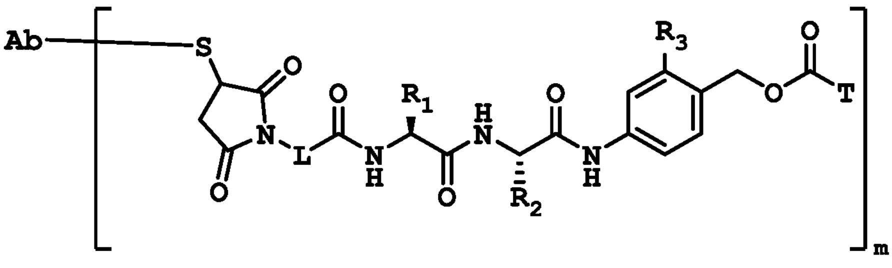

(C2-HER2),

wherein, L can contain an optionally substituted alkylene group, an optionally substituted polyethylene glycol group, and an optionally substituted aliphatic cyclylene group; $R_1$ can be optionally substituted isopropyl or optionally substituted benzyl; $R_2$ can be optionally substituted

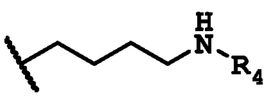

;

$R_3$ can be hydrogen or optionally substituted methyl; wherein, $R_4$ can be optionally substituted

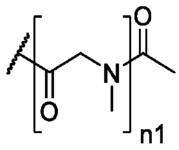
,

wherein n1 is a number from 4 to 18, and T can include Exatecan (CAS Accession No. 171335-80-1), and/or Belotecan (CAS Accession No. 256411-32-2), and/or Genz-644282(CAS Accession No. 529488-28-6).

For example, the present application provides a compound, or a tautomer, mesomer, racemate, enantiomer, and diastereomer thereof, or a mixture thereof, or a pharmaceutically acceptable salt, prodrug or solvate thereof, wherein the compound can include a structure represented by formula (C2-HER2):

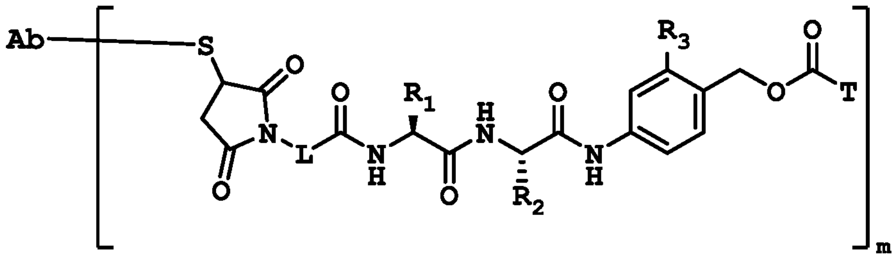
(C2-HER2),

wherein, L can contain an optionally substituted alkylene group, an optionally substituted polyethylene glycol group, and an optionally substituted aliphatic cyclylene group; $R_1$ can be optionally substituted isopropyl or optionally substituted benzyl; $R_2$ can be optionally substituted methyl, optionally substituted

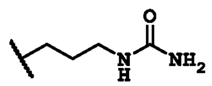
,

or optionally substituted

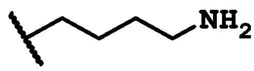
;

$R_3$ can be hydrogen or $R_5$; wherein, $R_5$ can be optionally substituted

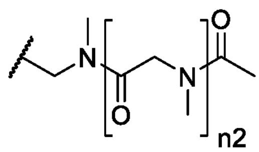
,

wherein n2 is a number from 4 to 18, and T can include Exatecan (CAS Accession No. 171335-80-1), and/or Belotecan (CAS Accession No. 256411-32-2), and/or Genz-644282(CAS Accession No. 529488-28-6).

For example, wherein, the Ab can include anti-HER2 antibody or an antigen-binding fragment thereof.

For example, wherein the antibody can be selected from the group consisting of a murine antibody, a chimeric antibody, a humanized antibody, and a fully human antibody.

For example, wherein, the antibody can include a monoclonal antibody.

For example, wherein, the antibody can include a bispecific antibody.

For example, wherein, the antigen-binding fragment can be selected from the group consisting of Fab, Fab', an Fv fragment, $F(ab')_2$, $F(ab)_2$, scFv, di-scFv, VHH, and dAb.

For example, wherein, the heavy chains HCDR1, HCDR2 and HCDR3 and the light chains LCDR1, LCDR2 and LCDR3 of the Ab include the heavy chains HCDR1, HCDR2 and HCDR3 and the light chains LCDR1, LCDR2 and LCDR3 of the anti-HER2 antibody, respectively.

For example, wherein, the heavy chain variable region VHs and the light chain variable region VLs of the Ab include the heavy chain variable region VHs and the light chain variable region VLs of the anti-HER2 antibody, respectively.

For example, wherein, the heavy chain and the light chain of the Ab include the heavy chain and the light chain of the anti-HER2 antibody, respectively.

For example, wherein, the Ab can include Trastuzumab or Pertuzumab.

For example, wherein, the m can be determined by a method selected from the group consisting of hydrophobic chromatography, sodium dodecyl sulfate polyacrylamide gel electrophoresis, and liquid chromatograph-mass spectrometer. For example, m is the average value of the molar ratio of drug molecules to monoclonal antibody molecules in the antibody drug conjugate obtained after conjugating a single monoclonal antibody molecule with a cytotoxic drug, and m can be an integer or decimal from 1 to 8, for example, m can be about 1 to about 2, about 1 to about 3, about 1 to about 4, about 1 to about 5, about 1 to about 6, about 1 to about 7, or about 1 to about 8; and for example, m can be about 2 to about 8, about 3 to about 8, about 4 to about 8, about 5 to about 8, about 6 to about 8, about 7 to about 8, or about 1, about 2, about 3, about 4, about 5, about 6, about 7 or about 8.

In another aspect, the present application also provides a compound, or a tautomer, mesomer, racemate, enantiomer, and diastereomer thereof, or a mixture thereof, or a pharmaceutically acceptable salt, prodrug or solvate thereof,

wherein, the compound can include a structure selected from the group consisting of

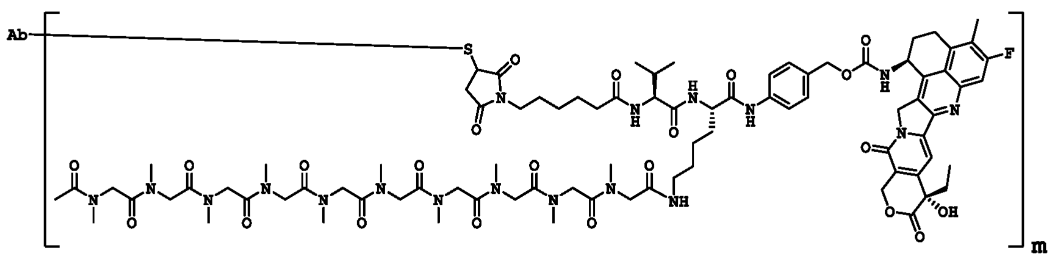

(C-HER2-1),

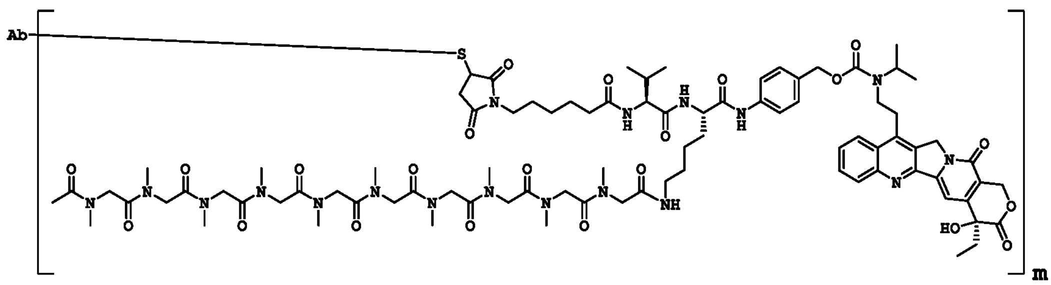

(C-HER2-2),

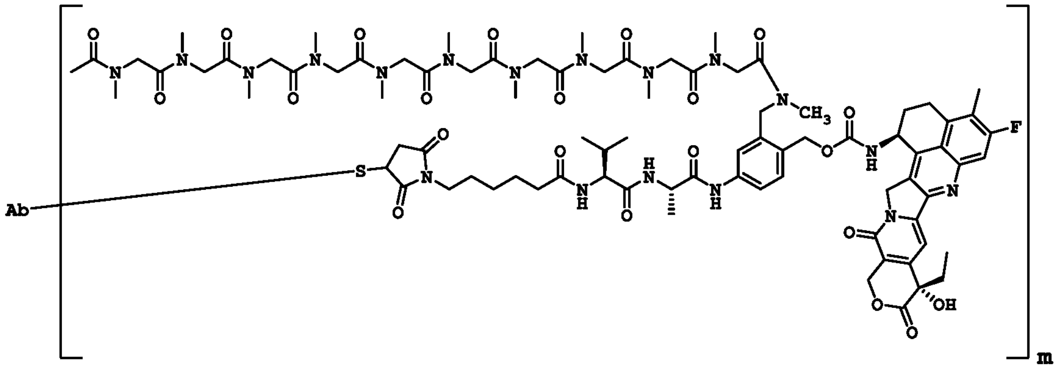

(C-HER2-3),

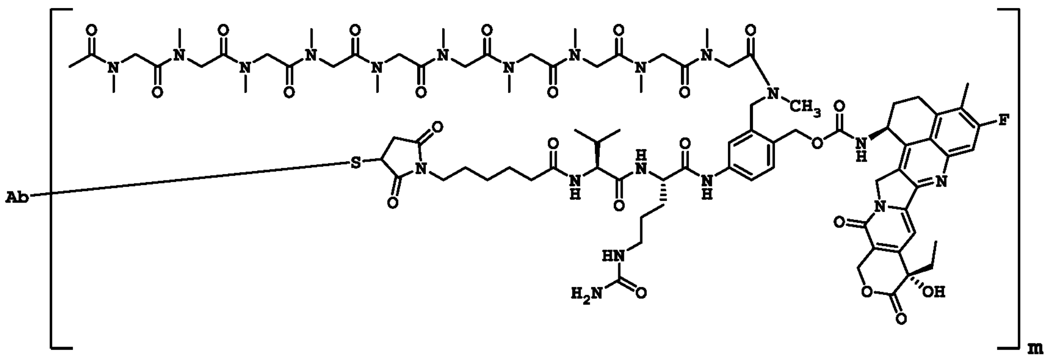

(C-HER2-4),

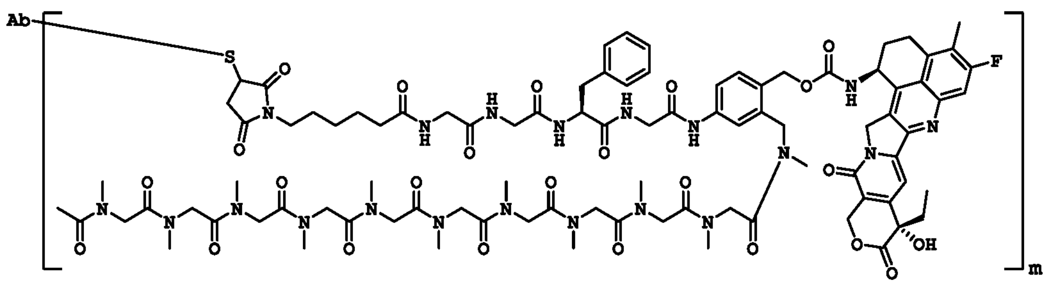

(C-HER2-5),

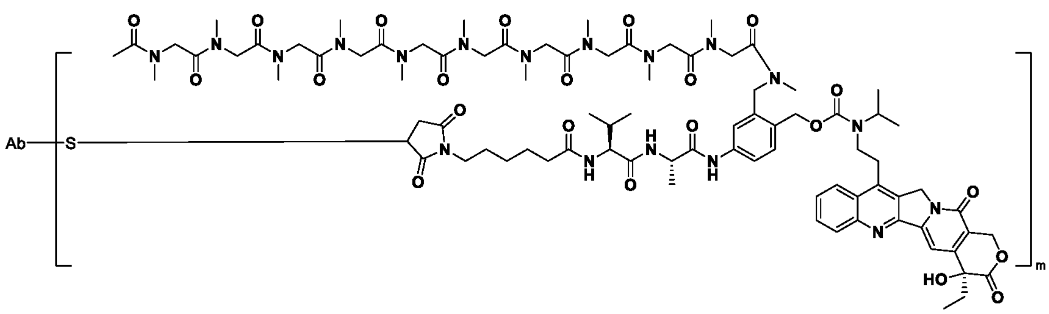

(C-HER2-6),

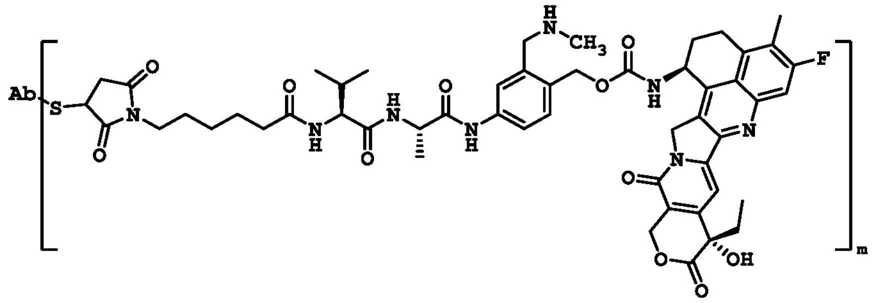

(C-HER2-7),

and

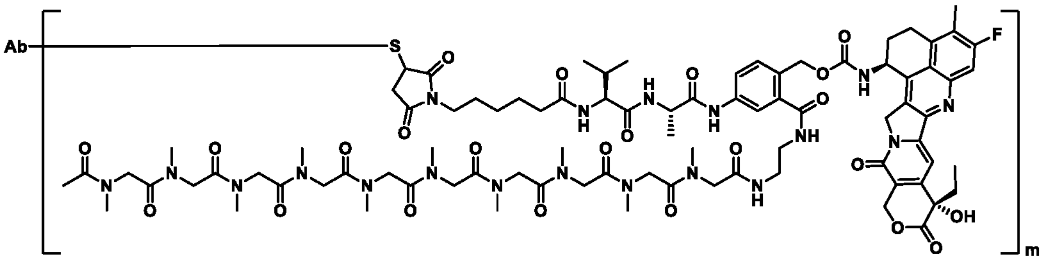

(C-HER2-8),

Ab is a ligand capable of binding to HER2, and m is a number from 1 to 8.

The ligand described in this application can be protein hormones, lectins, growth factors, antibodies, or other molecules capable of binding to cells, receptors and/or antigens. For example, the ligand of this application can be an anti-HER2 antibody or an antigen-binding fragment thereof.

In the present application, the ligand includes at least one CDR in the light chain variable region VL of an antibody. The CDRs described in this application can be defined according to Kabat; or can be defined according to Chothia, and the CDR sequences defined in various ways are all encompassed within the protection scope of this application.

For example, the antigen-binding protein of this application can include CDR1-3 of the heavy chain variable region and CDR1-3 of the light chain variable region, wherein CDR1-3 of the heavy chain variable region and CDR1-3 of the light chain variable region can be CDR1-3 of the heavy chain variable region and CDR1-3 of the light chain variable region of Trastuzumab and Pertuzumab, respectively. For example, the antigen binding protein of this application can have the binding ability to bind HER2.

For example, the antigen-binding protein of this application can include the heavy chain variable region and the light chain variable region, wherein the heavy chain variable region and the light chain variable region can be the heavy chain variable region and the light chain variable region of Trastuzumab and Pertuzumab, respectively. For example, the antigen binding protein of this application can have the binding ability to bind HER2.

For example, the antigen-binding protein of this application can include the heavy chain and the light chain, wherein the heavy chain and the light chain can be the heavy chain and the light chain of Trastuzumab and Pertuzumab, respectively.

For example, the heavy chain amino acid sequence of Trastuzumab can be as shown in SEQ ID NO: 3, and the light chain amino acid sequence of Trastuzumab can be as shown in SEQ ID NO: 4.

As a significant advantage to verify the compounds of the present application, for example, the ligands of the present application can be: for example, the heavy chain amino acid sequence of Sacituzumab can be as shown in SEQ ID NO: 1, and the light chain amino acid sequence of Sacituzumab can be as shown in SEQ ID NO: 2. For example, the heavy chain amino acid sequence of Trastuzumab can be as shown in SEQ ID NO: 3, and the light chain amino acid sequence of Trastuzumab can be as shown in SEQ ID NO: 4. For example, the heavy chain amino acid sequence of Pertuzumab can be as shown in SEQ ID NO: 5, and the light chain amino acid sequence of Pertuzumab can be as shown in SEQ ID NO: 6. For example, the heavy chain amino acid sequence of Enfortumab can be as shown in SEQ ID NO: 7, and the light chain amino acid sequence of Enfortumab can be as shown in SEQ ID NO: 8. For example, the heavy chain amino acid sequence of Patritumab can be as shown in SEQ ID NO: 9, and the light chain amino acid sequence of Patritumab can be as shown in SEQ ID NO: 10. For example, the heavy chain amino acid sequence of the antibody H01L02 can be as shown in SEQ ID NO: 11, and the light chain amino acid sequence of the antibody H01L02 can be as shown in SEQ ID NO: 12.

The antibody of this application can be prepared using techniques well known in the art, e.g., hybridoma methods, recombinant DNA techniques, phage display techniques, synthetic techniques, or a combination of these techniques, or other techniques known in the art. A variant may refer to an amino acid sequence mutant of an antibody, as well as a covalent derivative of a native polypeptide, provided that a biological activity comparable to that of the native polypeptide is retained. An amino acid sequence mutant generally differs from a native amino acid sequence by the substitution of one or more amino acids in the native amino acid sequence, or the deletion and/or insertion of one or more amino acids in the polypeptide sequence. A deletion mutant includes a fragment of a native polypeptide and N-terminal and/or C-terminal truncated mutants. Typically, an amino acid sequence mutant has at least 70%, 75%, 80%, 85%, 90%, 95%, 98% or 99% or more homology compared to a native sequence.

Since the antibody-drug conjugate provided in this application can target specific cell populations and bind to specific proteins (antigens) on the cell surfaces, so as to release the drug into the cells in an active form through the endocytosis of the conjugate or drug infiltration, the antibody-drug conjugate of this application can be used to treat a target disease. The antibody-drug conjugate of this application can be administered to a subject (e.g., a human) in a therapeutically effective amount through a suitable route. A subject in need of treatment can be a patient at risk, or suspected of having a disorder associated with the activity or expression level of a particular antigen. Such patients can be identified by routine physical examination.

While the antibody-drug conjugate of this application is used for treatment, it can be delivered by methods routine in the art. For example, it can be introduced into cells by using liposomes, hydrogels, cyclodextrins, biodegradable nanocapsules, or bioadhesive microspheres. Alternatively, the nucleic acid or vector can be delivered locally by direct injection or by using an infusion pump. Other methods can include the use of various transport and carrier systems through the use of conjugates and biodegradable polymers.

In one aspect, the present application provides a pharmaceutical composition, which can include the compound described in any one of this application, or a tautomer, mesomer, racemate, enantiomer, and diastereomer thereof, or a mixture thereof, or a pharmaceutically acceptable salt, prodrug or solvate thereof, and can include optionally a pharmaceutically acceptable carrier.

In addition to the active compound, the pharmaceutical composition described in this application can contain one or more excipients, which can be ingredients selected from the group consisting of fillers (diluents), binders, wetting agents, disintegrating agents and excipients, etc. Depending on the method of administration, the composition can contain from 0.1 to 99% by weight of the active compound.

The pharmaceutical composition containing the active ingredient can be in a form suitable for oral administration, e.g., tablets, dragees, lozenges, aqueous or oily suspensions, dispersible powders or granules, emulsions, hard or soft capsules, or syrups. Oral compositions can be prepared according to any method known in the art for preparing pharmaceutical compositions. The composition can contain binders, fillers, lubricants, disintegrants or pharmaceutically acceptable wetting agents, etc., as well as one or more ingredients selected from the group consisting of sweetening agents, flavoring agents, coloring agents and preservatives.

An aqueous suspension can contain an active substance and excipients suitable for preparing the aqueous suspension which can be used for mixing. An aqueous suspension can also contain one or more preservatives, e.g., one or more coloring agents, one or more flavoring agents, and one or more sweetening agents. An oily suspension can be formulated by suspending an active ingredient in a vegetable oil. An oily suspension can contain thickening agents. The above-mentioned sweetening and flavoring agents can also be added.

The pharmaceutical composition can also be dispersible powders and granules providing the active ingredients, which were used for preparing aqueous suspensions by adding water and mixing with one or more of dispersing agents, wetting agents, suspending agents or preservatives. Other excipients, e.g., sweetening, flavouring and colouring agents can also be added. These compositions are preserved by adding antioxidants, e.g., ascorbic acid. The pharmaceutical composition of this application can also be in the form of an oil-in-water emulsion.

The pharmaceutical composition can be in the form of a sterile injectable aqueous solution. Acceptable and usable vehicles or solvents are water, Ringer’s solution and isotonic sodium chloride solution. A sterile injectable preparation can be a sterile injectable oil-in-water microemulsion in which an active ingredient is dissolved in an oily phase. For example, the active ingredient is dissolved in a mixture of soybean oil and lecithin. The oil solution can then be added to a mixture of water and glycerin and treated to form a microemulsion. Injections or microemulsions can be injected into a patient’s bloodstream by local bolus injection. Alternatively, the solution and microemulsion can be administered in such a way that a constant circulating concentration of the compounds of this application can be maintained. To maintain this constant concentration, a continuous intravenous drug delivery device can be used. For example, the device can be an intravenous injection pump.

The pharmaceutical composition can be in the form of sterile injectable aqueous or oily suspensions for intramuscular and subcutaneous administration. The suspension can be formulated according to the known art using suitable dispersing or wetting agents and suspending agents described in this application above. A sterile injectable preparation can also be a sterile injectable solution or suspension prepared in a non-toxic parenterally acceptable diluent or solvent.

Alternatively, sterile fixed oils are conveniently employed as a solvent or suspending medium.

The compound of this application can be administered in the form of a suppository for rectal administration. These pharmaceutical compositions can be prepared by mixing the drug with a suitable non-irritating excipient which is solid at ordinary temperatures but liquid in the rectum and therefore will melt in the rectum to release the drug. Such materials include cocoa butter, glycerinated gelatin, hydrogenated vegetable oils, and mixtures of polyethylene glycols and fatty acid esters of polyethylene glycols of various molecular weights.

As is well known to those skilled in the art, the dosage of a drug to be administered depends on a variety of factors including, but not limited to, the activity of the particular compound used, the age of the patient, the weight of the patient, the health of the patient, the behavior of the patient, the diet of the patient, the administration time, the administration mode, the excretion rate, combination of drugs, etc.; in addition, the optimal treatment modality such as the mode of treatment, the compound described in this application, or a tautomer, mesomer, racemate, enantiomer, and diastereomer thereof, or a mixture thereof, or a pharmaceutically acceptable salt thereof, the daily dosage of the compound, or a tautomer, mesomer, racemate, enantiomer, and diastereomer thereof, or a mixture thereof, or a pharmaceutically acceptable salt thereof, or the type of the pharmaceutically acceptable salt thereof can be verified according to conventional therapeutic regimens.

The pharmaceutical composition of this application can contain a safe and effective amount of the antibody-drug conjugate of this application and a pharmaceutically acceptable carrier. Such carriers can include, but are not limited to, saline, buffers, dextrose, water, glycerin, ethanol, and combinations thereof. In general, the pharmaceutical preparation should match the mode of administration, and the pharmaceutical composition of this application can be prepared as a solution, for example, prepared by a conventional method with saline or an aqueous solution containing glucose and other adjuvants. The pharmaceutical composition can be manufactured under sterile conditions. The active ingredient can be administered in a therapeutically effective amount.

The effective amount of the antibody-drug conjugate described in this application can vary with the mode of administration, the severity of the disease to be treated, etc. Selection of an effective amount can be determined by one of ordinary skill in the art based on various factors (e.g., through clinical trials). The factors can include, but not limited to, the pharmacokinetic parameters of the diabody conjugate, e.g., bioavailability, metabolism, half-life, etc.; and the severity of the disease to be treated of the patient, the weight of the patient, the immune status of the patient, the route of administration, etc. In general, when the antibody-drug conjugate of this application is administered in an appropriate dose per day, satisfactory results can be obtained. For example, several divided doses can be administered daily or the dose can be proportionally reduced depending on the exigencies of the therapeutic situation.

The compounds of this application can be administered alone, or can be administered in combination with other pharmaceutically acceptable therapeutic agents. When using the pharmaceutical composition, a safe and effective amount of the compound of this application can be applied to a mammal (such as a human) in need of treatment, wherein the dosage when administration can be a pharmaceutically considered effective dosage, and the specific dosage can also consider factors such as the administration route, the health status of the patient, etc.

The present application provides use of the compound of this application, or a tautomer, mesomer, racemate, enantiomer, and diastereomer thereof, or a mixture thereof, or a pharmaceutically acceptable salt, prodrug or solvate thereof, and/or the pharmaceutical composition of this application in the preparation of a drug for treating and/or preventing tumors. For example, the tumor is selected from tumors associated with the expression of targets of the following group: HER2. For example, the tumor associated with the expression of the target includes a tumor with high expression of the target and/or a tumor positive for the target. For example, the tumor includes a solid tumor and/or a hematological tumor. For example, the tumor is selected from the group consisting of breast cancer, ovarian cancer, non-Hodgkin lymphoma, Hodgkin lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal cancer, gastric cancer, neuroglioma and mesothelioma.

The present application provides use of the compound of this application, or a tautomer, mesomer, racemate, enantiomer, and diastereomer thereof, or a mixture thereof, or a pharmaceutically acceptable salt, prodrug or solvate thereof, and/or the pharmaceutical composition of this application in the preparation of a drug for treating and/or preventing tumors. For example, the tumor is selected from tumors associated with the expression of targets of the following group: HER2. For example, the tumor associated with the expression of the target includes a tumor with high expression of the target and/or a tumor positive for the target. For example, the tumor includes a solid tumor and/or a hematological tumor. For example, the tumor is selected from the group consisting of breast cancer, ovarian cancer, non-Hodgkin lymphoma, Hodgkin lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal cancer, gastric cancer, neuroglioma and mesothelioma.

The present application provides a method for preventing and/or treating tumors, which can include administering to a subject the compound of this application, or a tautomer, mesomer, racemate, enantiomer, and diastereomer thereof, or a mixture thereof, or a pharmaceutically acceptable salt thereof, and/or the pharmaceutical composition of this application. For example, the tumor is selected from tumors associated with the expression of targets of the following group: HER2. For example, the tumor associated with the expression of the target includes a tumor with high expression of the target and/or a tumor positive for the target. For example, the tumor includes a solid tumor and/or a hematological tumor. For example, the tumor is selected from the group consisting of breast cancer, ovarian cancer, non-Hodgkin lymphoma, Hodgkin lymphoma, acute lymphocytic leukemia, anaplastic large cell lymphoma, multiple myeloma, prostate cancer, non-small cell lung cancer, small cell lung cancer, malignant melanoma, squamous cell carcinoma, glioblastoma, renal cell carcinoma, gastrointestinal tumors, pancreatic cancer, prostate cancer, colorectal cancer, gastric cancer, neuroglioma and mesothelioma.

Not to be limited by any theory, the following examples are only used to illustrate the compound of this application, preparation methods, uses thereof, etc., and are not intended to limit the scope of the invention of this application.

## Examples

### Example 1 Synthesis and preparation of compounds

The starting materials described in this application are commercially available products, or prepared by methods known in the art or prepared according to the methods described herein. Wherein, Fmoc is 9-fluorenylmethyloxycarbonyl protecting group, Boc is tert-butoxycarbonyl protecting group, and TBDMS/TBS is tert-butyldimethylsilyl protecting group.

### Synthesis of Compound (A-1)

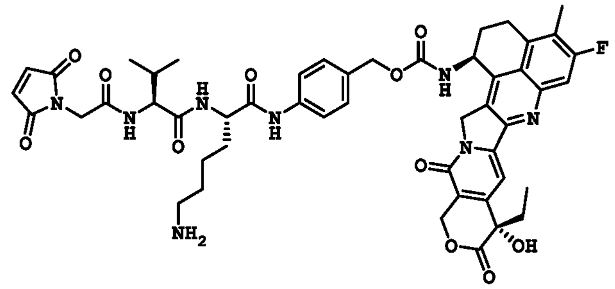

(A-1)

Synthetic route:

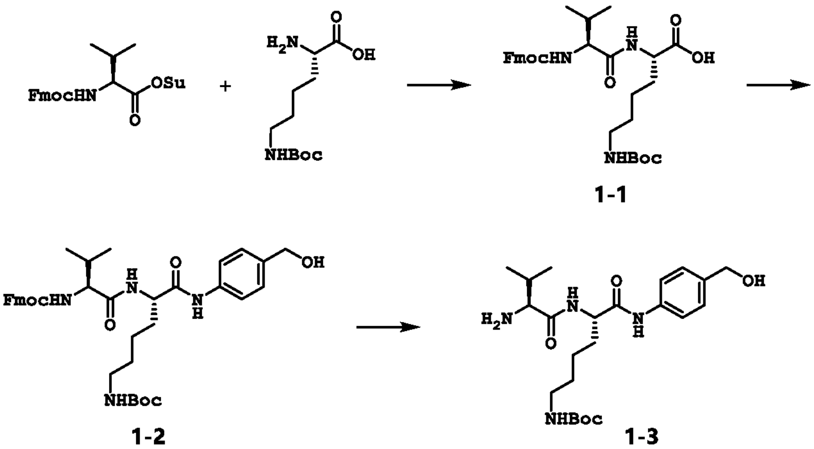

1-1

1-2

1-3

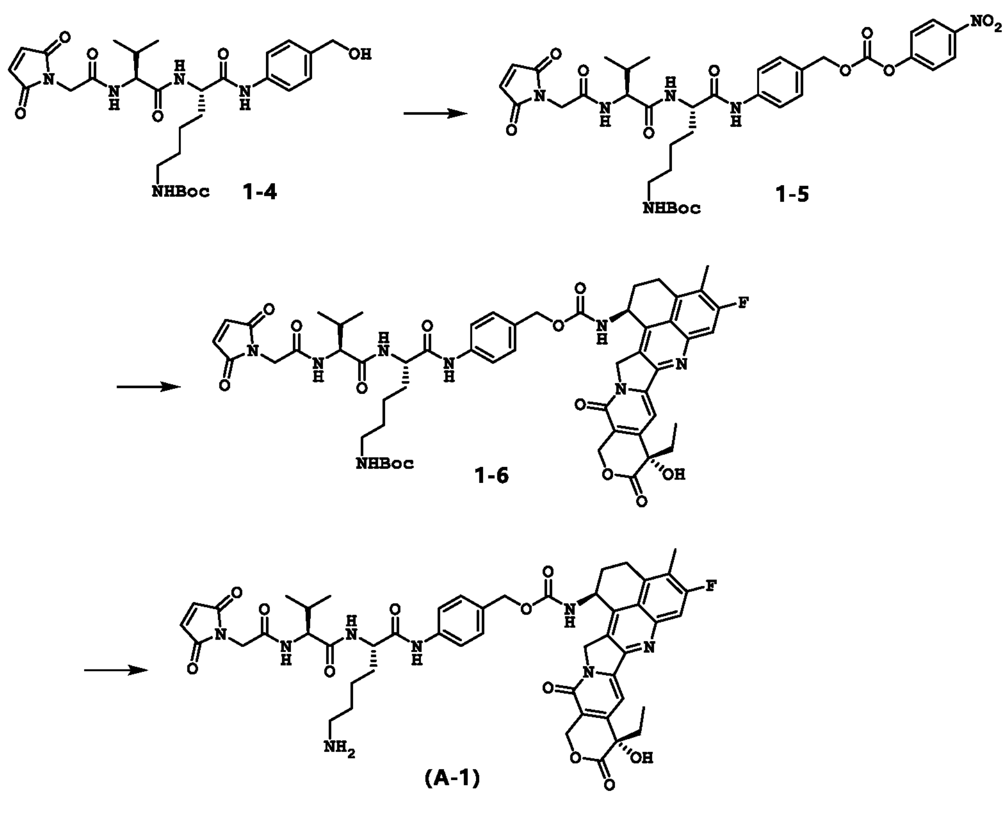

1-4

1-5

1-6

(A-1)

**Step 1: Synthesis of Intermediate 1-1**

Fmoc-Val-OSu (100 g, 229 mmol) was dissolved in 500 mL of tetrahydrofuran. Nε-(tert-butoxycarbonyl)-L-lysine (59.3 g, 241 mmol) and sodium bicarbonate (20.21 g, 241 mmol) were added into the 500 mL of aqueous solution, respectively. The reaction solution was stirred at room temperature for 48 hours, and the completion of the reaction was detected. The pH of the reaction solution was adjusted to about 6 with 1N dilute hydrochloric acid, and 500 ml of ethyl acetate was added for extraction. After separating the organic phase, it was washed once each with water and saturated brine, and then dried over anhydrous sodium sulfate and evaporated to dryness. The residue was recrystallized from methyl tert-butyl ether to give the product 1-1 (107 g, 82% yield) as a white solid. LC-MS (ESI, m/z) calculated: 567.29, found: 568.26 (M+H).

**Step 2: Synthesis of Intermediate 1-2**

Intermediate 1-1 (60 g, 106 mmol) was dissolved in a mixed solvent of dichloromethane and methanol (v:v = 2:1, 900 mL). p-aminobenzyl alcohol (19.52 g, 159 mmol) was added at room temperature followed by EEDQ (39.2 g, 159 mmol). The reaction solution was stirred at room temperature for 24 hours. The reaction solution was evaporated to dryness under reduced pressure, into which was added diethyl ether, and stirred until the solid was precipitated. After filtration, the solid was rinsed with diethyl ether for several times, and then dried to obtain Intermediate 1-2 (51 g, yield 72%). LC-MS (ESI, m/z) calculated: 672.35, found: 673.37 (M+H).

**Step 3: Synthesis of Intermediate 1-3**

Intermediate 1-2 (50 g, 74.3 mmol) was dissolved in 370 mL of DMF. Diethylamine (78 ml, 743 mmol) was added at room temperature. The reaction solution was stirred at room temperature for 2 hours. The reaction solution was evaporated to dryness under reduced pressure, into which were added ethyl acetate and diethyl ether, and stirred until the solid was precipitated. After filtration, the solid was rinsed with diethyl ether for several times, and then dried to obtain Intermediate 1-3 (31.5 g, yield 94%). LC-MS (ESI, m/z) calculated: 450.28, found: 451.32 (M+H).

**Step 4: Synthesis of Intermediate 1-4**

Intermediate 1-3 (5 g, 11.10 mmol) was dissolved in 100 ml of DMF, into which was added succinimide maleimidoacetate (2.80 g, 11.10 mmol) at room temperature. The reaction solution was stirred at room temperature overnight. The reaction solution was evaporated to dryness under reduced pressure, into which was added methyl tert-butyl ether, and stirred until the solid was precipitated. After filtration, the solid was rinsed with diethyl ether for several times, and then dried to obtain Intermediate 1-4, which was directly used in the next reaction. LC-MS (ESI, m/z) calculated: 587.30, found: 588.31 (M+H).

**Step 5: Synthesis of Intermediate 1-5**

Intermediate 1-4 (2 g, 3.40 mmol) was dissolved in 40 mL of DMF, into which were added DIPEA (1.189 ml, 6.81 mmol) and bis(4-nitrophenyl)carbonate (1.553 g, 5.10 mmol) at room temperature respectively. The mixture was stirred under argon at room temperature overnight, into which was added methyl tert-butyl ether, and stirred until the solid was precipitated. After filtration, the solid was rinsed with diethyl ether for several times, and then dried to obtain Intermediate 1-5, which was directly used in the next reaction. LC-MS (ESI, m/z) calculated: 752.30, found: 753.31 (M+H).

**Step 6: Synthesis of Intermediate 1-6**

Intermediate 1-5 (142 mg, 0.188 mmol) was dissolved in 400 μL of anhydrous DMF, into which was added 100 μL of anhydrous pyridine, and then were added Exatecan mesylate (available from Shanghai Haoyuan, 100 mg, 0.188 mmol) and HOBt (25.4 mg, 0.188 mmol). The reaction solution was stirred under argon at room temperature overnight, and prepared and purified by reverse-phase HPLC to obtain Intermediate 1-6 (80 mg, yield 40%). LC-MS (ESI, m/z) calculated: 1048.43, found: 1045.45 (M+H).

**Step 7: Synthesis of Compound (A-1)**

Intermediate 1-6 (100 mg, 0.095 mmol) was dissolved in 1 mL of anhydrous dichloromethane, into which was added 500 μL of trifluoroacetic acid in an ice bath, and then returned to room temperature and stirred for 30 minutes, and the solvent was removed under reduced pressure to obtain the final product, which was then prepared and purified by reverse-phase HPLC to obtain the final product compound (A-1) (70 mg, yield 77%). LC-MS (ESI, m/z) calculated: 1048.43, found: 1045.45 (M+H). LC-MS (ESI, m/z) calculated: 948.38, found: 949.37 (M+H).

**Synthesis of Compound (A-2)**

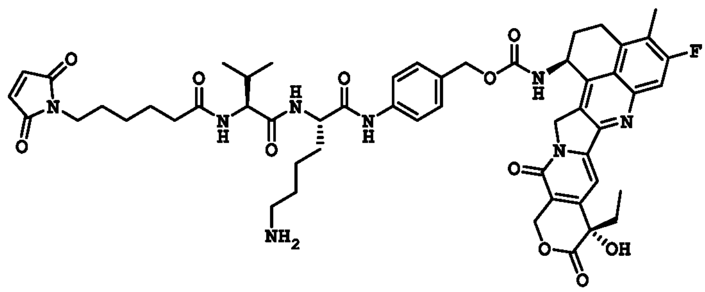

(A-2),

The synthesis of Compound (A-2) was the same as the synthesis steps of Compound (A-1), except that the raw material succinimide maleimidoacetate in step 4 was replaced with succinimidyl 6-(maleimido)hexanoate, and the product (A-2) was obtained after several steps of reaction as a beige amorphous powder. LC-MS (ESI, m/z) calculated: 1004.44, found: 1005.45 (M+H).

**Synthesis of Compound (A-3)**

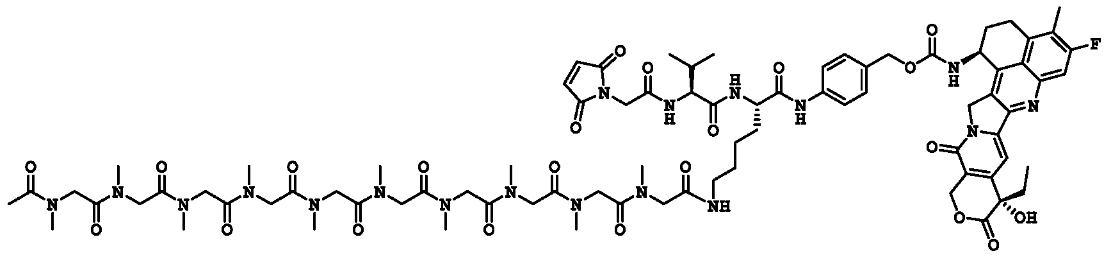

(A-3),

Compound (A-1) (100 mg, 0.105 mmol) was dissolved in 1 mL of anhydrous DMF, into which were added acetylated-10 polysarcosine (Ac-Sar10-COOH, 97 mg, 0.126 mmol), HATU (48 mg, 0.126 mmol) and DIPEA (37 μL, 0.211 mmol) respectively, and then stirred at room temperature overnight, and the solvent was removed under reduced pressure to obtain the final product, which was then prepared and purified by reverse-phase HPLC to obtain the final product compound (A-3) (87 mg, yield 49%). LC-MS (ESI, m/z) calculated: 1700.76, found: 1701.78 (M+H).

**Synthesis of Compound (A-4)**

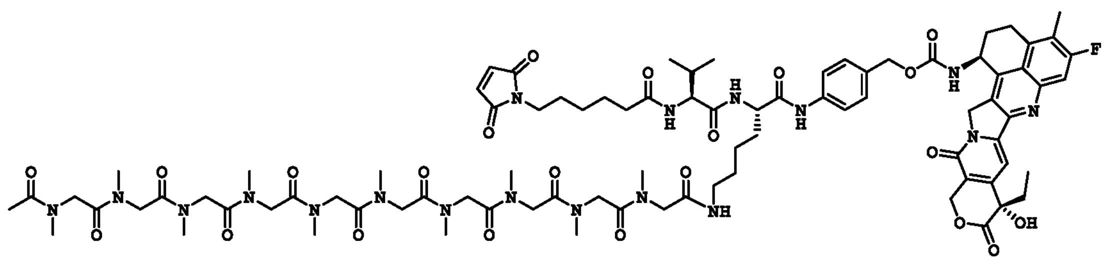

(A-4),

Compound (A-2) (100 mg, 0.099 mmol) was dissolved in 1 mL of anhydrous DMF, into which were added acetylated-10 polysarcosine (Ac-Sar10-COOH, 92 mg, 119 mmol), HATU (45mg, 119 mmol) and DIPEA (35 μL, 0.199 mmol) respectively, and then stirred at room temperature overnight, and the solvent was removed under reduced pressure to obtain the final product, which was then prepared and purified by reverse-phase HPLC to obtain the final product compound (A-4) (95 mg, yield 54%). LC-MS (ESI, m/z) calculated: 1756.83, found: 1757.85 (M+H).

**Synthesis of Compound (A-5)**

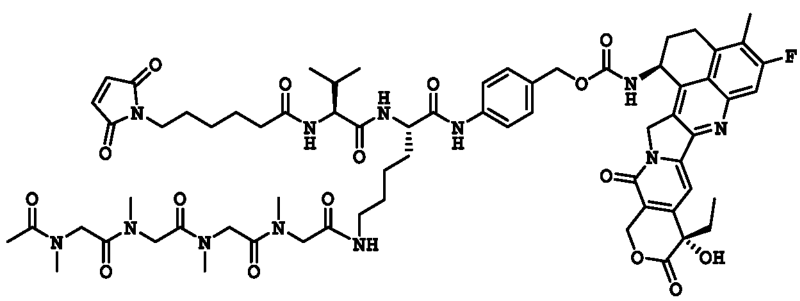

(A-5)

The synthesis of Compound (A-5) was the same as the synthesis steps of Compound (A-4), except that acetylated-10 polysarcosine (Ac-Sar10-COOH) in the last step is replaced with acetylated-4 polysarcosine (Ac-Sar4-COOH), and the product (A-5) was obtained after several steps of reaction as a beige amorphous powder. LC-MS (ESI, m/z) calculated: 1330.60, found: 1331.61 (M+H).

**Synthesis of Compound (A-6)**

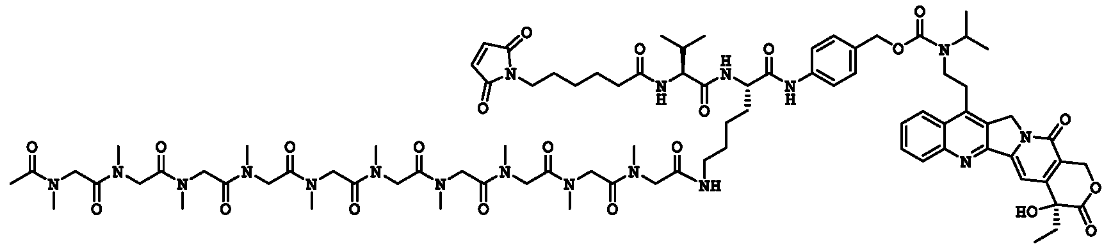

(A-6),

The synthesis of Compound (A-6) was the same as the synthesis steps of Compound (A-4), except that Exatecan mesylate in step 6 was replaced with Belotecan hydrochloride (available from Shanghai Haoyuan Chemicals), and the product (A-6) was obtained after several steps of reaction as a white amorphous powder. LC-MS (ESI, m/z) calculated: 1754.87, found: 1755.88 (M+H).

**Synthesis of Compound (A-7)**

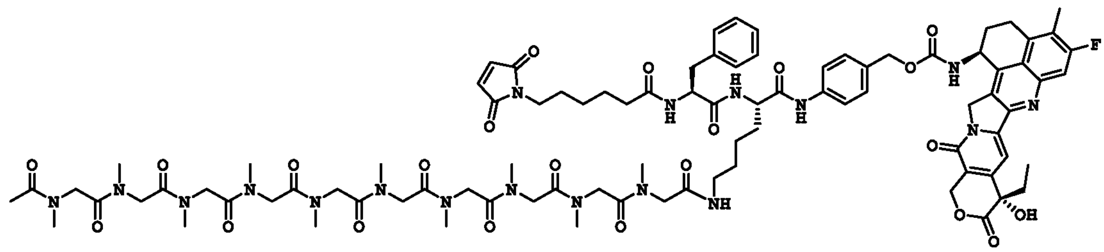

(A-7),

The synthesis of Compound (A-7) was the same as the synthesis steps of Compound (A-4), except that Fmoc-Val-OSu in step 1 was replaced with Fmoc-Phe-OSu, and the product (A-7) was obtained after several steps of reaction as a beige amorphous powder. LC-MS (ESI, m/z) calculated: 1804.83, found: 1805.85 (M+H).

**Synthesis of Compound (A-8)**

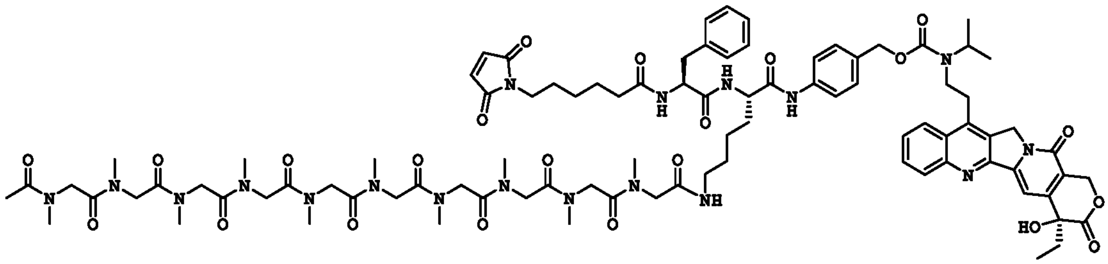

(A-8),

The synthesis of Compound (A-8) was the same as the synthesis steps of Compound (A-7), except that Exatecan mesylate in step 6 was replaced with Belotecan hydrochloride (available from Shanghai Haoyuan Chemicals), and the product (A-8) was obtained after several steps of reaction as a white amorphous powder. LC-MS (ESI, m/z) calculated: 1802.87, found: 1803.86 (M+H).

**Synthesis of Compound (A-9)**

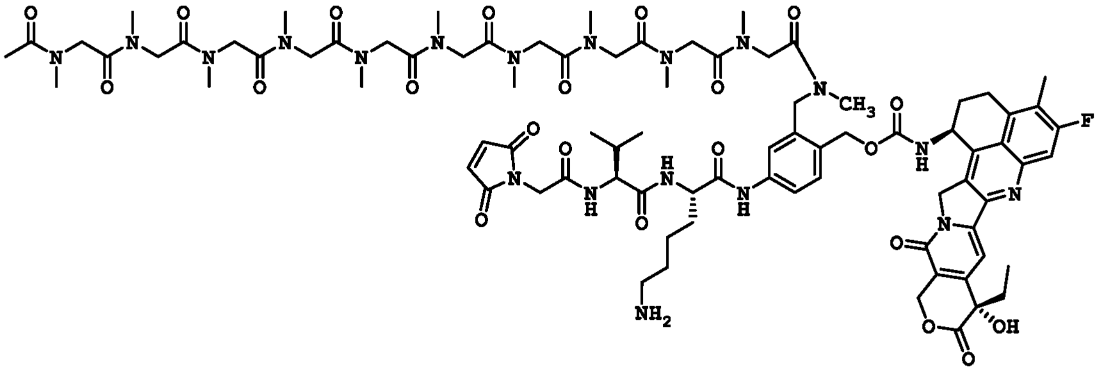

(A-9)

Synthetic route:

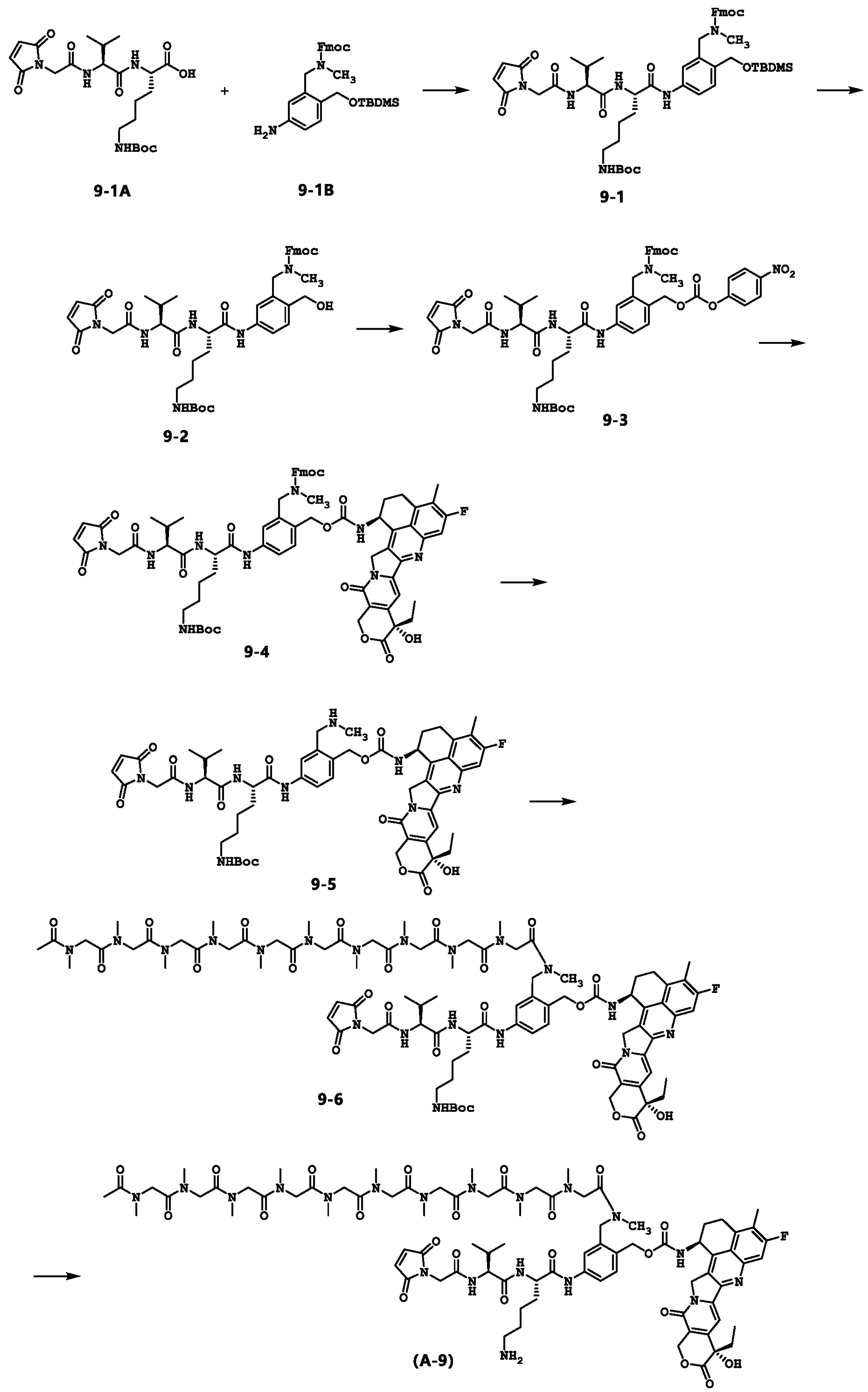

9-1A

9-1B

9-1

9-2

9-3

9-4

9-5

9-6

(A-9)

**Step 1: Synthesis of Intermediate 9-1**

Intermediates 9-1A (1.3 g, 2.69 mmol) and 9-1B (1.35 g, 2.69 mmol) were dissolved in a mixed solvent of dichloromethane and methanol (v:v = 2:1, 90 mL). p-EEDQ (800 mg, 3.23 mmol) was added at room temperature. The reaction solution was stirred at room temperature for 24 hours, and evaporated to dryness under reduced pressure, and 9-1 (1.8 g, yield 69%) was obtained by column chromatography. LC-MS (ESI, m/z) calculated: 966.49, found: 967.50 (M+H).

**Step 2: Synthesis of Intermediate 9-2**

Intermediate 9-1 (900 mg, 0.93 mmol) was dissolved in 20 mL of anhydrous tetrahydrofuran, and cooled in an ice bath under argon atmosphere, into which was added hydrofluoric acid-pyridine complex (1.8 g, 18.61 mmol). The reaction solution was stirred at 0°C for 2 hours, into which was added water to quench the reaction, and extracted with dichloromethane. The organic layer was separated, and dried over anhydrous sodium sulfate, and Intermediate 9-2 (610 mg, yield 77%) was obtained by column chromatography. LC-MS (ESI, m/z) calculated: 852.41, found: 853.43 (M+H).

**Step 3: Synthesis of Intermediate 9-3**

Intermediate 9-2 (600 mg, 0.703 mmol) was dissolved in 4 mL of anhydrous DMF, into which were added DIPEA (0.84 ml, 1.05 mmol) and bis(4-nitrophenyl)carbonate (321 mg, 1.05 mmol) at room temperature respectively. The mixture was stirred under argon at room temperature overnight, evaporated to remove the solvent under reduced pressure, into which was then added methyl tert-butyl ether, and stirred until the solid was precipitated. After filtration, the solid was rinsed with diethyl ether for several times, and then dried to obtain Intermediate 9-3, which was directly used in the next reaction. LC-MS (ESI, m/z) calculated: 1017.41, found: 1018.38 (M+H).

**Step 4: Synthesis of Intermediate 9-4**

Intermediate 9-3 (300 mg, 0.295 mmol) was dissolved in 400 μL of anhydrous DMF, into which was added 100 μL of anhydrous pyridine, and then were added Exatecan mesylate (available from Shanghai Haoyuan, 157 mg, 0.295 mmol) and HOBt (40 mg, 0.295 mmol). The reaction solution was stirred under argon at room temperature overnight, and prepared and purified by reverse-phase HPLC to obtain Intermediate 9-4 (160 mg, yield 41%). LC-MS (ESI, m/z) calculated: 1313.54, found: 1314.51 (M+H).

**Step 5: Synthesis of Intermediate 9-5**

Intermediate 9-4 (150 mg, 0.114 mmol) was dissolved in 1 mL of DMF. Diethylamine (120 μL, 1.14 mmol) was added at room temperature. The reaction solution was stirred at room temperature for 2 hours, and evaporated to dryness under reduced pressure to obtain Intermediate 9-5, which was directly used in the next reaction. LC-MS (ESI, m/z) calculated: 1091.48, found: 1092.51 (M+H).

**Step 6: Synthesis of Intermediate 9-6**

Compound 9-5 (120 mg, 0.110 mmol) was dissolved in 1 mL of anhydrous DMF, into which were added acetylated-10 polysarcosine (Ac-Sar10-COOH, 102 mg, 0.132 mmol), HATU (50 mg, 0.132 mmol) and DIPEA (38 μL, 0.22 mmol) respectively, and then stirred at room temperature overnight. The solvent was removed under reduced pressure to obtain the final product, which was then prepared and purified by reverse-phase HPLC to obtain Intermediate Compound 9-6 (105 mg, yield 52%). LC-MS (ESI, m/z) calculated: 1843.86, found: 1844.84 (M+H).

**Step 7: Synthesis of Compound (A-9)**

Intermediate 9-6 (100 mg, 0.054 mmol) was dissolved in 1 mL of anhydrous dichloromethane, into which was added 500 μL of trifluoroacetic acid in an ice bath, and then returned to room temperature and stirred for 30 minutes. The solvent was removed under reduced pressure to obtain the final product, which was then prepared and purified by reverse-phase HPLC to obtain the final product compound (A-9) (57 mg, yield 60%). LC-MS (ESI, m/z) calculated: 1048.43, found: 1045.45 (M+H). LC-MS (ESI, m/z) calculated: 1743.81, found: 1744.85 (M+H).

**Synthesis of Compound (A-10)**

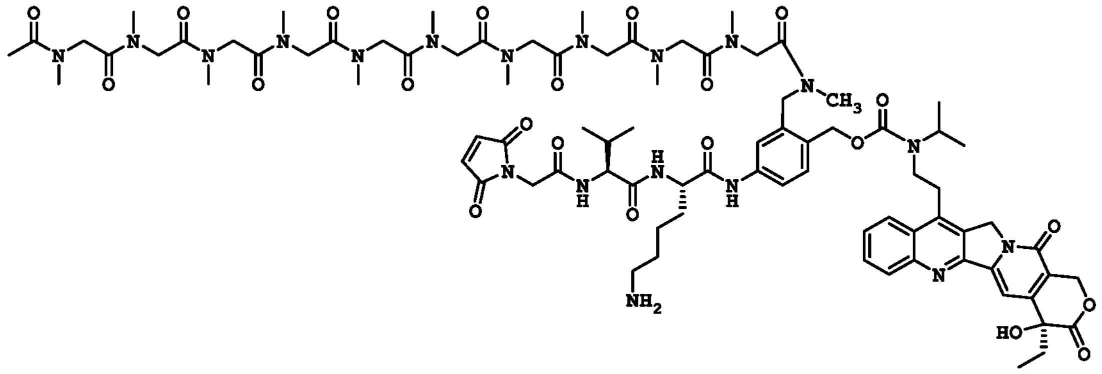

(A-10),

The synthesis of Compound (A-10) was the same as the synthesis steps of Compound (A-9), except that Exatecan mesylate in step 4 was replaced with Belotecan hydrochloride (available from Shanghai Haoyuan Chemicals), and the product (A-10) was obtained after several steps of reaction as a white amorphous powder. LC-MS (ESI, m/z) calculated: 1741.85, found: 1742.83 (M+H).

**Synthesis of Compound (A-11)**

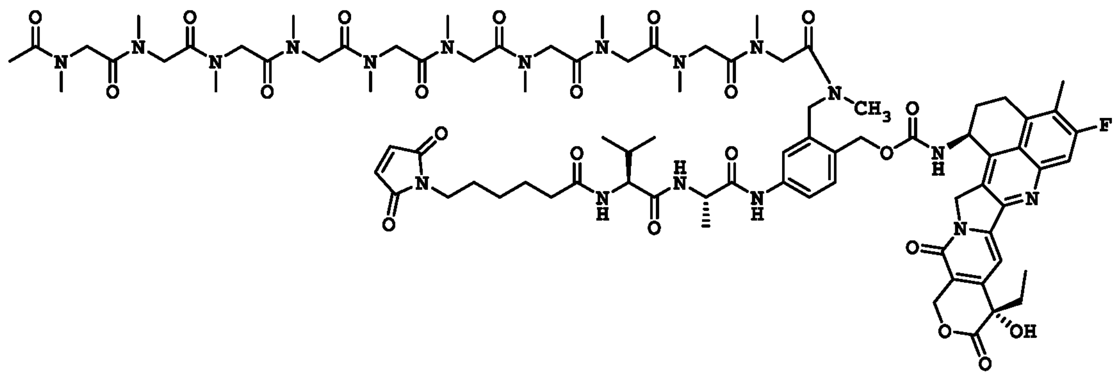

(A-11)

Synthetic route:

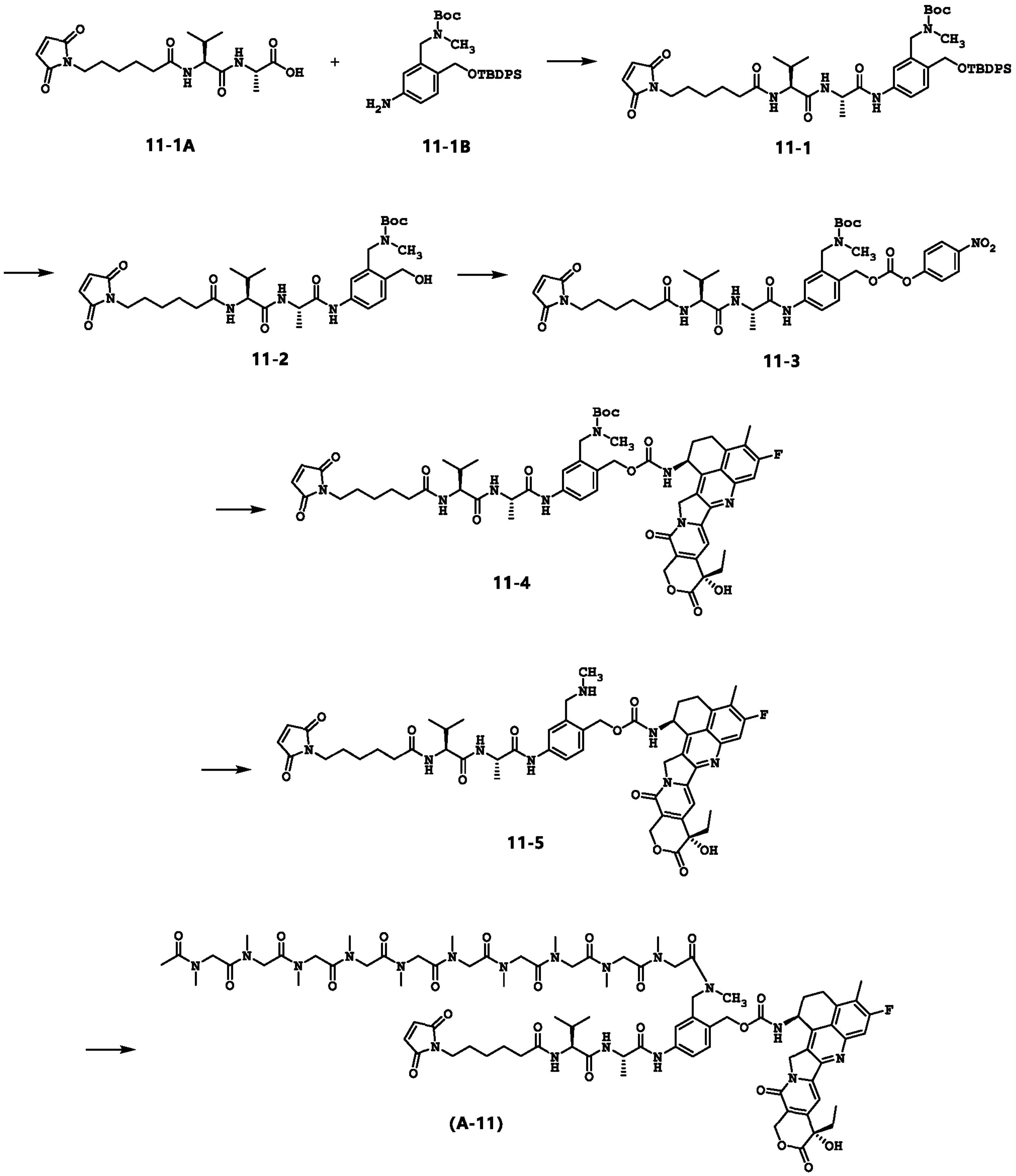

11-1A 11-1B 11-1

11-2 11-3

11-4

11-5

(A-11)

**Step 1: Synthesis of Intermediate 11-1**

Intermediates 11-1A (Mc-Val-Ala-OH, available from Shanghai Haoyuan Chemicals, 2.4 g, 6.29 mmol) and 11-1B (3.18 g, 6.29 mmol) were dissolved in a mixed solvent of dichloromethane and methanol (v:v = 2:1, 90 mL). p-EEDQ (1.86 g, 7.55 mmol) was added at room temperature. The reaction solution was stirred at room temperature for 24 hours, and evaporated to dryness under reduced pressure, and 11-1 (3.9 g, yield 71%) was obtained by column chromatography. LC-MS (ESI, m/z) calculated: 867.46, found: 868.49 (M+H).

**Step 2: Synthesis of Intermediate 11-2**

Intermediate 11-1 (2 g, 2.3 mmol) was dissolved in 50 mL of anhydrous tetrahydrofuran, and cooled in an ice

bath under argon atmosphere, into which was added hydrofluoric acid-pyridine complex (4.6 g, 46 mmol). The reaction solution was stirred at 0°C for 2 hours, into which was added water to quench the reaction, and extracted with dichloromethane. The organic layer was separated, and dried over anhydrous sodium sulfate, and Intermediate 11-2 (1.1 g, yield 76%) was obtained by column chromatography. LC-MS (ESI, m/z) calculated: 629.34, found: 630.31 (M+H).

**Step 3: Synthesis of Intermediate 11-3**

Intermediate 11-2 (700 mg, 1.11 mmol) was dissolved in 4 mL of anhydrous DMF, into which were added DIPEA (0.39 ml, 2.23 mmol) and bis(4-nitrophenyl)carbonate (406 mg, 1.33 mmol) at room temperature respectively. The mixture was stirred under argon at room temperature overnight, evaporated to remove the solvent under reduced pressure, into which was then added methyl tert-butyl ether, and stirred until the solid was precipitated. After filtration, the solid was rinsed with diethyl ether for several times, and then dried to obtain Intermediate 11-3, which was directly used in the next reaction. LC-MS (ESI, m/z) calculated: 794.35, found: 795.41 (M+H).

**Step 4: Synthesis of Intermediate 11-4**

Intermediate 11-3 (300 mg, 0.44 mmol) was dissolved in 400 μL of anhydrous DMF, into which was added 100 μL of anhydrous pyridine, and then were added Exatecan mesylate (available from Shanghai Haoyuan, 234 mg, 0.44 mmol) and HOBt (60 mg, 0.44 mmol). The reaction solution was stirred under argon at room temperature overnight, and prepared and purified by reverse-phase HPLC to obtain Intermediate 11-4 (230 mg, yield 48%). LC-MS (ESI, m/z) calculated: 1090.48, found: 1091.53 (M+H).

**Step 5: Synthesis of Intermediate 11-5**

Intermediate 11-4 (200 mg, 0.183 mmol) was dissolved in 1 mL of anhydrous dichloromethane, into which was added 300 μL of trifluoroacetic acid in an ice bath, and then returned to room temperature and stirred for 30 minutes, and the solvent was removed under reduced pressure to obtain the final product trifluoroacetate salt of Intermediate 11-5, which was directly used in the next reaction without further purification. LC-MS (ESI, m/z) calculated: 990.43, found: 991.47 (M+H).

**Step 6: Synthesis of Compound (A-11)**

Compound 11-5 (120 mg, 0.109 mmol) was dissolved in 1 mL of anhydrous DMF, into which were added acetylated-10 polysarcosine (Ac-Sar10-COOH, 84 mg, 0.109 mmol), HATU (50 mg, 0.130 mmol) and DIPEA (38 μL, 0.22 mmol) respectively, and then stirred at room temperature overnight, and the solvent was removed under reduced pressure to obtain the final product, which was then prepared and purified by reverse-phase HPLC to obtain Compound (A-11) (74 mg, yield 38%). LC-MS (ESI, m/z) calculated: 1742.81, found: 1743.85 (M+H).

**Synthesis of Compound (A-12)**

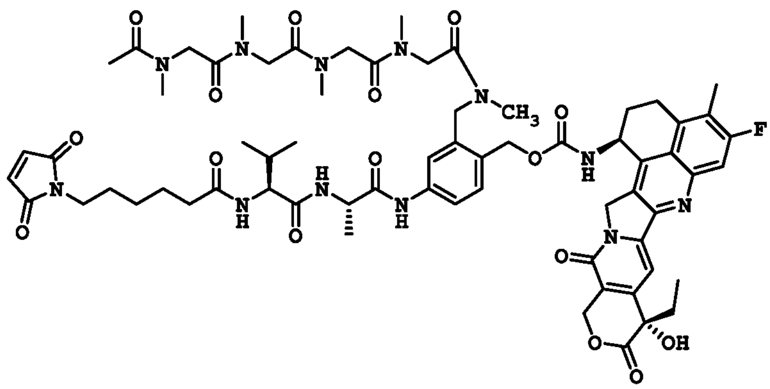

(A-12)

The synthesis of Compound (A-12) was the same as the synthesis steps of Compound (A-11), except that the raw compound Ac-Sar10-COOH in step 6 was replaced with Ac-Sar4-COOH, and the product (A-12) was obtained after

several steps of reaction as a beige amorphous powder. LC-MS (ESI, m/z) calculated: 1316.59, found: 1317.62 (M+H).

**Synthesis of Compound (A-13)**

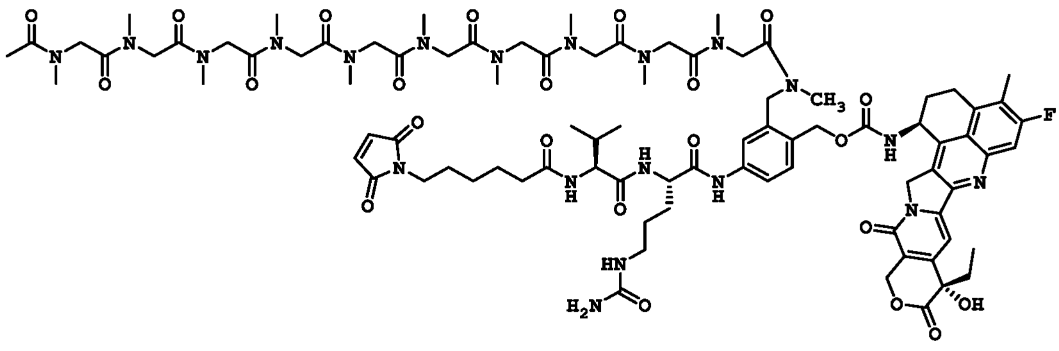

(A-13)

The synthesis of Compound (A-13) was the same as the synthesis steps of Compound (A-11), except that the raw compound 11-1A in step 1 was replaced with Mc-Val-Cit-OH (available from Shanghai Haoyuan Chemicals), and the product (A-13) was obtained after several steps of reaction as a beige amorphous powder. LC-MS (ESI, m/z) calculated: 1828.86, found: 1829.88 (M+H).

**Synthesis of Compound (A-14)**

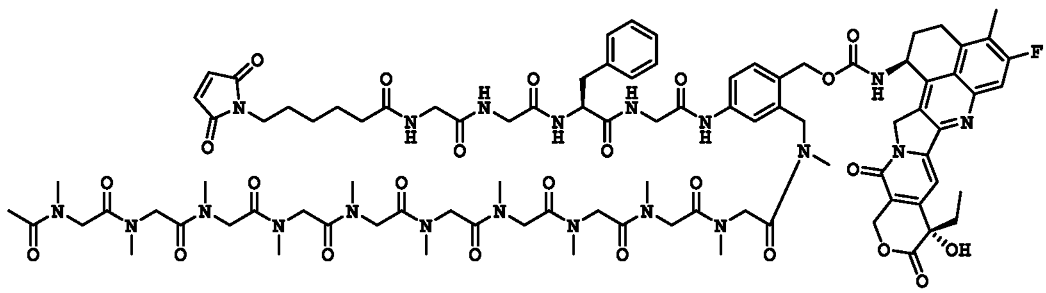

(A-14)

The synthesis of Compound (A-14) was the same as the synthesis steps of Compound (A-11), except that the raw compound 11-1A (Mc-VA-OH) in step 1 was replaced with Mc-GGFG-OH (available from Shanghai Haoyuan Chemicals), and the product (A-13) was obtained after several steps of reaction as a beige amorphous powder. LC-MS (ESI, m/z) calculated: 1890.84, found: 1891.90 (M+H).

**Synthesis of Compound (A-15)**

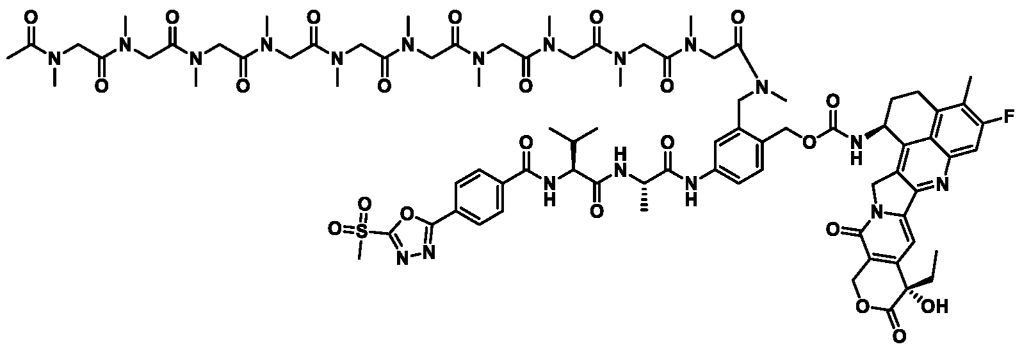

(A-15)

Synthetic route:

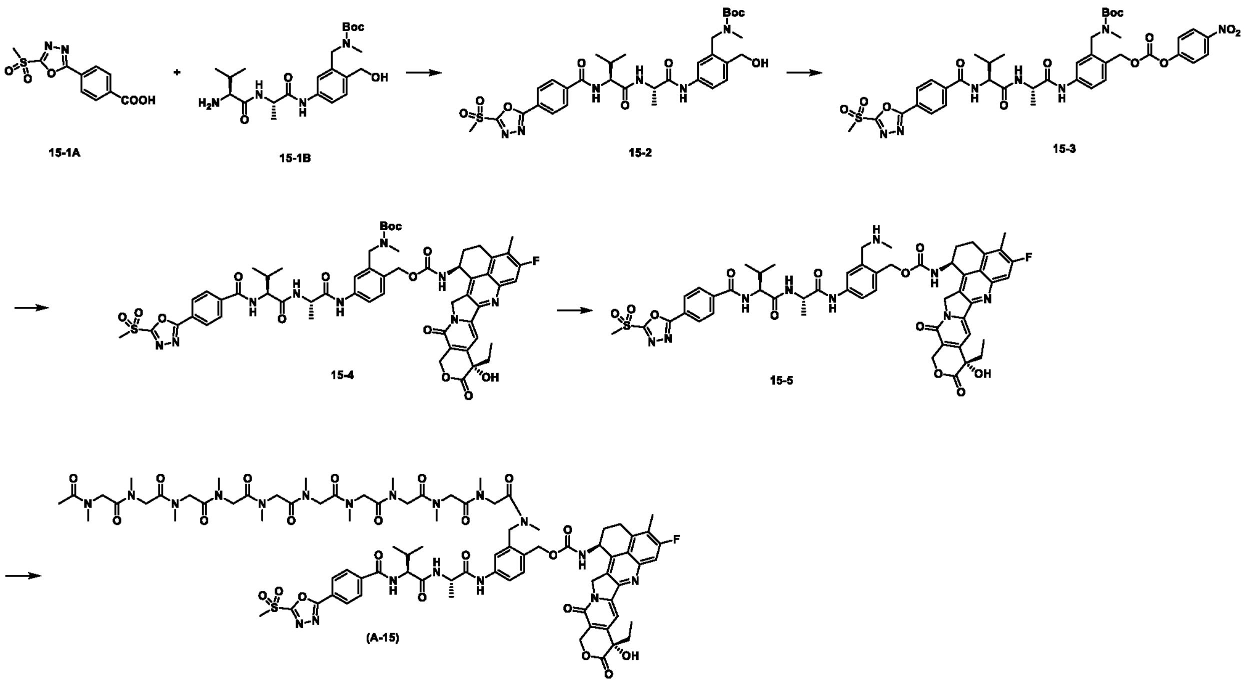


**Step 1: Synthesis of Intermediate 15-2**

Intermediates 15-1A (2.3 g, 8.57 mmol) and 15-1B (3.74 g, 8.57 mmol) were dissolved in a mixed solvent of dichloromethane and methanol (v:v = 2:1, 90 mL). p-EEDQ (4.24 g, 17.15 mmol) was added at room temperature. The reaction solution was stirred at room temperature for 24 hours, and evaporated to dryness under reduced pressure, and Intermediate 15-2 (3.23 g, 4.70 mmol, 54.9% yield) was obtained by column chromatography. LC-MS (ESI, m/z) calculated: 686.27, found: 687.21 (M+H).

**Step 2: Synthesis of Intermediate 15-3**

Intermediate 15-2 (2.3 g, 3.35 mmol) was dissolved in 40 mL of anhydrous DMF, into which were added DIPEA (1.170 ml, 6.70 mmol) and bis(4-nitrophenyl)carbonate (1.528 g, 5.02 mmol) at room temperature respectively. The mixture was stirred under argon at room temperature overnight, evaporated to remove the solvent under reduced pressure, into which was then added methyl tert-butyl ether, and stirred until the solid was precipitated. After filtration, the solid was rinsed with diethyl ether for several times, and then dried to obtain Intermediate 15-3 (2.1 g, 2.465 mmol, yield 73.6 %), which was directly used in the next reaction.

**Step 3: Synthesis of Intermediate 15-4**

Intermediate 15-3 (400 mg, 0.470 mmol) was dissolved in 4 mL of anhydrous DMF, into which was added 1 mL of anhydrous pyridine, and then were added Exatecan free base (available from Shanghai Haoyuan Chemicals) (204 mg, 0.470 mmol) and HOBt (63.4 mg, 0.470 mmol). The reaction solution was stirred under argon at room temperature overnight, evaporated to dryness, and then purified by column chromatography (DCM:MeOH=30:1) to obtain Intermediate 15-4 (244 mg, 0.213 mmol, yield 45.3%). LC-MS (ESI, m/z) calculated: 1147.41, found: 1148.48 (M+H).

**Step 4: Synthesis of Intermediate 15-5**

Intermediate 15-4 (200 mg, 0.183 mmol) was dissolved in 1 mL of anhydrous dichloromethane, into which was added 300 μL of trifluoroacetic acid in an ice bath, and then returned to room temperature and stirred for 30 minutes, and the solvent was removed under reduced pressure to obtain the final product trifluoroacetate salt of Intermediate 15-5, which was directly used in the next reaction without further purification. LC-MS (ESI, m/z) calculated: 1047.36, found: 1048.40 (M+H).

**Step 5: Synthesis of Compound A-15**

Intermediate Compound 15-5 (150 mg, 0.131 mmol) was dissolved in 1 mL of anhydrous DMF, into which were added acetylated-10 polysarcosine (Ac-Sar10-COOH) (101 mg, 0.131 mmol), DIPEA (114 μl, 0.654 mmol) and HATU (74.6 mg, 0.196 mmol) respectively, and then stirred at room temperature overnight, and the solvent was removed under reduced pressure to obtain the final product, which was then prepared and purified by reverse-phase HPLC to obtain Compound A-15 (107 mg, 0.059 mmol, yield 45.4 %). LC-MS (ESI, m/z) calculated: 1799.74, found: 1800.77 (M+H).

**Synthesis of Compound (A-16)**

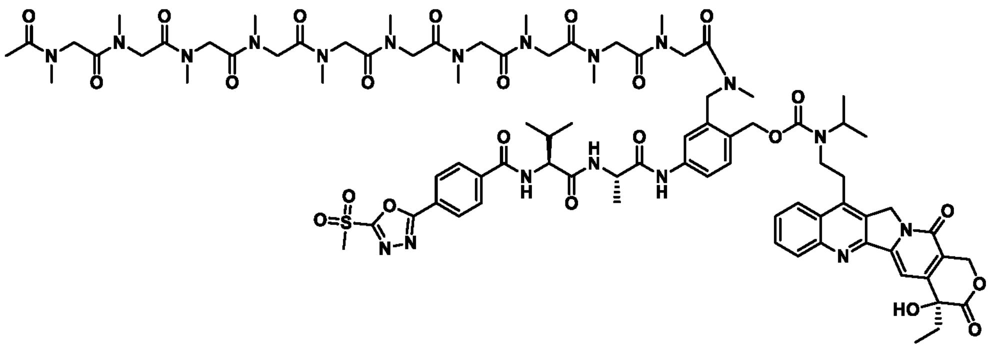

(A-16)

The synthesis of Compound (A-16) was the same as the synthesis steps of Compound (A-15), except that Exatecan mesylate in step 3 was replaced with Belotecan hydrochloride (available from Shanghai Haoyuan Chemicals), and the product (A-16) was obtained after several steps of reaction as a beige amorphous powder. LC-MS (ESI, m/z) calculated: 1797.78, found: 1798.81 (M+H).

**Synthesis of Compound (A-17)**

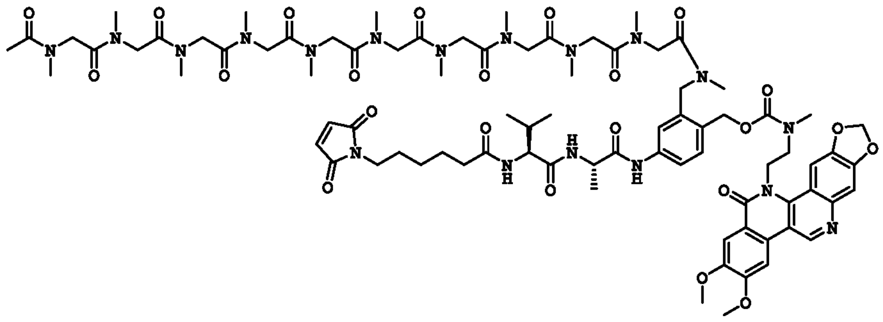

(A-17)

The synthesis of Compound (A-17) was the same as the synthesis steps of Compound (A-11), except that Exatecan mesylate in step 4 was replaced with Genz-644282 (available from Shanghai Haoyuan Chemicals), and the product (A-17) was obtained after several steps of reaction as a beige amorphous powder. LC-MS (ESI, m/z) calculated: 1714.80, found: 1715.83 (M+H).

**Synthesis of Compound (A-18)**

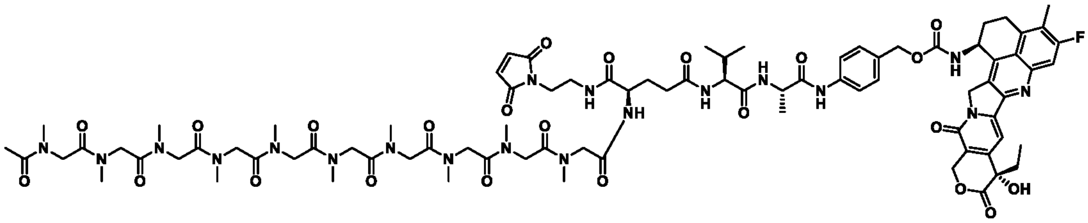

(A-18)

Synthetic route:

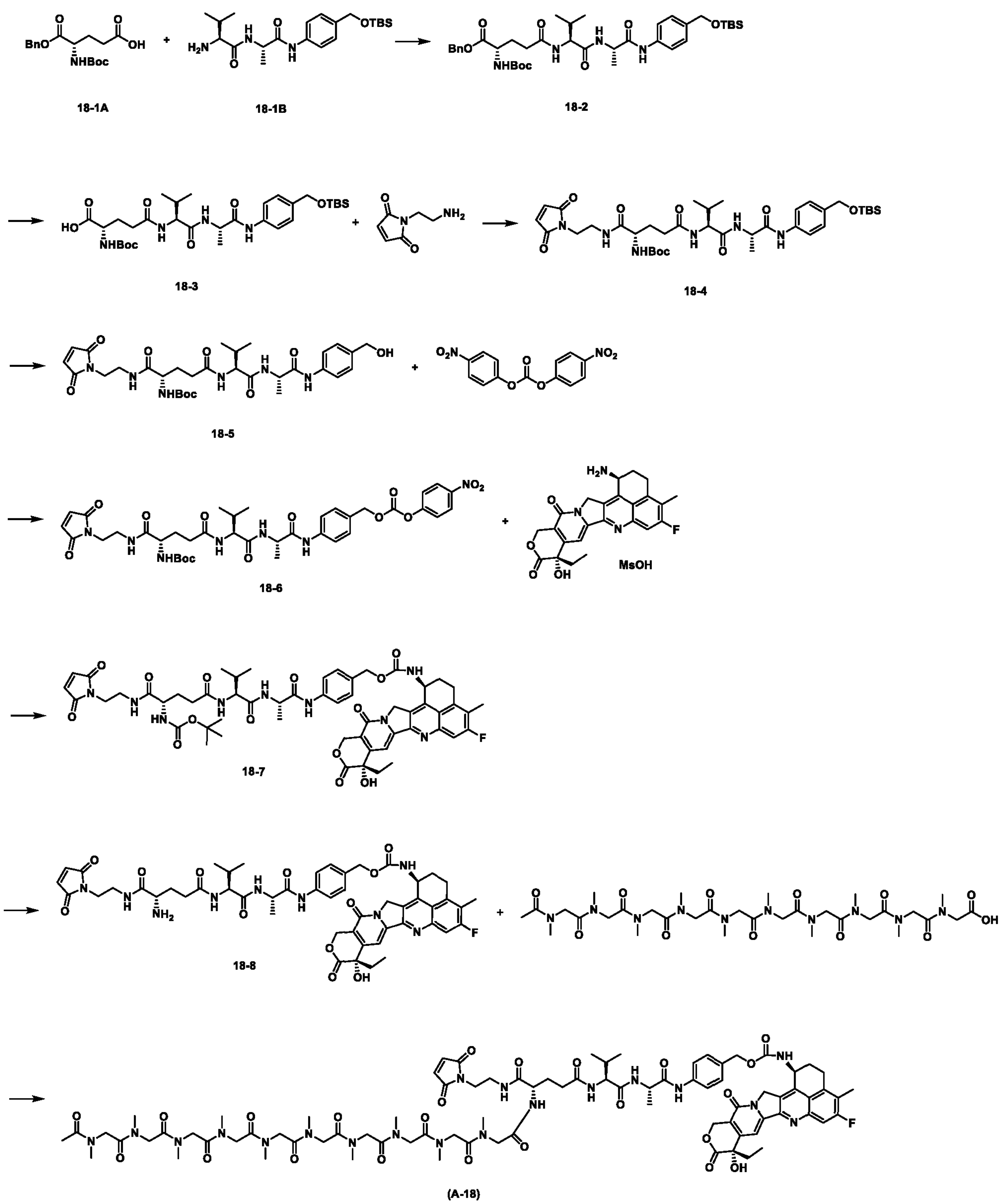

18-1A 18-1B 18-2

18-3 18-4

18-5

18-6

18-7

18-8

(A-18)

### Step 1: Synthesis of Intermediate 18-2

Intermediates 18-1A (Shanghai Bidepharm, 4.94 g, 14.82 mmol) and 15-1B (synthesized according to the method in WO2016038383, 6.04 g, 14.82 mmol) were dissolved in a mixed solvent of dichloromethane and methanol (v:v = 2:1, 120 mL). p-EEDQ (7.33 g, 29.6 mmol) was added at room temperature. The reaction solution was stirred at room temperature for 24 hours, and evaporated to dryness under reduced pressure to obtain the product, which was stirred in the presence of diethyl ether and filtered, and then dried in vacuum to obtain Intermediate 18-2 (6.3 g, 59% yield). LC-MS (ESI, m/z) calculated: 726.40, found: 727.41 (M+H).

**Step 2: Synthesis of Intermediate 18-3**

Intermediate 18-2 (3.0 g, 4.13 mmol) was dissolved in 50 ml of THF, into which was added 10% Pd-C (800 mg). After 8 hours of hydrogenation at normal pressure, the completion of the reaction was detected by TLC, and the palladium carbon was removed by filtration. After evaporation to dryness, the reaction solution was directly used in the next reaction. LC-MS (ESI, m/z) calculated: 636.36, found: 637.34 (M+H).

**Step 3: Synthesis of Intermediate 18-4**

Intermediate Compound 18-3 (2.6 g, 4.08 mmol) was dissolved in 100 mL of anhydrous DMF, into which were added N-(2-aminoethyl)maleimide hydrochloride (721 mg, 4.08 mmol), DIPEA (1.42 mL, 8.17 mmol) and HATU (2.0 g, 5.31 mmol) respectively, and then stirred at room temperature overnight, and the solvent was removed under reduced pressure to obtain the final product, which was then purified by column chromatography to obtain Compound 18-4 (2.2 g, 71% yield). LC-MS (ESI, m/z) calculated: 758.40, found: 759.42 (M+H).

**Step 4: Synthesis of Intermediate 18-5**

Intermediate 18-4 (1.0 g, 1.32 mmol) was dissolved in 20 mL of anhydrous tetrahydrofuran, and cooled in an ice bath under argon atmosphere, into which was added hydrofluoric acid-pyridine complex (2.6 g, 26.4 mmol). The reaction solution was stirred at 0°C for 2 hours, into which was added water to quench the reaction, and extracted with dichloromethane. The organic layer was separated, and dried over anhydrous sodium sulfate, and Intermediate 18-5 (570 mg, yield 67%) was obtained by column chromatography. LC-MS (ESI, m/z) calculated: 644.32, found: 645.35 (M+H).

**Step 5: Synthesis of Intermediate 18-6**

Intermediate 18-5 (500 mg, 0.776 mmol) was dissolved in 10 mL of anhydrous DMF, into which were added DIPEA (271 μl, 1.55 mmol) and bis(4-nitrophenyl)carbonate (236 mg, 0.776 mmol) at room temperature respectively. The mixture was stirred under argon at room temperature overnight, evaporated to remove the solvent under reduced pressure, into which was then added methyl tert-butyl ether, and stirred until the solid was precipitated. After filtration, the solid was rinsed with diethyl ether for several times, and then dried to obtain Intermediate 18-6 (530 mg, 84% yield), which was directly used in the next reaction.

**Step 6: Synthesis of Intermediate 18-7**

Intermediate 18-6 (400 mg, 0.494 mmol) was dissolved in 4 mL of anhydrous DMF, into which was added 1 mL of anhydrous pyridine, and then were added Exatecan mesylate (available from Shanghai Haoyuan, 263 mg, 0.494 mmol) and HOBt (66.7 mg, 0.494 mmol). The reaction solution was stirred under argon at room temperature overnight, and prepared and purified by reverse-phase HPLC to obtain Intermediate 18-7 (320 mg, yield 58%). LC-MS (ESI, m/z) calculated: 1105.46, found: 1106.48 (M+H).

**Step 7: Synthesis of Intermediate 18-8**

Intermediate 15-4 (300 mg, 0.27 mmol) was dissolved in 3 mL of anhydrous dichloromethane, into which was added 1 mL of trifluoroacetic acid in an ice bath, and then returned to room temperature and stirred for 30 minutes, and the solvent was removed under reduced pressure to obtain the final product trifluoroacetate salt of Intermediate 18-8, which was directly used in the next reaction without further purification. LC-MS (ESI, m/z) calculated: 1005.40, found: 1006.41 (M+H).

**Step 8: Synthesis of Compound A-18**

Intermediate Compound 15-5 (300 mg, 0.268 mmol) was dissolved in 5 mL of anhydrous DMF, into which were added acetylated-10 polysarcosine (Ac-Sar10-COOH) (206 mg, 0.268 mmol), DIPEA (94 μl, 0.536 mmol) and HATU (122 mg, 0.321 mmol) respectively, and then stirred at room temperature overnight, and the solvent was removed under reduced pressure to obtain the final product, which was then prepared and purified by reverse-phase HPLC to obtain Compound A-18 (170 mg, 36% yield). LC-MS (ESI, m/z) calculated: 1757.78, found: 1758.82 (M+H).

**Synthesis of Compound (A-19)**

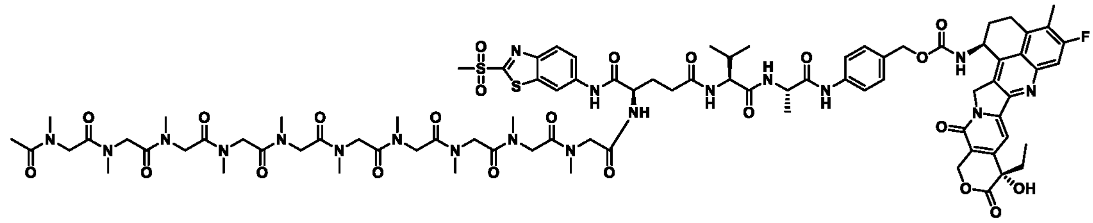

(A-19)

The synthesis of Compound (A-19) was the same as the synthesis steps of Compound (A-18), except that N-(2-aminoethyl)maleimide hydrochloride in step 3 was replaced with 2-(methylsulfonyl)benzo[d]thiazol-6-amine, and the product (A-19) was obtained after several steps of reaction. LC-MS (ESI, m/z) calculated: 1845.73, found: 1846.75 (M+H).

**Synthesis of Compound (A-20)**

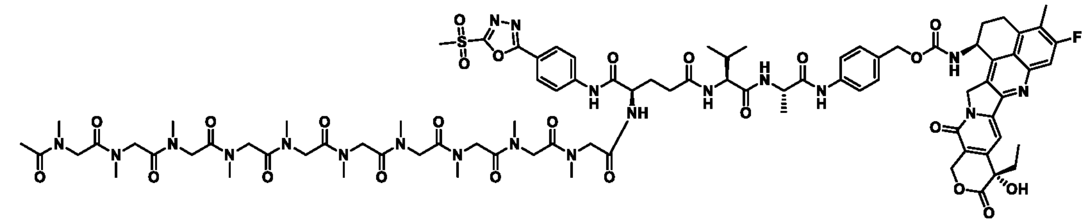

(A-20)

The synthesis of Compound (A-20) was the same as the synthesis steps of Compound (A-18), except that N-(2-aminoethyl)maleimide hydrochloride in step 3 was replaced with 4-(5-(methylsulfonyl)-1,3,4-oxadiazol-2-yl)aniline, and the product (A-20) was obtained after several steps of reaction. LC-MS (ESI, m/z) calculated: 1856.76, found: 1857.80 (M+H).

**Synthesis of Compound (A-21)**

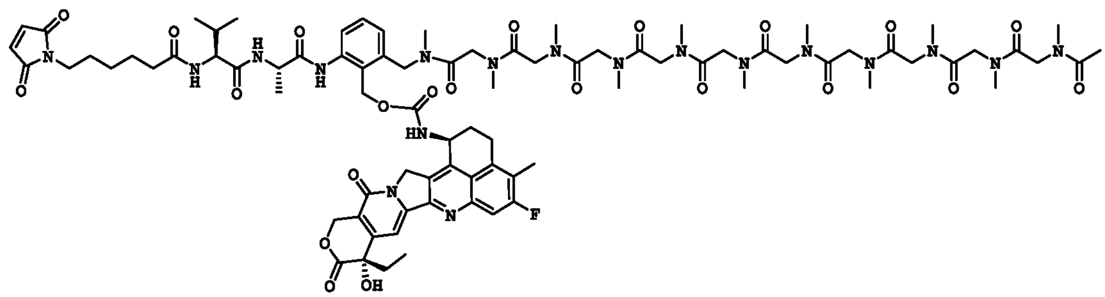

(A-21)

The synthesis of Compound (A-21) was the same as the synthesis steps of Compound (A-11), except that Intermediate

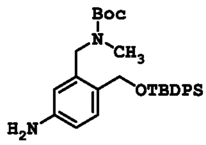

(11-1B)

in step 1 was replaced with

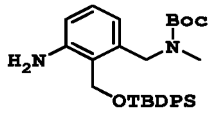

(21-1B),

and the product (A-21) was obtained after several steps of reaction. LC-MS (ESI, m/z) calculated: 1742.81, found: 1743.85 (M+H).

**Synthesis of Compound (A-22)**

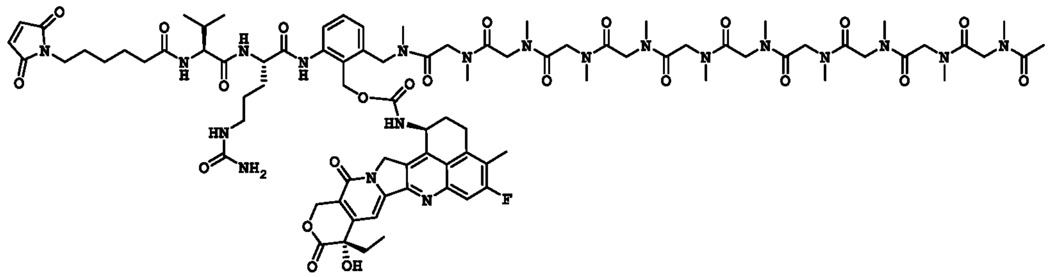

(A-22)

The synthesis of Compound (A-22) was the same as the synthesis steps of Compound (A-21), except that the raw compound Mc-Val-Ala-OH in step 1 was replaced with Mc-Val-Cit-OH, and the product (A-22) was obtained after several steps of reaction as a beige amorphous powder. LC-MS (ESI, m/z) calculated: 1828.86, found: 1829.87 (M+H).

**Synthesis of Compound (A-23)**

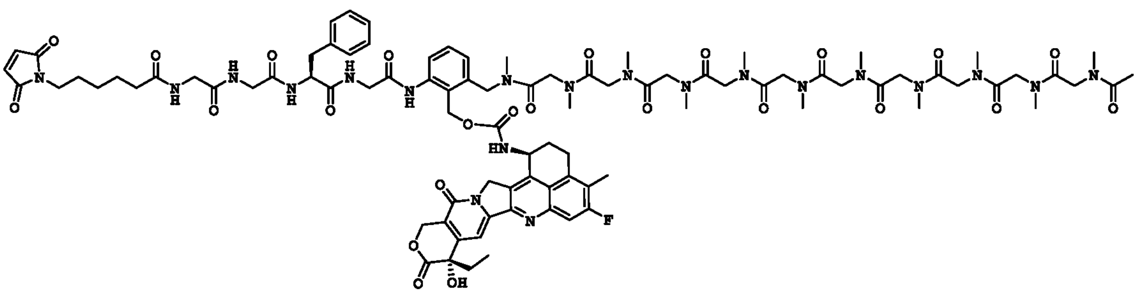

(A-23)

The synthesis of Compound (A-23) was the same as the synthesis steps of Compound (A-21), except that the raw compound Mc-Val-Ala-OH in step 1 was replaced with Mc-GGFG-OH, and the product (A-23) was obtained after several steps of reaction as a beige amorphous powder. LC-MS (ESI, m/z) calculated: 1890.84, found: 1891.86 (M+H).

**Synthesis of Compound (A-28)**

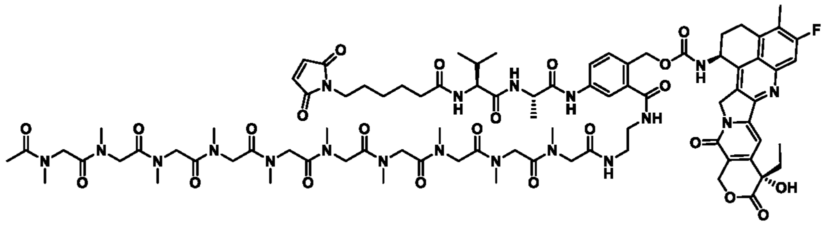

(A-28)

Synthetic route:

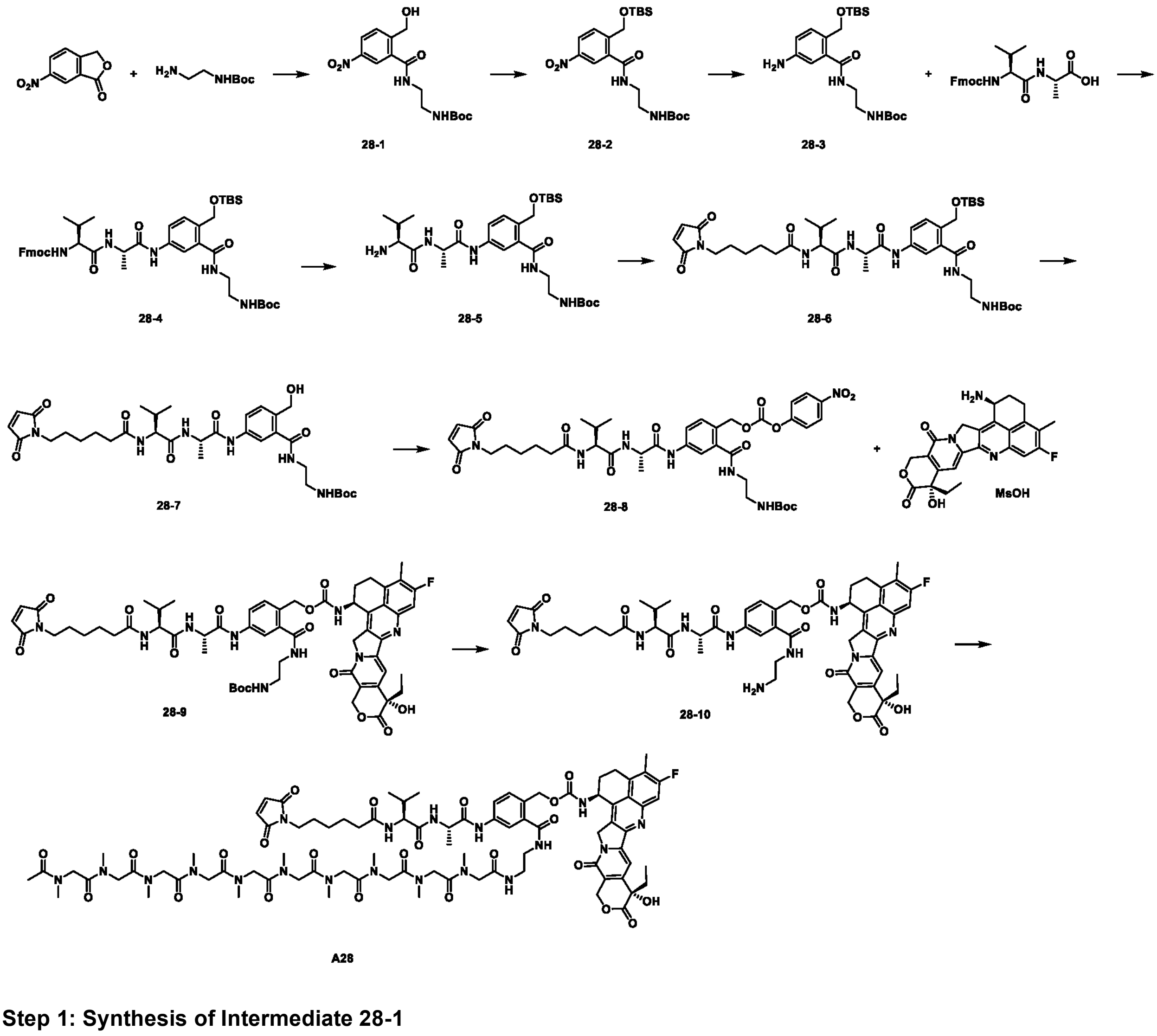

28-1 28-2 28-3 28-4 28-5 28-6 28-7 28-8 28-9 28-10 A28

**Step 1: Synthesis of Intermediate 28-1**

6-nitroisobenzofuran-1(3*H*)-one (10 g, 55.8 mmol) and tert-butyl (2-aminoethyl)carbamate (9.84 g, 61.4 mmol) were weighed into a 100 mL round-bottom flask, heated to 90°C and stirred overnight. After adding methyl tert-butyl ether (MTBE), the mixture was stirred and filtered, and the solid was washed with MTBE for several times and dried in vacuum to obtain Intermediate 28-1 (13.6 g, yield 72%). LC-MS (ESI, m/z) calculated: 339.14, found: 340.17 (M+H).

**Step 2: Synthesis of Intermediate 28-2**

Intermediate 28-1 (10 g, 29.5 mmol) and imidazole (8.02 g, 118 mmol) were dissolved in 300 mL of dichloromethane, into which was added TBS-Cl (6.66 g, 44.2 mmol) while cooling in an ice bath, and stirred at room temperature overnight. Water was added into the reaction solution to quench the reaction. After separating the organic phase, it was washed once each with water and saturated brine, and then dried over anhydrous sodium sulfate and evaporated to dryness. The crude product was purified by column chromatography to obtain Intermediate 28-2 (12.3 g, yield 92%). LC-MS (ESI, m/z) calculated: 453.23, found: 454.33 (M+H).

**Step 3: Synthesis of Intermediate 28-3**

Intermediate 28-2 (6.0 g, 13.23 mmol) was dissolved in THF:EtOH (1:1) (500 mL), into which was added 1 g of 10% Pd-C under argon, followed by ammonium formate (8.34 g, 132 mmol). The mixture was stirred overnight at room temperature, and filtered to remove palladium carbon. The filtrate was evaporated to dryness. Dichloromethane and water were added. After separating the organic phase, it was washed once each with water and saturated brine, and then dried over anhydrous sodium sulfate and evaporated to dryness to obtain Intermediate 28-3 (5.6 g, 100%). LC-MS (ESI, m/z) calculated: 423.26, found: 424.31 (M+H).

**Step 4: Synthesis of Intermediate 28-4**

Intermediate 28-3 (5.5 g, 12.98 mmol) and Fmoc-Val-Alal-OH (5.33 g, 12.98 mmol) were dissolved in a mixed solvent of dichloromethane and methanol (v:v = 2:1, 300 mL). EEDQ (4.82 g, 19.47 mmol) was added at room temperature. The reaction solution was stirred at room temperature for 24 hours. The reaction solution was evaporated to dryness under reduced pressure, into which was added diethyl ether, and stirred until the solid was precipitated. After filtration, the solid was rinsed with diethyl ether for several times, and then dried to obtain Intermediate 28-4 (6.4 g, yield 60%). LC-MS (ESI, m/z) calculated: 815.43, found: 816.48 (M+H).

**Step 5: Synthesis of Intermediate 28-5**

Intermediate 28-4 (6.0 g, 7.35 mmol) was dissolved in 100 mL of DMF. Diethylamine (7.68 ml, 73.5 mmol) was added at room temperature. The reaction solution was stirred at room temperature for 2 hours. The reaction solution was evaporated to dryness under reduced pressure, into which were added ethyl acetate and diethyl ether, and stirred until the solid was precipitated. After filtration, the solid was rinsed with diethyl ether for several times, and then dried to obtain Intermediate 28-5 (4.3 g, yield 98%). LC-MS (ESI, m/z) calculated: 593.36, found: 594.40 (M+H).

**Step 6: Synthesis of Intermediate 28-6**

Intermediate 28-5 (4.0 g, 6.74 mmol) was dissolved in 100 ml of DMF, into which was added succinimide maleimidoacetate (2.07 g, 6.74 mmol) at room temperature. The reaction solution was stirred at room temperature overnight. The reaction solution was evaporated to dryness under reduced pressure, into which was added methyl tert-butyl ether, and stirred until the solid was precipitated. After filtration, the solid was rinsed with diethyl ether for several times, and then dried to obtain Intermediate 28-6, which was directly used in the next reaction. LC-MS (ESI, m/z) calculated: 786.43, found: 787.45 (M+H).

**Step 7: Synthesis of Intermediate 28-7**

Intermediate 28-6 (3.5 g, 4.45 mmol) was dissolved in 100 mL of anhydrous tetrahydrofuran, and cooled in an ice bath under argon atmosphere, into which was added hydrofluoric acid-pyridine complex (8.8 g, 89 mmol). The reaction solution was stirred at 0°C for 2 hours, into which was added water to quench the reaction, and extracted with dichloromethane. The organic layer was separated, and dried over anhydrous sodium sulfate, and Intermediate 28-7 (1.6 g, yield 53%) was obtained by column chromatography. LC-MS (ESI, m/z) calculated: 672.35, found: 673.37 (M+H).

**Step 8: Synthesis of Intermediate 28-8**

Intermediate 28-7 (1.0 g, 1.49 mmol) was dissolved in 20 mL of anhydrous DMF, into which were added DIPEA (0.519 ml, 2.97 mmol) and bis(4-nitrophenyl)carbonate (678 mg, 2.23 mmol) at room temperature respectively. The mixture was stirred under argon at room temperature overnight, evaporated to remove the solvent under reduced pressure, into which was then added methyl tert-butyl ether, and stirred until the solid was precipitated. After filtration, the

solid was rinsed with diethyl ether for several times, and then dried to obtain Intermediate 28-8, which was directly used in the next reaction. LC-MS (ESI, m/z) calculated: 837.35, found: 838.36 (M+H).

**Step 9: Synthesis of Intermediate 28-9**

Intermediate 28-8 (300 mg, 0.358 mmol) was dissolved in 4 mL of anhydrous DMF, into which was added 1 mL of anhydrous pyridine, and then were added Exatecan mesylate (available from Shanghai Haoyuan, 190 mg, 0.358 mmol) and HOBt (55 mg, 0.358 mmol). The reaction solution was stirred under argon at room temperature overnight, and prepared and purified by reverse-phase HPLC to obtain Intermediate 28-9 (234 mg, yield 58%). LC-MS (ESI, m/z) calculated: 1133.49, found: 1134.52 (M+H).

**Step 10: Synthesis of Intermediate 28-10**

Intermediate 28-10 (200 mg, 0.176 mmol) was dissolved in 1 mL of anhydrous dichloromethane, into which was added 300 μL of trifluoroacetic acid in an ice bath, and then returned to room temperature and stirred for 30 minutes, and the solvent was removed under reduced pressure to obtain the final product trifluoroacetate salt of Intermediate 28-10, which was directly used in the next reaction without further purification. LC-MS (ESI, m/z) calculated: 1033.43, found: 1034.45 (M+H).

**Step 11: Synthesis of Compound A28**

Compound 28-10 obtained in step 10 was dissolved in 1 mL of anhydrous DMF, into which were added acetylated-10 polysarcosine (Ac-Sar10-COOH, 136 mg, 0.176 mmol), HATU (87 mg, 0.229 mmol) and DIPEA (154 μL, 0.88 mmol) respectively, and then stirred at room temperature overnight, and the solvent was removed under reduced pressure to obtain the final product, which was then prepared and purified by reverse-phase HPLC to obtain Compound (A-11) (135 mg, yield 43%). LC-MS (ESI, m/z) calculated: 1785.82, found: 1786.85 (M+H).

**Synthesis of Compound (A-29)**

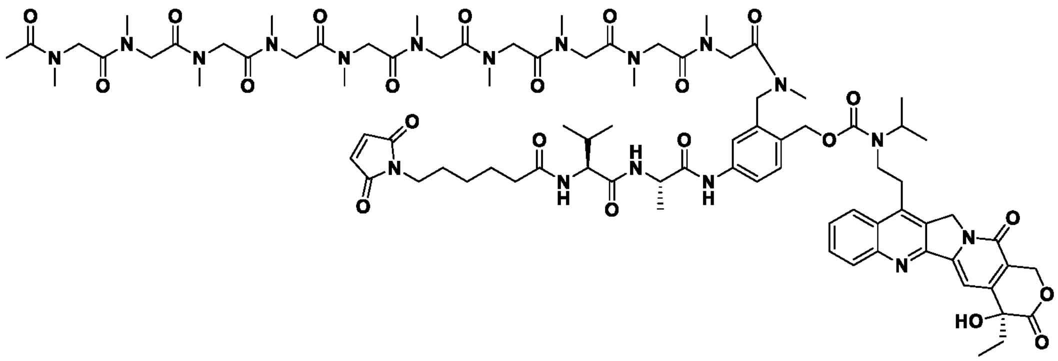

(A-29)

The synthesis of Compound (A-29) was the same as the synthesis steps of Compound (A-11), except that Exatecan mesylate in step 4 was replaced with Belotecan hydrochloride (available from Shanghai Haoyuan Chemicals), and the product (A-29) was obtained after several steps of reaction as a white amorphous powder. LC-MS (ESI, m/z) calculated: 1740.85, found: 1741.82 (M+H).

**Synthesis of PEG derivative AP-1 corresponding to A-11 molecule**

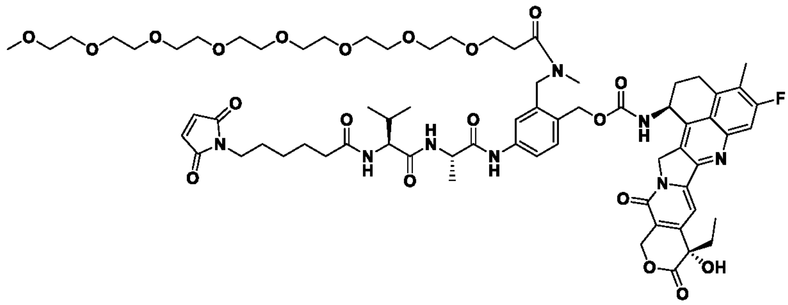

(AP-1)

The synthesis of Compound (AP-1) was the same as the synthesis steps of Compound (A-11), except that the raw compound Ac-Sar10-COOH in step 1 was replaced with m-PEG8-acid (CAS 1093647-41-6), and the product (AP-1) was obtained after several steps of reaction as a beige amorphous powder. LC-MS (ESI, m/z) calculated: 1384.64, found: 1385.66 (M+H).

**Synthesis of control MC-Val-Cit-PABC-DX8951**

Referring to the method of WO2020233174A1, Mc-Val-Cit-OH (available from Shanghai Haoyuan Chemicals) was reacted with bis(4-nitrophenyl)carbonate to obtain activated nitroester, which was then further reacted with Exatecan mesylate (available from Shanghai Haoyuan) to obtain the control compound MC-Val-Cit-PAB-DX8951.

**Example 2 General method for preparing ADC**

The antibody or antigen-binding fragment, e.g., a stock solution of Trastuzumab targeting HER2 (the heavy chain sequence is as shown in SEQ ID NO: 3, and the light chain sequence is as shown in SEQ ID NO: 4) and/or e.g., hRS7 monoclonal antibody targeting Trop-2 (Sacituzumab), was diluted to 2 mg/mL with a reaction buffer (pH 7) of 50 mM potassium dihydrogen phosphate-sodium hydroxide ($KH_2PO4$-NaOH)/150 mM sodium chloride (NaCl)/1 mM diethyltriaminepentaacetic acid (DTPA), into which was added tris(2-carboxyethyl) phosphine hydrochloride (TCEP) in a 6.0-fold excess molar ratio, and the reaction solution was stirred at 35°C for 2.5 hours.

The above reaction solution was cooled to 8°C, into which was added an appropriate amount of dimethylacetamide (DMA) without purification, and then were added the control drug molecules or drug linker conjugates A1 to A29 (10 mg/ml, pre-dissolved in DMA) in a 6-15-fold excess molar ratio respectively, ensuring that the volume ratio of DMA in the reaction system did not exceed 20%, and was stirred at 37°C for 3 hours for conjugation.

The conjugating reaction mixture was purified by filtration with a histidine-acetic acid/sucrose gel pH 6.0 using a desalting column, and the peak samples were collected according to the UV280 UV absorption values. The samples were then sterilized through a 0.15-micron pore size filter and stored at -60°C.

Preparation of Antibody Conjugate 1 (Control ADC1)

Referring to the above general method, a 10-fold excess of the control MC-Val-Cit-PAB-DX8951 was conjugated with the reduced Trastuzumab monoclonal antibody to obtain the corresponding Antibody Conjugate 1, of which the aggregate content was analyzed by size exclusion chromatography (SEC-HPLC), and the analytical spectrum was shown in FIG. 1. The proportion of Aggregate 1 was about 26%, and the proportion of Aggregate 2 was about 20%. The drug load (DAR) was about 4 to 5 as obtained by UV analysis.

Preparation of Antibody Conjugate 2 (Control ADC2)

Referring to the above general method, a 12-fold excess of the control Deruxtecan (available from Shanghai Haoyuan Chemicals) was conjugated with the reduced Trastuzumab monoclonal antibody to obtain the corresponding Antibody Conjugate 2, of which the aggregate content was analyzed by size exclusion chromatography (SEC-HPLC), and the analytical spectrum was shown in FIG. 2. The proportion of the aggregate was about 2%. The drug load (DAR) was about 7-8 as obtained by hydrophobic interaction high performance liquid chromatography (HIC-HPLC), and the analytical spectrum was shown in FIG. 3.

Preparation of Antibody Conjugate 3

Referring to the above general method, an 8-fold excess of Compound (A-2) was conjugated with the reduced Trastuzumab monoclonal antibody to obtain the corresponding Antibody Conjugate 3, of which the aggregate content was analyzed by size exclusion chromatography (SEC-HPLC), and the analytical spectrum was shown in FIG. 4. The proportion of Aggregate 1 was about 6.8%, and the proportion of Aggregate 2 was about 7.7%. The drug load (DAR) was about 6 to 7 as obtained by UV analysis.

Preparation of Antibody Conjugate 4

Referring to the above general method, a 12-fold excess of Compound (A-4) was conjugated with the reduced Trastuzumab monoclonal antibody to obtain Antibody Conjugate 4 with a drug load (DAR) of about 7 to 8, of which the aggregate content was analyzed by size exclusion chromatography (SEC-HPLC), and the analytical spectrum was shown in FIG. 5. The proportion of Aggregate 1 was about 0.3%. The drug load (DAR) was about 7 to 8 as obtained by hydrophobic interaction high performance liquid chromatography (HIC-HPLC), and the analytical spectrum was shown in FIG. 6.

Preparation of Antibody Conjugate 5

Referring to the above general method, a 12-fold excess of Compound (A-11) was conjugated with the reduced Trastuzumab monoclonal antibody to obtain the corresponding Antibody Conjugate 5, of which the aggregate content was analyzed by size exclusion chromatography (SEC-HPLC), and the analytical spectrum was shown in FIG. 7. The purity of the monomer of Antibody Conjugate 5 was 99.41%. The drug load (DAR) was about 7 to 8 as obtained by hydrophobic interaction high performance liquid chromatography (HIC-HPLC), and the analytical spectrum was shown in FIG. 8. The mass number difference between the light and heavy chains of Trastuzumab (Fig. 34) and the light and heavy chains of Antibody Conjugate 1 (Fig. 35) after reduction was compared by mass spectrometry, and the exact drug load was calculated to be 7.8.

Preparation of Antibody Conjugate 6

Referring to the above general method, a 12-fold excess of Compound (A-14) was conjugated with the reduced Trastuzumab monoclonal antibody to obtain the corresponding Antibody Conjugate 6, of which the aggregate content was analyzed by size exclusion chromatography (SEC-HPLC), and the analytical spectrum was shown in FIG. 9. The purity of the monomer of Antibody Conjugate 6 was 99.60%. The drug load (DAR) was about 7 to 8 as obtained by hydrophobic interaction high performance liquid chromatography (HIC-HPLC), and the analytical spectrum was shown in FIG. 10. The mass number difference between the light and heavy chains of Trastuzumab (Fig. 34) and the light and heavy chains of Antibody Conjugate 2 (Fig. 36) after reduction was compared by mass spectrometry, and the exact drug load was calculated to be 8.0.

Preparation of Antibody Conjugate 7

Referring to the above general method, a 15-fold excess of Compound (A-24) was conjugated with the reduced Trastuzumab monoclonal antibody to obtain the corresponding Antibody Conjugate 7, of which the aggregate content was analyzed by size exclusion chromatography (SEC-HPLC), and the analytical spectrum was shown in FIG. 11. The purity of the monomer of Antibody Conjugate 7 was 96.36%. The drug load (DAR) was about 7 to 8 as obtained by hydrophobic interaction high performance liquid chromatography (HIC-HPLC), and the analytical spectrum was shown in FIG. 12.

Preparation of Antibody Conjugate 8 (Control ADC3)

Referring to the above general method, a 12-fold excess of Compound AP-1 was conjugated with the reduced Trastuzumab monoclonal antibody to obtain the corresponding Antibody Conjugate 8, of which the aggregate content was analyzed by size exclusion chromatography (SEC-HPLC), and the analytical spectrum was shown in FIG. 13. The purity of the monomer of Antibody Conjugate 7 was 97.92%. The drug load (DAR) was about 7 to 8 as obtained by hydrophobic interaction high performance liquid chromatography (HIC-HPLC), and the analytical spectrum was shown in FIG. 14.

Preparation of Antibody Conjugate 9

Referring to the above general method, a 12-fold excess of Compound (A-11) was conjugated with the reduced Patritumab monoclonal antibody to obtain the corresponding Antibody Conjugate 9, of which the aggregate content was analyzed by size exclusion chromatography (SEC-HPLC), and the analytical spectrum was shown in FIG. 15. The purity of the monomer of Antibody Conjugate 9 was 97.92%. The drug load (DAR) was about 7 to 8 as obtained by hydrophobic interaction high performance liquid chromatography (HIC-HPLC), and the analytical spectrum was shown in FIG. 16.

Preparation of Antibody Conjugate 10

Referring to the above general method, a 12-fold excess of Compound (A-11) was conjugated with the reduced H01L02 monoclonal antibody to obtain the corresponding Antibody Conjugate 10, of which the aggregate content was analyzed by size exclusion chromatography (SEC-HPLC), and the analytical spectrum was shown in FIG. 17. The purity of the monomer of Antibody Conjugate 10 was 99.10%. The drug load (DAR) was about 7 to 8 as obtained by hydrophobic interaction high performance liquid chromatography (HIC-HPLC), and the analytical spectrum was shown in FIG. 18.

Preparation of Antibody Conjugate 11

Referring to the above general method, a 12-fold excess of Compound (A-28) was conjugated with the reduced Trastuzumab monoclonal antibody to obtain the corresponding Antibody Conjugate 11, of which the aggregate content was analyzed by size exclusion chromatography (SEC-HPLC), and the analytical spectrum was shown in FIG. 19. The purity of the monomer of Antibody Conjugate 11 was 99.59%. The drug load (DAR) was about 7 to 8 as obtained by hydrophobic interaction high performance liquid chromatography (HIC-HPLC), and the analytical spectrum was shown in FIG. 20.

Preparation of Antibody Conjugate 12

Referring to the above general method, a 12-fold excess of Compound (A-29) was conjugated with the reduced Trastuzumab monoclonal antibody to obtain the corresponding Antibody Conjugate 12, of which the aggregate content was analyzed by size exclusion chromatography (SEC-HPLC), and the analytical spectrum was shown in FIG. 21. The purity of the monomer of Antibody Conjugate 12 was 99.67%. The drug load (DAR) was about 7 to 8 as obtained by hydrophobic interaction high performance liquid chromatography (HIC-HPLC), and the analytical spectrum was shown in FIG. 22.

Preparation of Antibody Conjugate 13

A stock solution of the hRS7 monoclonal antibody targeting Trop-2 was dialyzed into a buffer (pH7.0) of 50 mM sodium dihydrogen phosphate-disodium hydrogen phosphate ($NaH_2PO_4$-$Na_2HPO_4$)/50 mM sodium chloride (NaCl). After measuring the concentration of the monoclonal antibody in the solution, the antibody was diluted to 5 mg/mL with the buffer above. The reaction tube was placed in an ice bath to cool down for 10 minutes. Tris(2-carboxyethyl)phosphine hydrochloride (TCEP) was added in a 2.5-fold molar ratio, and the reaction solution was stirred at 4°C overnight. An appropriate amount of dimethylacetamide (DMA) was further added into the above unpurified reaction solution, and then the control drug molecules or drug linker conjugates A-11 (10 mM pre-dissolved in DMA) were added in a 6.0-fold excess molar ratio respectively, ensuring that the volume ratio of DMA in the reaction system did not exceed 10%, and the mixture was stirred at 4°C for 2 hours for conjugation. After the conjugation, the Cysteine small molecule was added to the reaction solution in a 4.0-fold molar ratio to consume the excess drug linker conjugate A-11, and the reaction was stirred at 4°C for 30 minutes for quenching. The conjugating reaction mixture was purified by filtration with a histidine-acetic acid/sodium chloride pH 5.5 using a desalting column, and the filtered sample was collected. One-tenth volume of activated carbon-histidine-acetic acid/sodium chloride suspension (300 mg/mL) was added to the sample, and the mixture was stirred at room temperature for 2 hours to fully absorb free small drug molecules. The samples were then sterilized through a 0.22-micron pore size filter and stored at -80°C. The aggregate content was analyzed by size exclusion chromatography (SEC-HPLC), and the analytical spectrum was shown in FIG. 31. The purity of the monomer of Antibody Conjugate 13 was 98.95%.

The concentration of the conjugated drug in the antibody-drug conjugate can be calculated by measuring the UV absorbances of the antibody-drug conjugate aqueous solution at two wavelengths of 280 nm and 370 nm, and then calculated as follows.

The total absorbance at any given wavelength is equal to the sum of the absorbances of all light-absorbing chemicals in the system (additivity of absorbances). Therefore, based on the assumption that the molar absorption

coefficients of the antibody and the drug do not change before and after conjugation of the antibody and the drug, the antibody concentration and the drug concentration in the antibody-drug conjugate are represented by the following formula.

$$A_{280} = A_{D,280} + A_{A,280} = \varepsilon_{D,280}C_D + \varepsilon_{A,280}C_A \quad (1)$$

$$A_{370} = A_{D,370} + A_{A,370} = \varepsilon_{D,370}C_D + \varepsilon_{A,370}C_A \quad (2)$$

Wherein, $A_{280}$ represents the absorbance of the antibody-drug conjugate solution at 280 nm, and $A_{370}$ represents the absorbance of the antibody-drug conjugate solution at 370 nm. $A_{D,280}$ represents the absorbance of the drug linker conjugate at 280 nm, and $A_{D,370}$ represents the absorbance of the drug linker conjugate at 370 nm. $A_{A,280}$ represents the absorbance of the antibody at 280 nm, and $A_{A,370}$ represents the absorbance of the antibody at 370 nm. $\varepsilon_{D,280}$ represents the molar absorption coefficient of the drug linker conjugate at 280 nm, $\varepsilon_{D,370}$ represents the molar absorption coefficient of the drug linker conjugate at 370 nm, $\varepsilon_{A,280}$ represents the molar absorption coefficient of the antibody at 280 nm, and $\varepsilon_{A,370}$ represents the molar absorption coefficient of the antibody at 370 nm. $C_D$ represents the concentration of the drug linker conjugate in the antibody-drug conjugate solution, and $C_A$ represents the concentration of the antibody in the antibody-drug conjugate solution.

$\varepsilon_{A,280}$ can be estimated from the amino acid sequence of an antibody by a known computational method (Protein Science, 1995, vol. 4, 2411-2423), and $\varepsilon_{A,370}$ is generally 0. By the absorbances of the drug linker conjugate at a certain molar concentration in solution, $\varepsilon_{D,280}$ and $\varepsilon_{D,370}$ were obtained according to the Lambert-Beer law. The values of $A_{280}$ and $A_{370}$ are determined by a microplate reader or UV spectrophotometer, and the values of the above four molar absorption coefficients are brought into the simultaneous equations (1) and (2) to calculate the values of $C_A$ and $C_D$. Then the average number of conjugated drug molecules per antibody molecule is DAR = $C_D$ / $C_A$. The UV absorption coefficients of Compound A-11 are $\varepsilon_{D,280}$=6480, $\varepsilon_{D,370}$=16483, and are used for the calculation of the drug-to-antibody ratio in this example. The DAR value of Antibody Conjugate 13 is about 4.0.

Preparation of Antibody Conjugate 14

A stock solution of the hRS7 monoclonal antibody targeting Trop-2 was dialyzed into a buffer (pH7.0) of 50 mM sodium dihydrogen phosphate-disodium hydrogen phosphate ($NaH_2PO_4$-$Na_2HPO_4$)/50 mM sodium chloride (NaCl). After measuring the concentration of the monoclonal antibody in the solution, the antibody was diluted to 5 mg/mL with the buffer above. The reaction tube was placed in an ice bath to cool down for 10 minutes. Tris(2-carboxyethyl)phosphine hydrochloride (TCEP) was added in a 2.5-fold molar ratio, and the reaction solution was stirred at 4°C overnight. An appropriate amount of dimethylacetamide (DMA) was further added into the above unpurified reaction solution, and then the control drug molecules or drug linker conjugates A-14 (10 mM pre-dissolved in DMA) were added in a 6.0-fold excess molar ratio respectively, ensuring that the volume ratio of DMA in the reaction system did not exceed 10%, and the mixture was stirred at 4°C for 2 hours for conjugation. After the conjugation, the Cysteine small molecule was added to the reaction solution in a 4.0-fold molar ratio to consume the excess drug linker conjugate A-14, and the reaction was stirred at 4°C for 30 minutes for quenching. The conjugating reaction mixture was purified by filtration with a histidine-acetic acid/sodium chloride pH 5.5 using a desalting column, and the filtered sample was collected. One-tenth volume of activated carbon-histidine-acetic acid/sodium chloride suspension (300 mg/mL) was added to the sample, and the mixture was stirred at room temperature for 2 hours to fully absorb free small drug molecules. The samples were then sterilized through a 0.22-micron pore size filter and stored at -80°C. The aggregate content was analyzed by size exclusion chromatography (SEC-HPLC), and the analytical spectrum was shown in FIG. 32. The purity of the monomer of Antibody Conjugate 14 was 98.20%. The UV absorption coefficients of Compound A-14 are $\varepsilon_{D,280}$=5932, $\varepsilon_{D,370}$=14997, and are used for calculating the drug-to-antibody ratio in this example. The DAR value of Antibody Conjugate 14 is about 4.2.

Preparation of Antibody Conjugate 15

A stock solution of the hRS7 monoclonal antibody targeting Trop-2 was dialyzed into a buffer (pH7.0) of 50 mM sodium dihydrogen phosphate-disodium hydrogen phosphate ($NaH_2PO_4$-$Na_2HPO_4$)/50 mM sodium chloride (NaCl). After measuring the concentration of the monoclonal antibody in the solution, the antibody was diluted to 5 mg/mL with the buffer above. The reaction tube was placed in an ice bath to cool down for 10 minutes. Tris(2-carboxyethyl)phosphine hydrochloride (TCEP) was added in a 2.5-fold molar ratio, and the reaction solution was stirred at 4°C overnight. An appropriate amount of dimethylacetamide (DMA) was further added into the above unpurified reaction solution, and then the control drug molecules or drug linker conjugates A-24 (10 mM pre-dissolved in DMA) were added in a 6.0-fold excess

molar ratio respectively, ensuring that the volume ratio of DMA in the reaction system did not exceed 10%, and the mixture was stirred at 4°C for 2 hours for conjugation. After the conjugation, the Cysteine small molecule was added to the reaction solution in a 4.0-fold molar ratio to consume the excess drug linker conjugate A-24, and the reaction was stirred at 4°C for 30 minutes for quenching. The conjugating reaction mixture was purified by filtration with a histidine-acetic acid/sodium chloride pH 5.5 using a desalting column, and the filtered sample was collected. One-tenth volume of activated carbon-histidine-acetic acid/sodium chloride suspension (300 mg/mL) was added to the sample, and the mixture was stirred at room temperature for 2 hours to fully absorb free small drug molecules. The samples were then sterilized through a 0.22-micron pore size filter and stored at -80°C. The aggregate content was analyzed by size exclusion chromatography (SEC-HPLC), and the analytical spectrum was shown in FIG. 33. The purity of the monomer of Antibody Conjugate 15 was 96.36%. The UV absorption coefficients of Compound A-24 are $\varepsilon_{D,280}$=4723, $\varepsilon_{D,370}$=12467, and are used for calculating the drug-to-antibody ratio in this example. The DAR value of Antibody Conjugate 15 is about 4.0.

Compared with the ADC molecule prepared from the control molecule, the compound of this application and the ADC prepared therefrom have higher monomer purity under the condition of a higher drug load, suggesting that such ADC molecules have better stability. In addition, the results of hydrophobic interaction high performance liquid chromatography (HIC-HPLC) analysis showed that the ADC molecule of this application had a lower retention time than the control molecule, and was closer to the naked antibody, suggesting that such ADC molecules had better hydrophilicity, and may have longer in vivo half-life and stronger in vivo efficacy.

**Example 3 Antibody conjugate stability test**

ADC drugs based on camptothecin topoisomerase inhibitors, such as Sacituzumab govitecan (Trodelvy) and Trastuzumab Deruxtecan (Enhertu), usually have higher drug-to-antibody loading ratios (DARs), and since camptothecin molecules have aromatic fused ring structures which have strong hydrophobicity, the stability of such ADC molecules must be affected to a certain extent. The ADC prepared in the present invention has a polysarcosine peptide chain introduced at a specific position, and thus has better "barrier effect" and can have better stability. We have designed an accelerated antibody stability experiment to verify this hypothesis.

Molecules for comparison include:

1. Trastuzumab

2. Antibody Conjugate 2

3. Antibody Conjugate 5

4. Antibody Conjugate 6

5. Antibody Conjugate 7

5. Antibody Conjugate 8

We diluted the molecular samples to be tested with a preparation buffer (20 mM Histidine-acetic acid, 150 mM NaCl, pH 5.5) to a concentration of 5 mg/ml, from which 50 μL was taken for testing directly or being incubated in a 55°C water bath for 1 hour, 2 hours, 5 hours, 24 hours, 48 hours and 72 hours respectively before testing (the accelerated stability experiment). The changes in sample concentration were detected by the UV method, and the content of ADC molecules with normal structures (main peak) and polymeric molecules in the sample were quantified using a TSK-GEL SWXL3000 size exclusion column on an Agilent 1260 Infinity II Bio-inert LC system, respectively.

The mobile phase of HPLC was 200 mM phosphate buffer (pH 7.0) + 150 mM KCl + 15% IPA, the column temperature was 25°C, the injection volume was 7 μL, the flow rate was 0.75 mL/min, and the UV detection wavelengths were 280 nm and 370 nm.

The statistical results of sample concentration changes were shown in FIG. 23, from which we found that Trastuzumab and its corresponding Antibody Drug Conjugate 5 and Antibody Drug Conjugate 6 all have good thermal stability, and small differences in concentration changes over the course of 0-72 hours. However, the samples of Antibody Drug Conjugate 2, Antibody Drug Conjugate 7 and Antibody Drug Conjugate 8 showed a rapid decrease in ADC concentration and precipitation after 24 hours, resulting in a rapid decrease in the sample concentration.

The change results of the increase in the proportion of aggregate molecules in the accelerated stability experiment samples above were further analyzed by size exclusion chromatography. The statistical result was shown in FIG. 24, which is consistent with the trend of sample concentration changes. The stability of the samples is ranked from high to low as Trastuzumab ≈ Antibody Conjugate 5 > Antibody Conjugate 6 > Antibody Conjugate 7 > Antibody Conjugate

8 ≈ Antibody Conjugate 2.

The results above verified that the hydrophilic polysarcosine peptide chain at a specific position of the present invention has a "barrier effect", which brings an "unexpected" effect of improving the stability to the antibody drug conjugate molecules.

**Example 4 In vitro cytotoxic activity test**

NCI-N87 human gastric cancer cells were selected as the cell lines for in vitro activity detection in this experiment to observe the dose-effect profiles of Antibody Conjugate 2, Antibody Conjugate 5, Antibody Conjugate 6, Trastuzumab monoclonal antibody, and IgG-deruxtecan on cell killing. The final concentrations of the antibody drug conjugates prepared in Example 2 after addition were designed as a series of 9 concentrations from 500 to 0.1 nM (5-fold dilution) with 500 nM as the initial concentration. The changes in killing (or inhibition) over 120 hours were observed. Chemiluminescence staining (Luminescent Cell Viability Assay) was carried out, and $IC_{50}$ was calculated after reading the fluorescence data.

As shown in FIG. 25, when NCI-N87 cells with high expression of Her2 were treated with Antibody Conjugate 2, Antibody Conjugate 4, Antibody Conjugate 5, Antibody Conjugate 6, as well as Trastuzumab mAb and IgG-deruxtecan, the Antibody Conjugate 2, Antibody Conjugate 4, Antibody Conjugate 5, and Antibody Conjugate 6 all could inhibit tumor cell proliferation significantly, and were significantly stronger than Trastuzumab monoclonal antibody and IgG-deruxtecan.

As shown in FIG. 26, when OV-CAR3 human ovarian adenocarcinoma cells with low expression of Her2 were treated with Antibody Conjugate 5, Antibody Conjugate 11 and DS8201a (Antibody Conjugate 2), all the three antibody conjugates could inhibit tumor cell proliferation significantly, and Antibody Conjugate 5 and Antibody Conjugate 11 were better than DS8201a (Antibody Conjugate 2).

As shown in FIG. 27 and FIG. 28, when NCI-N87 cells and SK-BR-3 cells with high expression of Her2 were treated with Antibody Conjugate 12 and Belotecan small molecules, the Antibody Conjugate 12 could inhibit tumor cell proliferation significantly, and was significantly stronger than the loaded small molecular drug Belotecan.

Based on the results of the above activity test, the ADCs prepared from the compounds of this application all showed good in vitro anti-tumor activity. Especially for tumor cells with low expression of related antigens, the antibody conjugates of this application have more obvious advantages.

**Example 5 Determination of in vivo anti-tumor efficacy**

The efficacy of the combination of the present invention can be measured in vivo, i.e., an allograft or xenograft of cancer cells was implanted in rodents, and the tumors were treated with the combination. Test mice were treated with drugs or controls and monitored for several weeks or longer to measure the time to tumor doubling, log cell killing, and tumor inhibition.

In vivo anti-tumor experiment (1)

COLO205 human colon cancer cells (ATCC) with low expression of HER2 were suspended in physiological saline. $4\times10^7$ cells were subcutaneously transplanted into the right flank of female nude mice, which were randomized into groups on day 6. Taking the grouping day as day 0, Antibody Conjugate 2 (DS8201) and Antibody Conjugate 5 were administered intravenously to the tail at doses of 3 mg/kg and 10 mg/kg respectively on day 0, as well as 10 mg/kg of Kadcyla (T-DM1). As a control group, a PBS buffer-administered group was set.

The results were shown in FIG. 29. For COLO205 tumors with low expression of HER2, administration of 10 mg/kg of Kadcyla (T-DM1) did not show tumor growth inhibitory activity, while 3 mg/kg and 10 mg/kg of DS8201 and Antibody Conjugate 5 both showed dose-dependent anti-tumor activity, wherein the efficacy of Antibody Conjugate 5 was significantly stronger than that of the control Antibody Conjugate 2.

It should be noted that, regarding the expression of HER2 in tumors, the results obtained by the determination based on immunohistochemical staining classified those with a score of 3+ as high expression, those with a score of 2+ as medium expression, and those with a score of 1+ as low expression. In addition, the case which had a score of 0 in the determining method, but for example had a positive result by other determining methods such as a flow cytometer-based determining method was classified as low expression.

In vivo anti-tumor experiment (2)

HCC1954 human breast cancer cells (ATCC) with medium expression of HER2 were suspended in physiological saline. $4\times10^7$ cells were subcutaneously transplanted into the right flank of female nude mice, which were randomized into groups on day 6. Taking the grouping day as day 0, Antibody Conjugate 2 (DS8201) and Antibody Conjugate 5

were administered intravenously to the tail at doses of 1 mg/kg, 3 mg/kg and 10 mg/kg respectively on day 0. As control groups, an IgG-deruxtecan conjugate-administered group and an IgG-A11 conjugate-administered group were set by conjugating IgG to the corresponding toxin linker, respectively.

The results were shown in FIG. 30. For HCC1954 tumors with medium expression of HER2, administration of 1 mg/kg, 3 mg/kg, 10 mg/kg of Antibody Conjugate 2 (DS8201) and Antibody Conjugate 5 all showed dose-dependent anti-tumor activity, wherein 10 mg/kg of DS8201, 3 mg/kg and 10 mg/kg of Antibody Conjugate 5 all showed dose-dependent anti-tumor activity, wherein the efficacy of Antibody Conjugate 5 was significantly stronger than that of the control Antibody Conjugate 2.

It can be seen from the activity test results that, the ADCs prepared from the compounds of this application all showed certain in vivo anti-tumor activity, and could show significantly stronger anti-tumor activity compared with the control sample. All the tumor-bearing mice tolerated the above drugs well, and no symptoms such as weight loss occurred.

**Example 6 Detection of in vivo pharmacokinetics**

In this example, the pharmacokinetic properties of Antibody Conjugate 5, Antibody Conjugate 6 and the control drug DS8201a in rats were evaluated. Specifically, in this example, 3 mg/kg of Antibody Conjugate 5, Antibody Conjugate 6 and DS8201 were administered to rats by tail injection. The average toxicokinetic parameters of the bound antibodies in each dose group were shown in Table below. The order of the half-life of the bound antibodies was Antibody Conjugate 5 ≈ Antibody Conjugate 6 > DS8201a.

| | | | **101** | **102** | **103** | **Average value** | **SD** |
|---|---|---|---|---|---|---|---|
| | **Parameter** | **Unit** | **Value** | **Value** | **Value** | **Value** | |
| DS8201 (3mg/kg) | t1/2 | d | 5.37 | 3.81 | 6.049 | 5.07 | 1.15 |
| | Tmax | d | 0.167 | 0.167 | 0.167 | 0.17 | 0.00 |
| | Cmax | ng/ml | 230425 | 98450 | 183975 | 170950 | 66945 |
| | AUC 0-t | ng/ml*d | 905104 | 609295 | 1164404 | 892934 | 277754 |
| Antibody Conjugate 5 (3 mg/kg) | t1/2 | d | 8.78 | 8.35 | 8.32 | 8.48 | 0.26 |
| | Tmax | d | 1 | 0.167 | 1.00 | 0.72 | 0.48 |
| | Cmax | ng/ml | 85138 | 128325 | 81425 | 98296 | 26072 |
| | AUC 0-t | ng/ml*d | 740447 | 918463 | 803401 | 820770 | 90270 |
| Antibody Conjugate 6 (3 mg/kg) | t1/2 | d | 9.89 | 9.91 | 8.990 | 9.60 | 0.53 |
| | Tmax | d | 0.17 | 0.167 | 0.167 | 0.17 | 0.00 |
| | Cmax | ng/ml | 99075 | 165600 | 116925 | 127200 | 34432 |
| | AUC 0-t | ng/ml*d | 618863 | 913240 | 860973 | 797692 | 157060 |

The PK results of the above antibody conjugates in rats showed that the ADCs obtained by the new technical solution had better stability and longer half-life in rats. Therefore, the ADCs of this application could show better safety and efficacy than DS-8201a, thus bringing benefits to cancer patients with medium and low HER2 expression.

The foregoing detailed description is provided in an illustrative and exemplary manner, and is not intended to limit the scope of the appended claims. Various modifications of the embodiments currently enumerated in the present application will be apparent to those of ordinary skill in the art, and are encompassed within the scope of the appended claims and their equivalents.

**Claims**

1. A compound, or a tautomer, mesomer, racemate, enantiomer, and diastereomer thereof, or a mixture thereof, or a pharmaceutically acceptable salt, prodrug or solvate thereof, wherein the compound comprises a structure represented by formula (C-HER2):

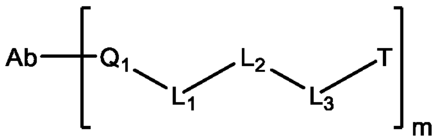

(C-HER2),

wherein, $Q_1$ comprises a linker,
$L_1$ comprises $-L_{1a}-C(=O)-$,
wherein, $L_{1a}$ is selected from the group consisting of optionally substituted alkylene groups, optionally substituted polyethylene glycol groups, optionally substituted alkenylene groups, optionally substituted alkynylene groups, optionally substituted aliphatic cyclylene groups, optionally substituted aliphatic heterocyclylene groups, optionally substituted arylene groups, and optionally substituted heteroarylene groups;
$L_2$ comprises an optionally substituted polypeptide residue,
$L_3$ comprises an optionally substituted spacer group;
wherein, $L_2$ and/or $L_3$ comprise optionally substituted polysarcosine residues,
T comprises a drug unit,
Ab is a ligand capable of binding to HER2, and m is a number from 1 to 8.

2. The compound according to claim 1, or a tautomer, mesomer, racemate, enantiomer, and diastereomer thereof, or a mixture thereof, or a pharmaceutically acceptable salt, prodrug or solvate thereof, wherein, $Q_1$ comprises a linker coupled with a mercapto group.

3. The compound according to any one of claims 1-2, or a tautomer, mesomer, racemate, enantiomer, and diastereomer thereof, or a mixture thereof, or a pharmaceutically acceptable salt, prodrug or solvate thereof, wherein, $Q_1$ is selected from the group consisting of optionally substituted

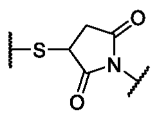,

optionally substituted

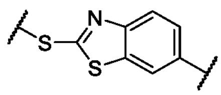,

optionally substituted

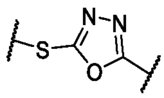,

and optionally substituted

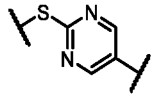.

4. The compound according to any one of claims 1-3, or a tautomer, mesomer, racemate, enantiomer, and diastereomer thereof, or a mixture thereof, or a pharmaceutically acceptable salt, prodrug or solvate thereof, wherein, $L_{1a}$ is selected from the group consisting of optionally substituted $C_1$-$C_7$ alkylene groups, optionally substituted diethylene glycol to octaethylene glycol groups, optionally substituted $C_3$-$C_6$ aliphatic cyclylene groups, optionally substituted arylene groups, and optionally substituted heteroarylene groups.

**5.** The compound according to any one of claims 1-4, or a tautomer, mesomer, racemate, enantiomer, and diastereomer thereof, or a mixture thereof, or a pharmaceutically acceptable salt, prodrug or solvate thereof,
wherein, $L_{1a}$ is selected from the group consisting of optionally substituted methylene groups, optionally substituted ethylene groups, optionally substituted propylene groups, optionally substituted butylene groups, optionally substituted pentylene groups, optionally substituted diethylene glycol groups, optionally substituted tetraethylene glycol groups, optionally substituted hexaethylene glycol groups, optionally substituted octaethylene glycol groups, and optionally substituted cyclohexylene groups.

**6.** The compound according to any one of claims 1-5, or a tautomer, mesomer, racemate, enantiomer, and diastereomer thereof, or a mixture thereof, or a pharmaceutically acceptable salt, prodrug or solvate thereof,
wherein, $L_2$ comprises optionally substituted polypeptide residues composed of amino acids selected from the group consisting of phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

**7.** The compound according to any one of claims 1-6, or a tautomer, mesomer, racemate, enantiomer, and diastereomer thereof, or a mixture thereof, or a pharmaceutically acceptable salt, prodrug or solvate thereof,
wherein, $L_2$ comprises optionally substituted polypeptide residues composed of amino acids selected from the group consisting of glycine, phenylalanine, valine, alanine, arginine, citrulline, aspartic acid, asparagine, and lysine.

**8.** The compound according to any one of claims 1-7, or a tautomer, mesomer, racemate, enantiomer, and diastereomer thereof, or a mixture thereof, or a pharmaceutically acceptable salt, prodrug or solvate thereof,
wherein, $L_2$ comprises optionally substituted polypeptide residues selected from the group consisting of phenylalanine-lysine (Phe-Lys), valine-alanine (Val-Ala), valine-citrulline (Val-Cit), glutamic acid-valine-alanine (Glu-Val-Ala), glutamic acid-valine-citrulline (Glu-Val-Cit), valine-lysine (Val-Lys), alanine-alanine-alanine (Ala-Ala-Ala), alanine-alanine-asparagine (Ala-Ala-Asn), and glycine-glycine-phenylalanine-glycine (Gly-Gly-Phe-Gly).

**9.** The compound according to any one of claims 1-8, or a tautomer, mesomer, racemate, enantiomer, and diastereomer thereof, or a mixture thereof, or a pharmaceutically acceptable salt, prodrug or solvate thereof,
wherein, when $L_2$ comprises a lysine residue, the lysine residue is substituted with a structure $R_1$ comprising a polysarcosine residue.

**10.** The compound according to claim 9, or a tautomer, mesomer, racemate, enantiomer, and diastereomer thereof, or a mixture thereof, or a pharmaceutically acceptable salt, prodrug or solvate thereof,
wherein, the $R_1$ is optionally substituted

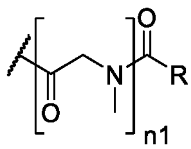

,

wherein n1 is a number from 4 to 18, and R is selected from the group consisting of $C_1$-$C_6$ alkyl, $C_1$-$C_6$ cycloalkyl, and $C_1$-$C_6$ alkoxy.

**11.** The compound according to claim 10, or a tautomer, mesomer, racemate, enantiomer, and diastereomer thereof, or a mixture thereof, or a pharmaceutically acceptable salt, prodrug or solvate thereof,
wherein, n1 is from 4 to 18.

**12.** The compound according to any one of claims 1-11, or a tautomer, mesomer, racemate, enantiomer, and diastereomer thereof, or a mixture thereof, or a pharmaceutically acceptable salt, prodrug or solvate thereof,
wherein, $L_3$ is selected from the group consisting of optionally substituted

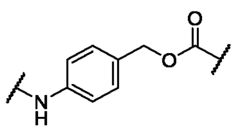

,

and optionally substituted

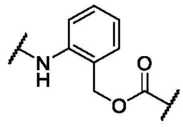

.

**13.** The compound according to any one of claims 1-12, or a tautomer, mesomer, racemate, enantiomer, and diastereomer thereof, or a mixture thereof, or a pharmaceutically acceptable salt, prodrug or solvate thereof,
wherein, the benzene ring of $L_3$ is linked to the optionally substituted polysarcosine residues through a structural unit -X-, and the structural unit -X- is selected from the group consisting of optionally substituted

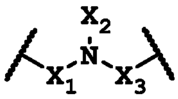

,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; and the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

**14.** The compound according to any one of claims 1-13, or a tautomer, mesomer, racemate, enantiomer, and diastereomer thereof, or a mixture thereof, or a pharmaceutically acceptable salt, prodrug or solvate thereof,
wherein, the benzene ring of $L_3$ is linked to the optionally substituted polysarcosine residues through a structural unit -X-, and the structural unit -X- is selected from the group consisting of optionally substituted

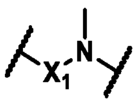

,

and optionally substituted

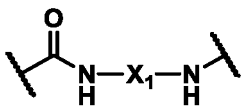

,

wherein $X_1$ is selected from the group consisting of $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S, and the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl.

**15.** The compound according to any one of claims 13-14, or a tautomer, mesomer, racemate, enantiomer, and diastereomer thereof, or a mixture thereof, or a pharmaceutically acceptable salt, prodrug or solvate thereof,
wherein, the structural unit -X- is optionally substituted

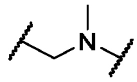,

and the optionally substituted polysarcosine residue comprises

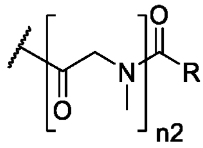,

wherein n2 is a number from 4 to 18, and R is selected from the group consisting of $C_1$-$C_6$ alkyl, $C_1$-$C_6$ cycloalkyl, and $C_1$-$C_6$ alkoxy.

**16.** The compound according to any one of claims 13-15, or a tautomer, mesomer, racemate, enantiomer, and diastereomer thereof, or a mixture thereof, or a pharmaceutically acceptable salt, prodrug or solvate thereof, wherein, the structural unit -X- is selected from the group consisting of optionally substituted

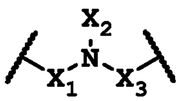,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl, and the optionally substituted polysarcosine residue comprises

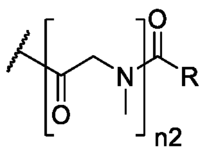,

wherein n2 is a number from 4 to 18, and R is selected from the group consisting of $C_1$-$C_6$ alkyl, $C_1$-$C_6$ cycloalkyl, and $C_1$-$C_6$ alkoxy.

**17.** The compound according to any one of claims 13-14, or a tautomer, mesomer, racemate, enantiomer, and diastereomer thereof, or a mixture thereof, or a pharmaceutically acceptable salt, prodrug or solvate thereof, wherein, the structural unit -X- is optionally substituted

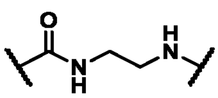,

and the optionally substituted polysarcosine residue comprises

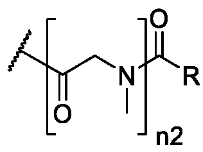

,

wherein n2 is a number from 4 to 18, and R is selected from the group consisting of $C_1$-$C_6$ alkyl, $C_1$-$C_6$ cycloalkyl, and $C_1$-$C_6$ alkoxy.

**18.** The compound according to any one of claims 13-14 and 17, or a tautomer, mesomer, racemate, enantiomer, and diastereomer thereof, or a mixture thereof, or a pharmaceutically acceptable salt, prodrug or solvate thereof, wherein, the structural unit -X- is selected from the group consisting of optionally substituted

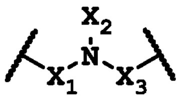

,

wherein $X_1$ is selected from the group consisting of carbonyl, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_2$ is selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; wherein $X_3$ is a covalent bond or selected from the group consisting of hydrogen, $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms, where the heteroalkyl comprises 1-3 atoms selected from N, O or S; the $C_1$-$C_8$ alkyl, $C_1$-$C_8$ alkoxy, $C_1$-$C_6$ cycloalkyl, linear heteroalkyl comprising 1-8 atoms, and linear-cyclic heteroalkyl comprising 1-8 atoms are each independently optionally substituted with one or more substituents selected from deuterium, halogen, cyano, nitro, amino, alkyl, carboxy, alkoxy, or cycloalkyl, and the optionally substituted polysarcosine residue comprises

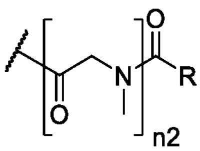

,

wherein n2 is a number from 4 to 18, and R is selected from the group consisting of $C_1$-$C_6$ alkyl, $C_1$-$C_6$ cycloalkyl, and $C_1$-$C_6$ alkoxy.

**19.** The compound according to any one of claims 15-18, or a tautomer, mesomer, racemate, enantiomer, and diastereomer thereof, or a mixture thereof, or a pharmaceutically acceptable salt, prodrug or solvate thereof, wherein, n2 is from 4 to 18.

**20.** The compound according to any one of claims 1-19, or a tautomer, mesomer, racemate, enantiomer, and diastereomer thereof, or a mixture thereof, or a pharmaceutically acceptable salt, prodrug or solvate thereof, wherein, T comprises a compound with anti-tumor activity.

**21.** The compound according to any one of claims 1-20, or a tautomer, mesomer, racemate, enantiomer, and diastereomer thereof, or a mixture thereof, or a pharmaceutically acceptable salt, prodrug or solvate thereof, wherein, T comprises a topoisomerase inhibitor.

**22.** The compound according to any one of claims 1-21, or a tautomer, mesomer, racemate, enantiomer, and diastereomer thereof, or a mixture thereof, or a pharmaceutically acceptable salt, prodrug or solvate thereof, wherein, T comprises camptothecin and non-camptothecin topoisomerase I inhibitors.

**23.** The compound according to any one of claims 1-22, or a tautomer, mesomer, racemate, enantiomer, and diastereomer thereof, or a mixture thereof, or a pharmaceutically acceptable salt, prodrug or solvate thereof,
wherein, T is a structure selected from the group consisting of

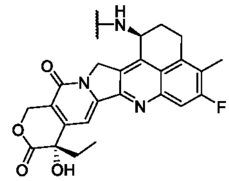
,

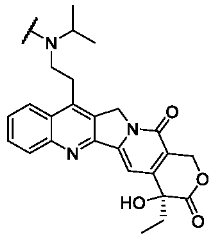
, and
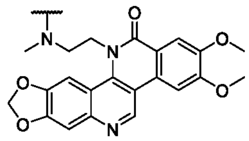
.

24. The compound according to any one of claims 1-23, or a tautomer, mesomer, racemate, enantiomer, and diastereomer thereof, or a mixture thereof, or a pharmaceutically acceptable salt, prodrug or solvate thereof,
wherein, the Ab comprises an anti-HER2 antibody or an antigen-binding fragment thereof.

25. The compound according to claim 24, or a tautomer, mesomer, racemate, enantiomer, and diastereomer thereof, or a mixture thereof, or a pharmaceutically acceptable salt, prodrug or solvate thereof,
wherein, the antibody is selected from the group consisting of a murine antibody, a chimeric antibody, a humanized antibody, and a fully human antibody.

26. The compound according to any one of claims 24-25, or a tautomer, mesomer, racemate, enantiomer, and diastereomer thereof, or a mixture thereof, or a pharmaceutically acceptable salt, prodrug or solvate thereof,
wherein, the antibody comprises a monoclonal antibody.

27. The compound according to any one of claims 24-26, or a tautomer, mesomer, racemate, enantiomer, and diastereomer thereof, or a mixture thereof, or a pharmaceutically acceptable salt, prodrug or solvate thereof,
wherein, the antibody comprises a bispecific antibody.

28. The compound according to any one of claims 24-27, or a tautomer, mesomer, racemate, enantiomer, and diastereomer thereof, or a mixture thereof, or a pharmaceutically acceptable salt, prodrug or solvate thereof,
wherein, the antigen-binding fragment is selected from the group consisting of Fab, Fab', an Fv fragment, $F(ab')_2$, $F(ab)_2$, scFv, di-scFv, VHH, and dAb.

29. The compound according to any one of claims 1-28, or a tautomer, mesomer, racemate, enantiomer, and diastereomer thereof, or a mixture thereof, or a pharmaceutically acceptable salt, prodrug or solvate thereof,
wherein, the heavy chains HCDR1, HCDR2 and HCDR3 and the light chains LCDR1, LCDR2 and LCDR3 of the Ab comprise the heavy chains HCDR1, HCDR2 and HCDR3 and the light chains LCDR1, LCDR2 and LCDR3 of the anti-HER2 antibody, respectively.

30. The compound according to any one of claims 1-29, or a tautomer, mesomer, racemate, enantiomer, and diastereomer thereof, or a mixture thereof, or a pharmaceutically acceptable salt, prodrug or solvate thereof,
wherein, the heavy chains HCDR1, HCDR2 and HCDR3 and the light chains LCDR1, LCDR2 and LCDR3 of the Ab comprise the heavy chains HCDR1, HCDR2 and HCDR3 and the light chains LCDR1, LCDR2 and LCDR3 of Trastuzumab or Pertuzumab, respectively.

31. The compound according to any one of claims 1-30, or a tautomer, mesomer, racemate, enantiomer, and diastereomer thereof, or a mixture thereof, or a pharmaceutically acceptable salt, prodrug or solvate thereof,
wherein, the heavy chain variable region VH and the light chain variable region VL of the Ab comprise the heavy chain variable region VH and the light chain variable region VL of the anti-HER2 antibody, respectively.

**32.** The compound according to any one of claims 1-31, or a tautomer, mesomer, racemate, enantiomer, and diastereomer thereof, or a mixture thereof, or a pharmaceutically acceptable salt, prodrug or solvate thereof, wherein, the heavy chain variable region VH and the light chain variable region VL of the Ab comprise the heavy chain variable region VH and the light chain variable region VL of Trastuzumab or Pertuzumab, respectively.

**33.** The compound according to any one of claims 1-32, or a tautomer, mesomer, racemate, enantiomer, and diastereomer thereof, or a mixture thereof, or a pharmaceutically acceptable salt, prodrug or solvate thereof, wherein, the heavy chain and the light chain of the Ab comprise the heavy chain and the light chain of the anti-HER2 antibody, respectively.

**34.** The compound according to any one of claims 1-33, or a tautomer, mesomer, racemate, enantiomer, and diastereomer thereof, or a mixture thereof, or a pharmaceutically acceptable salt, prodrug or solvate thereof, wherein, the heavy chain and the light chain of the Ab comprise the heavy chain and the light chain of Trastuzumab or Pertuzumab, respectively.

**35.** The compound according to any one of claims 1-34, or a tautomer, mesomer, racemate, enantiomer, and diastereomer thereof, or a mixture thereof, or a pharmaceutically acceptable salt, prodrug or solvate thereof, wherein, the Ab comprises Trastuzumab or Pertuzumab.

**36.** The compound according to any one of claims 1-35, or a tautomer, mesomer, racemate, enantiomer, and diastereomer thereof, or a mixture thereof, or a pharmaceutically acceptable salt, prodrug or solvate thereof, wherein, the m is determined by a method selected from the group consisting of hydrophobic chromatography, sodium dodecyl sulfate polyacrylamide gel electrophoresis, and liquid phase mass spectrometry.

**37.** A compound, or a tautomer, mesomer, racemate, enantiomer, and diastereomer thereof, or a mixture thereof, or a pharmaceutically acceptable salt, prodrug or solvate thereof, wherein, the compound comprises a structure selected from the group consisting of

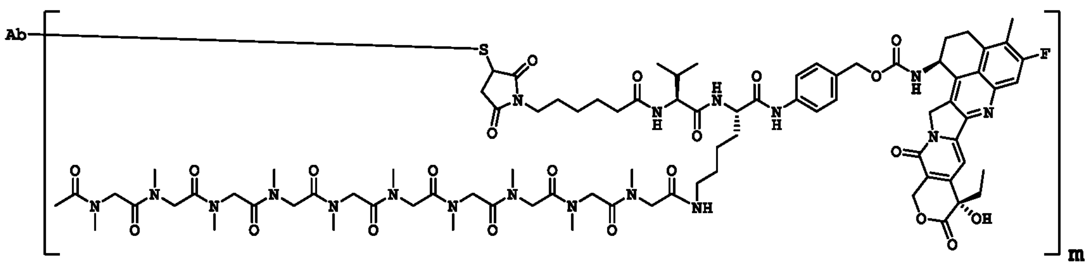

(C-HER2-1),

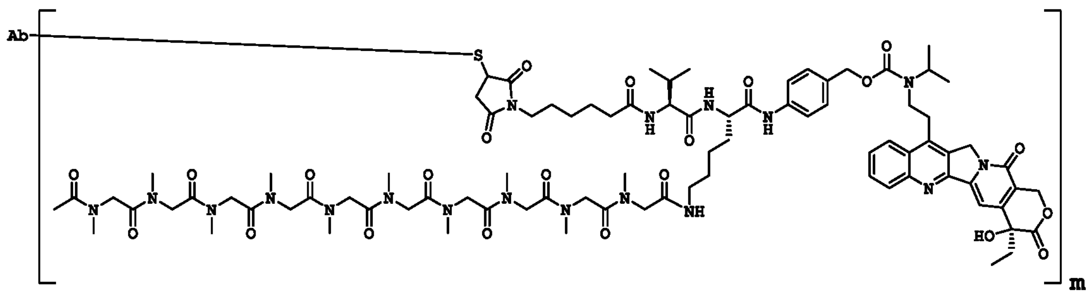

(C-HER2-2),

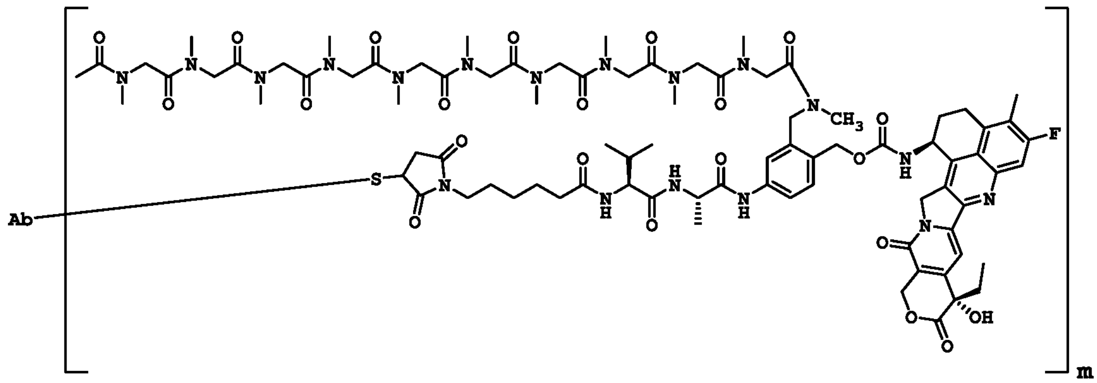

(C-HER2-3),

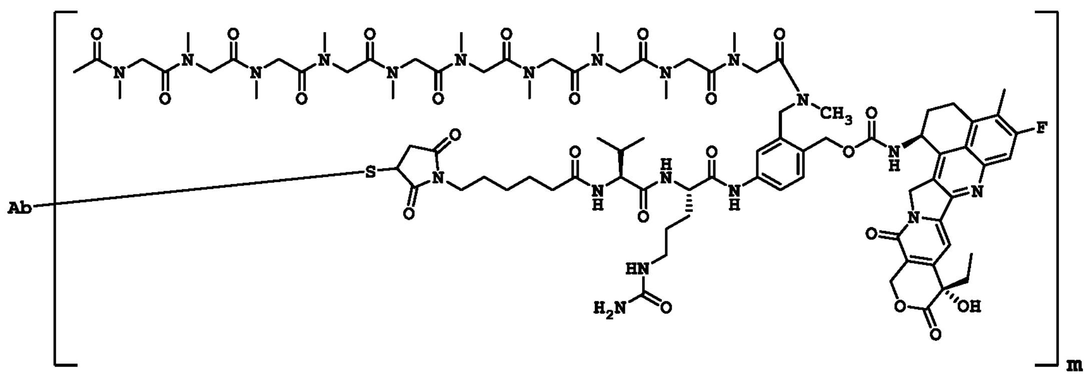

(C-HER2-4),

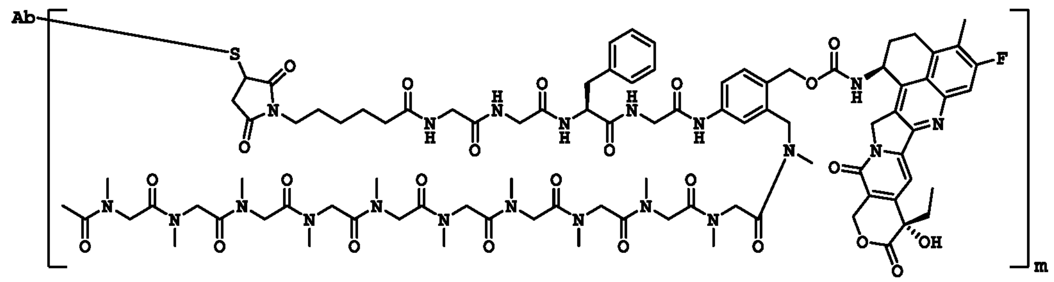

(C-HER2-5),

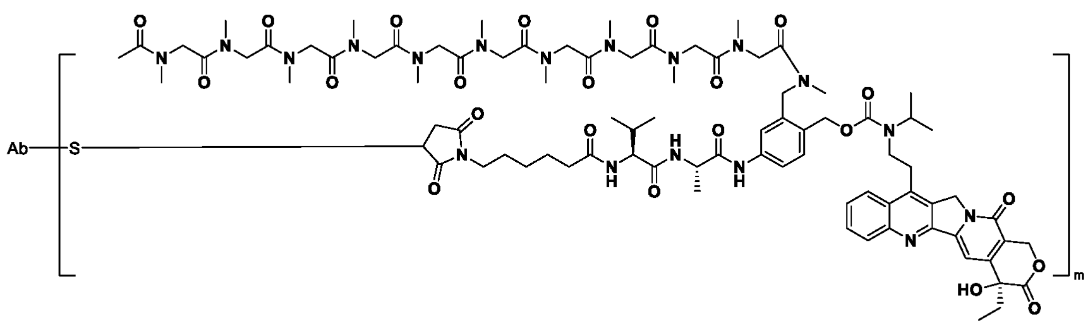

(C-HER2-6),

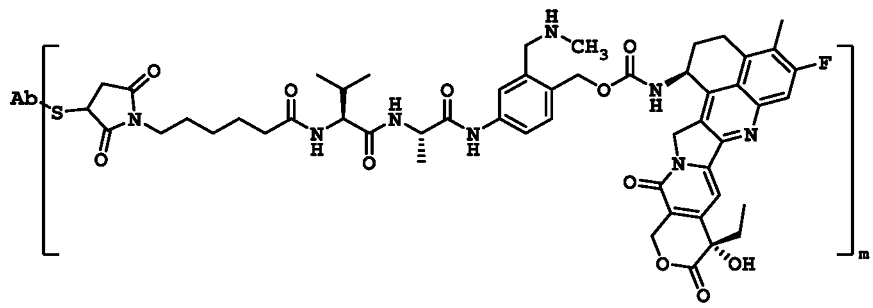

(C-HER2-7),

and

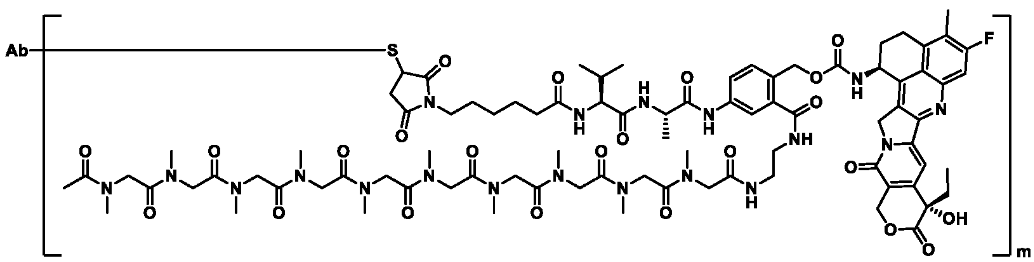

(C-HER2-8),

Ab is a ligand capable of binding to HER2, and m is a number from 1 to 8.

**38.** A pharmaceutical composition comprising the compound of any one of claims 1-37, or a tautomer, mesomer, racemate, enantiomer, and diastereomer thereof, or a mixture thereof, or a pharmaceutically acceptable salt, prodrug or solvate thereof, and optionally a pharmaceutically acceptable carrier.

**39.** Use of the compound of any one of claims 1-37, or a tautomer, mesomer, racemate, enantiomer, and diastereomer thereof, or a mixture thereof, or a pharmaceutically acceptable salt, prodrug or solvate thereof, and/or the pharmaceutical composition according to claim 38 in the preparation of a drug for treating and/or preventing a tumor.

**40.** The use according to claim 39, wherein the tumor is a tumor associated with the expression of HER2.

**41.** The use according to claim 40, wherein the tumor associated with the expression of the target comprises a tumor with high expression of the target and/or a tumor positive for the target.

**42.** The use according to any one of claims 39-41, wherein the tumor comprises a solid tumor and/or a hematological tumor.

**43.** The use according to any one of claims 39-42, wherein the tumor is selected from the group consisting of lung cancer, urethral cancer, colorectal cancer, prostate cancer, ovarian cancer, pancreatic cancer, breast cancer, bladder cancer, gastric cancer, gastrointestinal stromal tumor, cervical cancer, esophagus cancer, squamous cell carcinoma, peritoneal carcinoma, liver cancer, hepatocellular carcinoma, colon cancer, rectal cancer, colorectal cancer, endometrial cancer, uterine cancer, salivary gland cancer, renal cancer, vulva cancer, thyroid cancer, penile cancer, leukemia, malignant lymphoma, plasmocytoma, myeloma, or sarcoma, lung cancer, urethral cancer, colorectal cancer, prostate cancer, ovarian cancer, pancreatic cancer, breast cancer, bladder cancer, gastric cancer, gastrointestinal stromal tumor, cervical cancer, esophagus cancer, squamous cell carcinoma, peritoneal carcinoma, liver cancer, hepatocellular carcinoma, colon cancer, rectal cancer, colorectal cancer, endometrial cancer, uterine cancer, salivary gland cancer, renal cancer, vulva cancer, thyroid cancer, penile cancer, leukemia, malignant lymphoma, plasmocytoma, myeloma, and sarcoma.

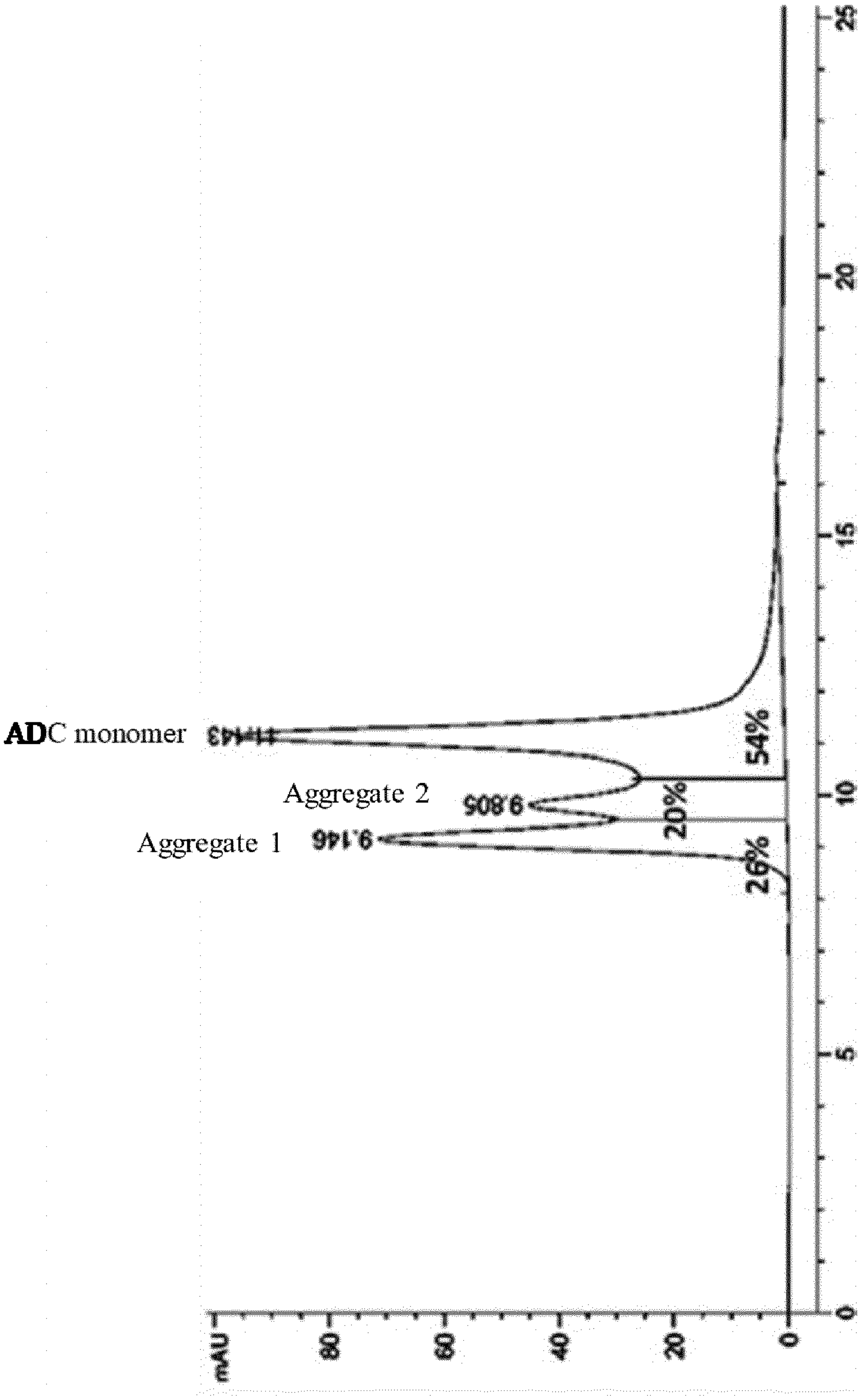
ADC monomer
11.143
Aggregate 2
9.805
Aggregate 1
9.146
54%
20%
26%
mAU
80
60
40
20
0
0
5
10
15
20
25

FIG. 1

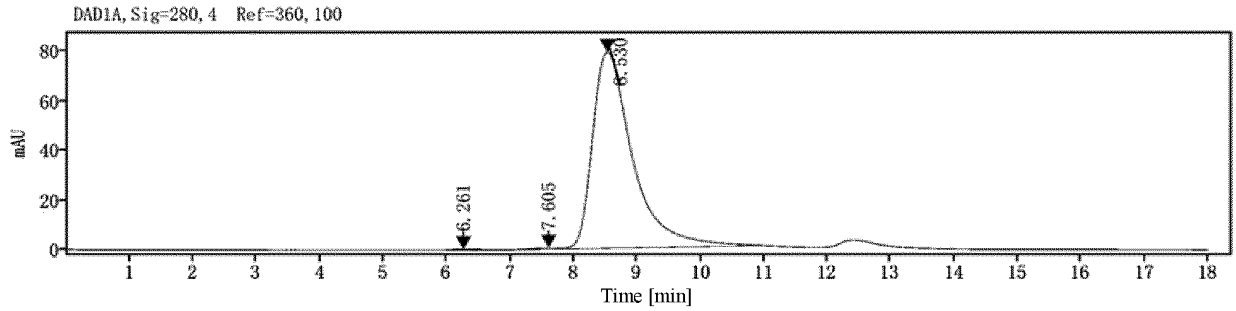
DAD1A, Sig=280, 4 Ref=360, 100
mAU
Time [min]
6.261
7.605
8.530

FIG. 2

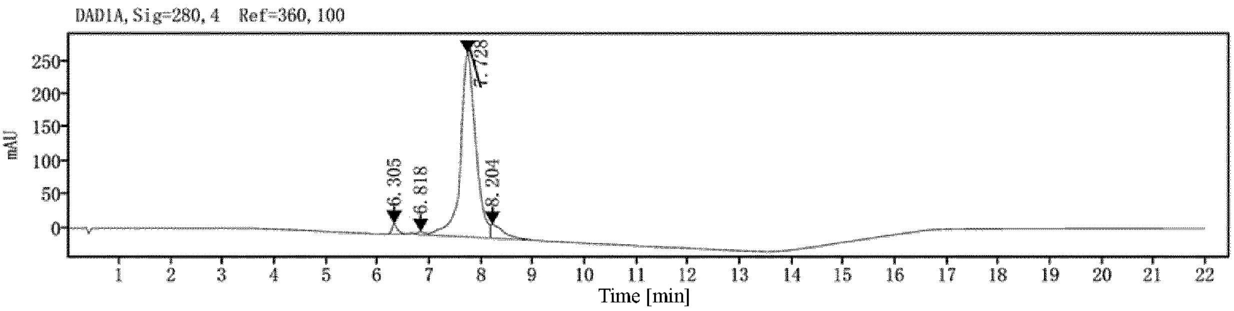
DAD1A, Sig=280, 4 Ref=360, 100
mAU
Time [min]
6.305
6.818
7.728
8.204

FIG. 3

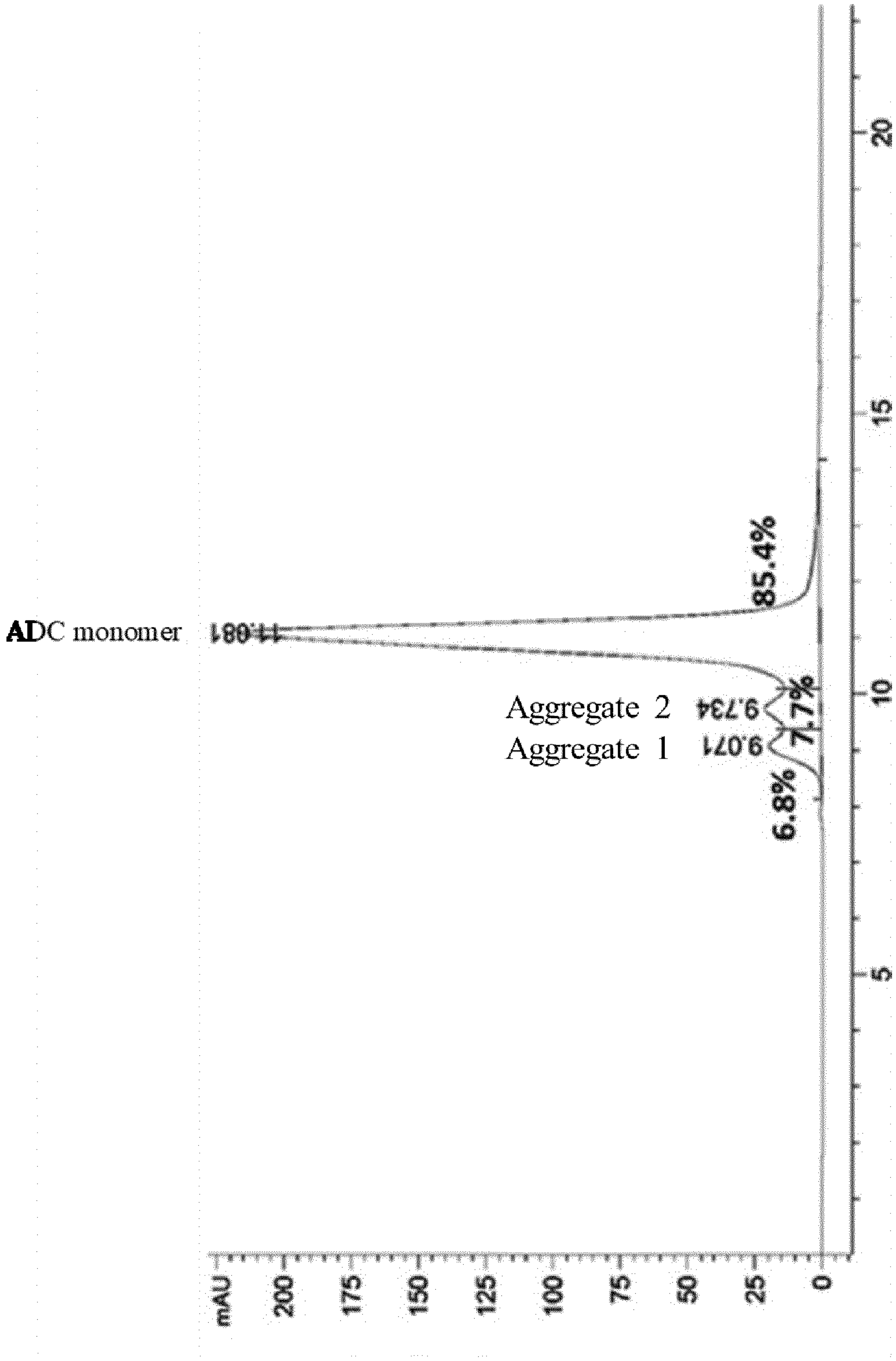
ADC monomer
11.081
85.4%
Aggregate 2
9.734
7.7%
Aggregate 1
9.071
6.8%
mAU
200
175
150
125
100
75
50
25
0
5
10
15
20

FIG. 4

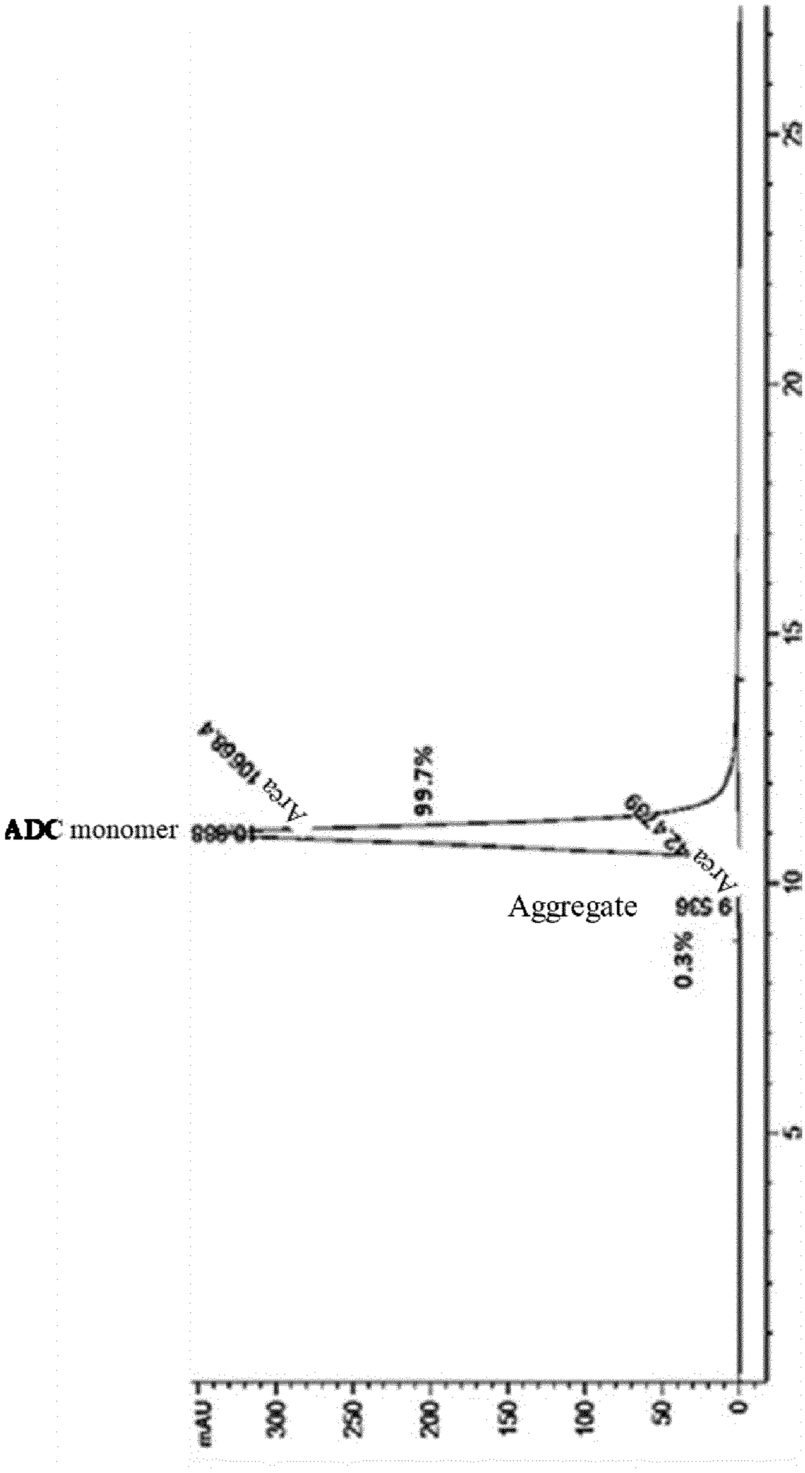
ADC monomer
Aggregate
99.7%
0.3%
mAU
300
250
200
150
100
50
0
5
10
15
20
25

FIG. 5

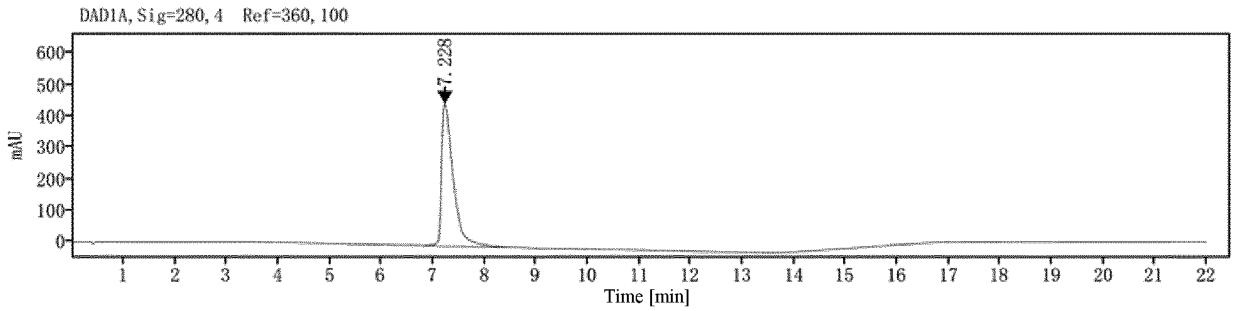


FIG. 6

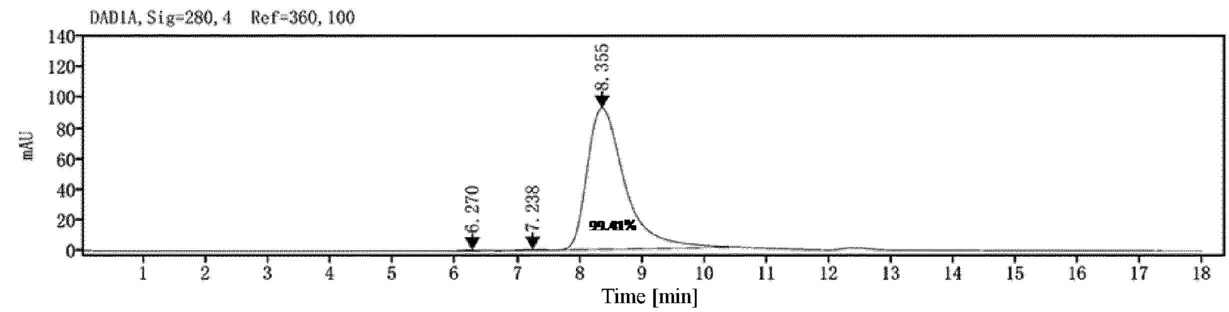


FIG. 7

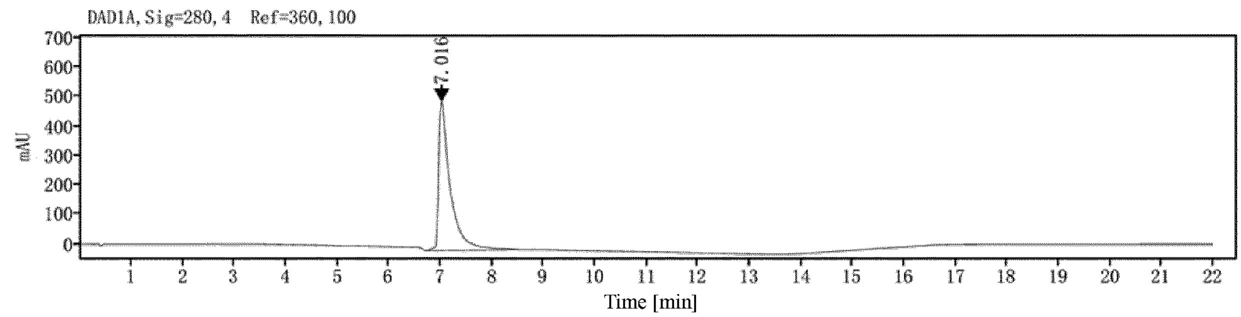


FIG. 8

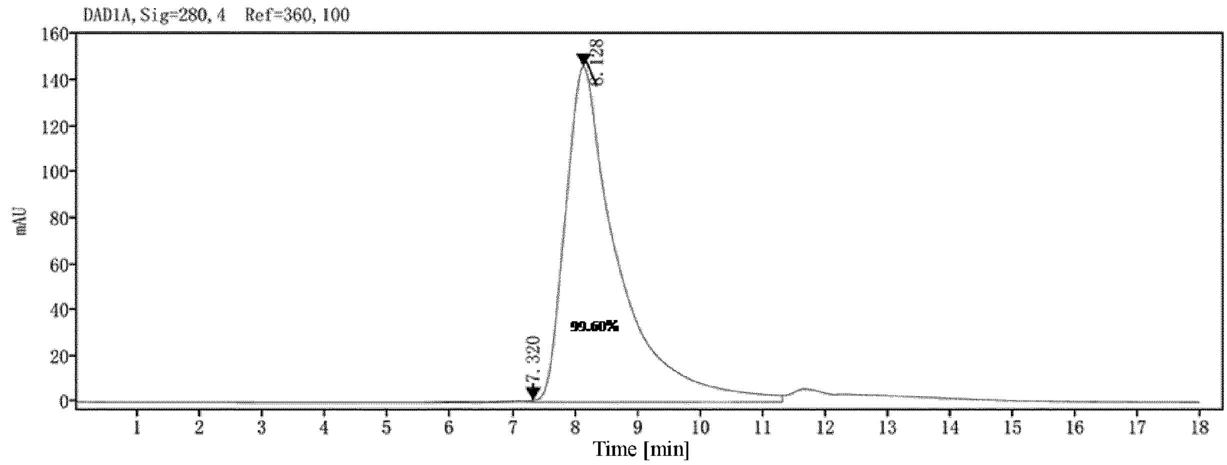


FIG. 9

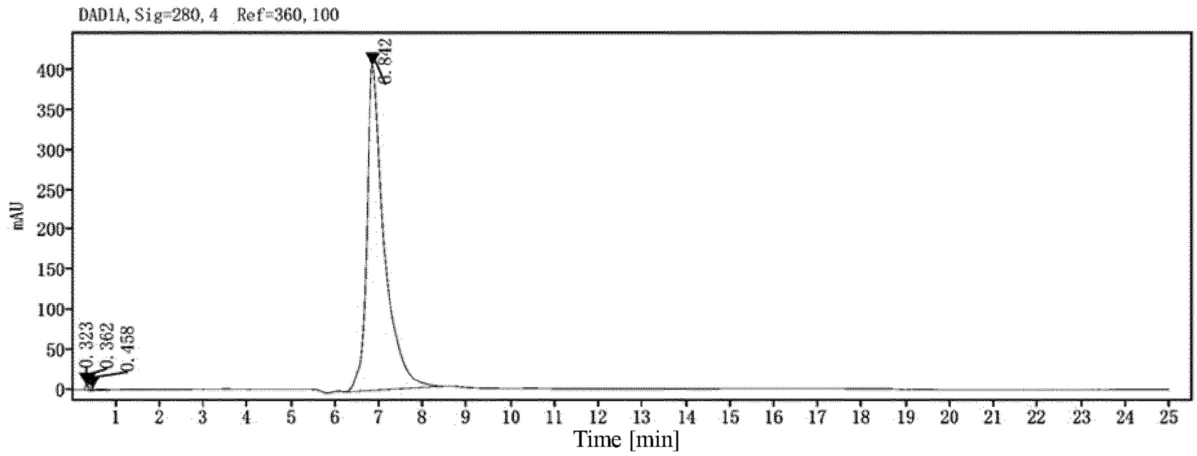


FIG. 10

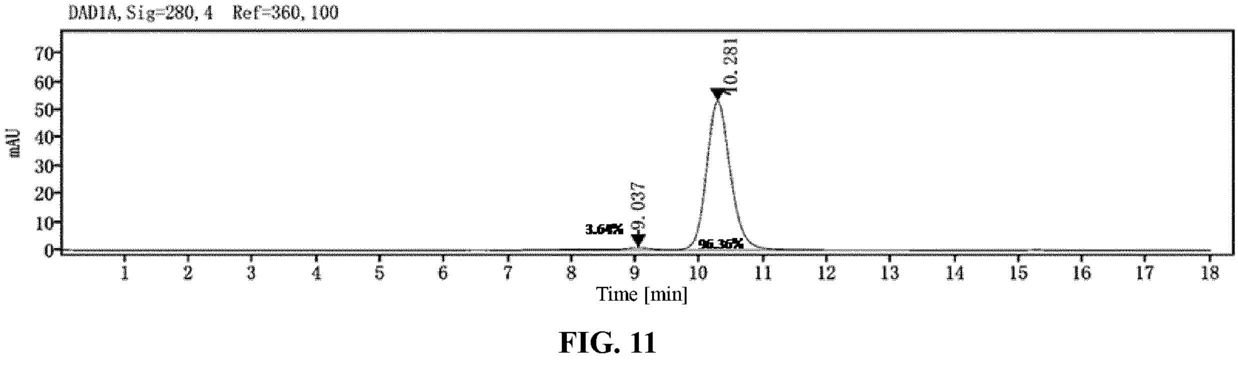


FIG. 11

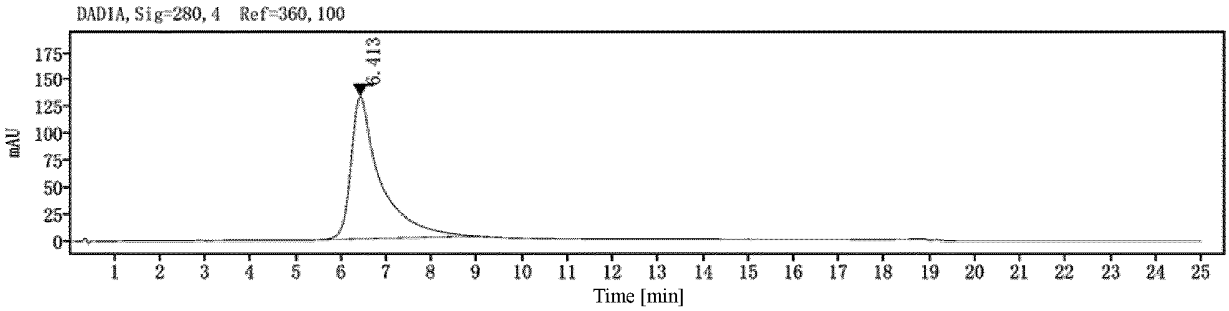


FIG. 12

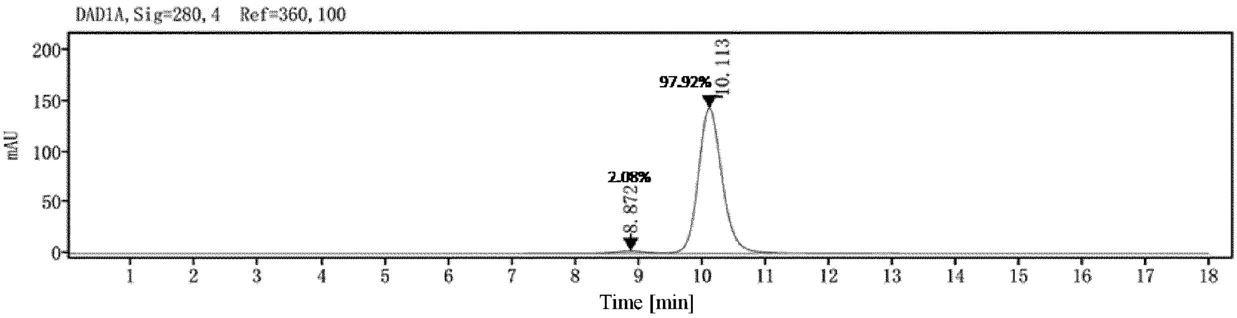


FIG. 13

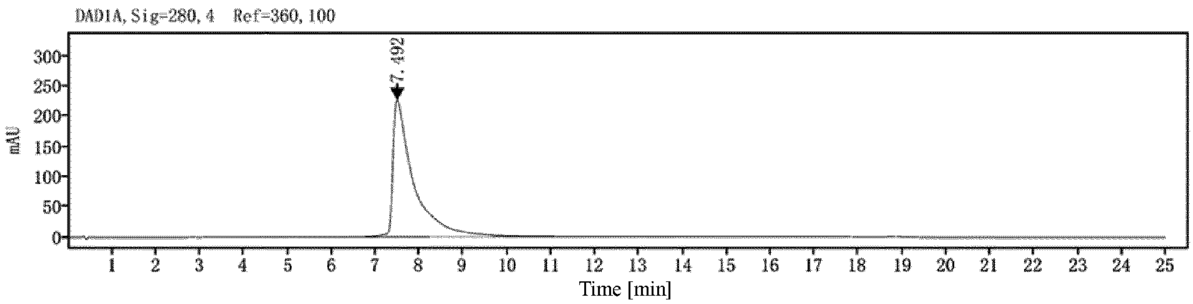


FIG. 14

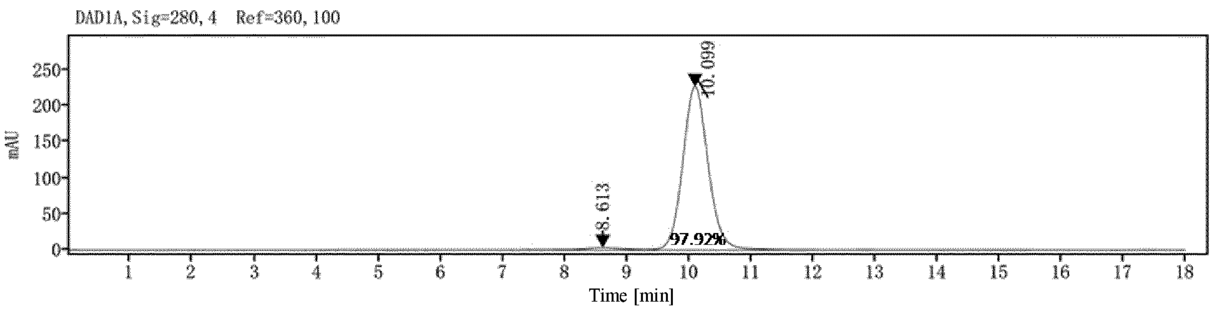


FIG. 15

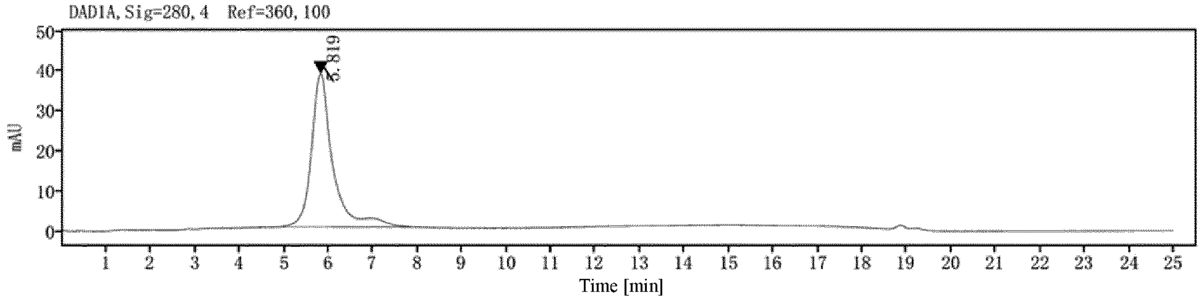


FIG. 16

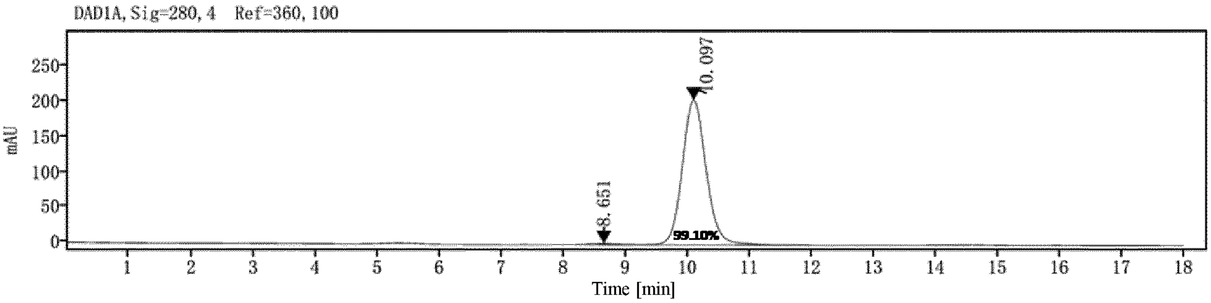


FIG. 17

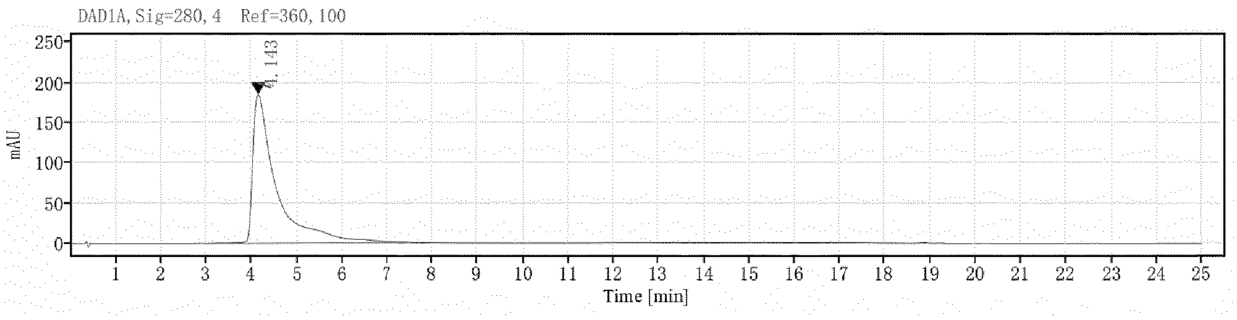


FIG. 18

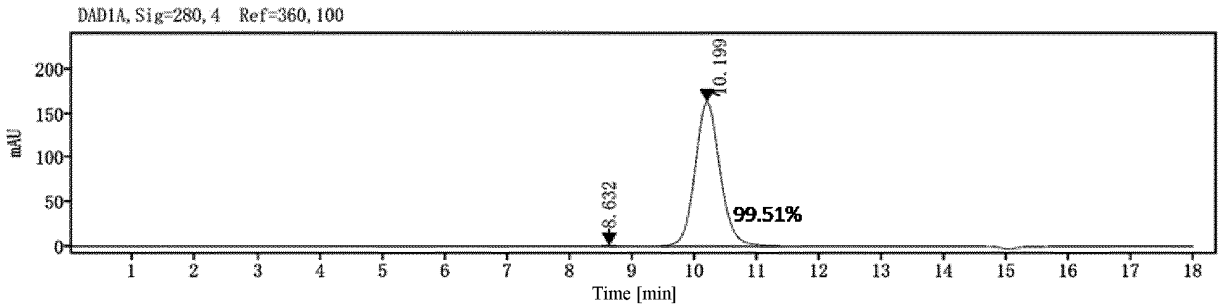


FIG. 19

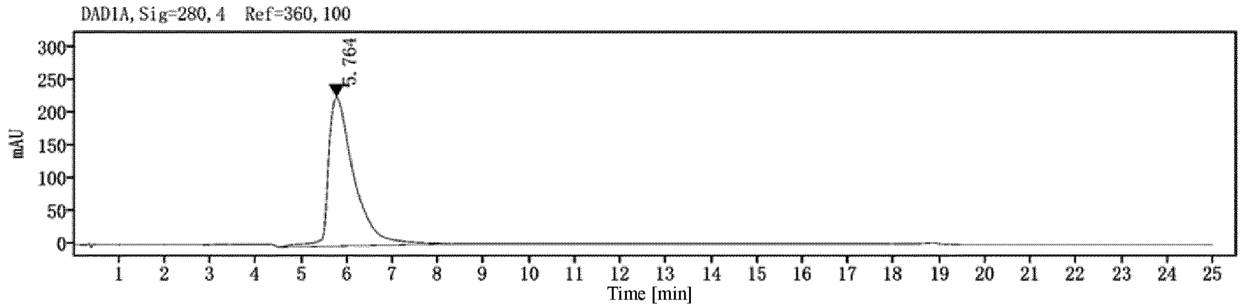


FIG. 20

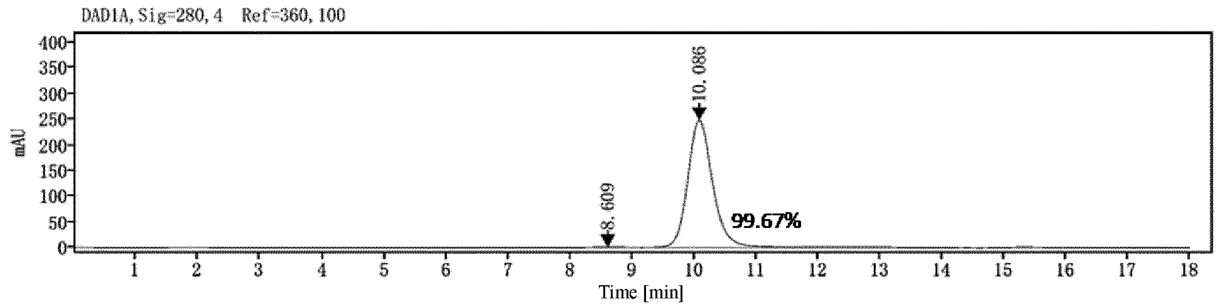


FIG. 21

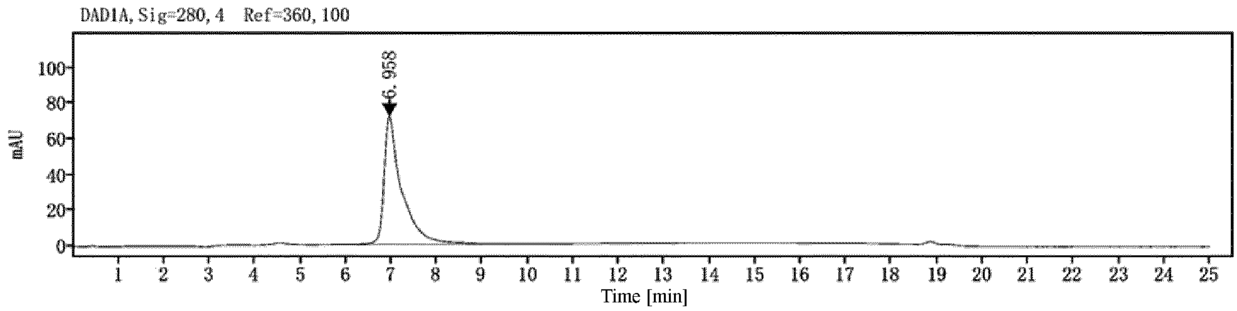


FIG. 22

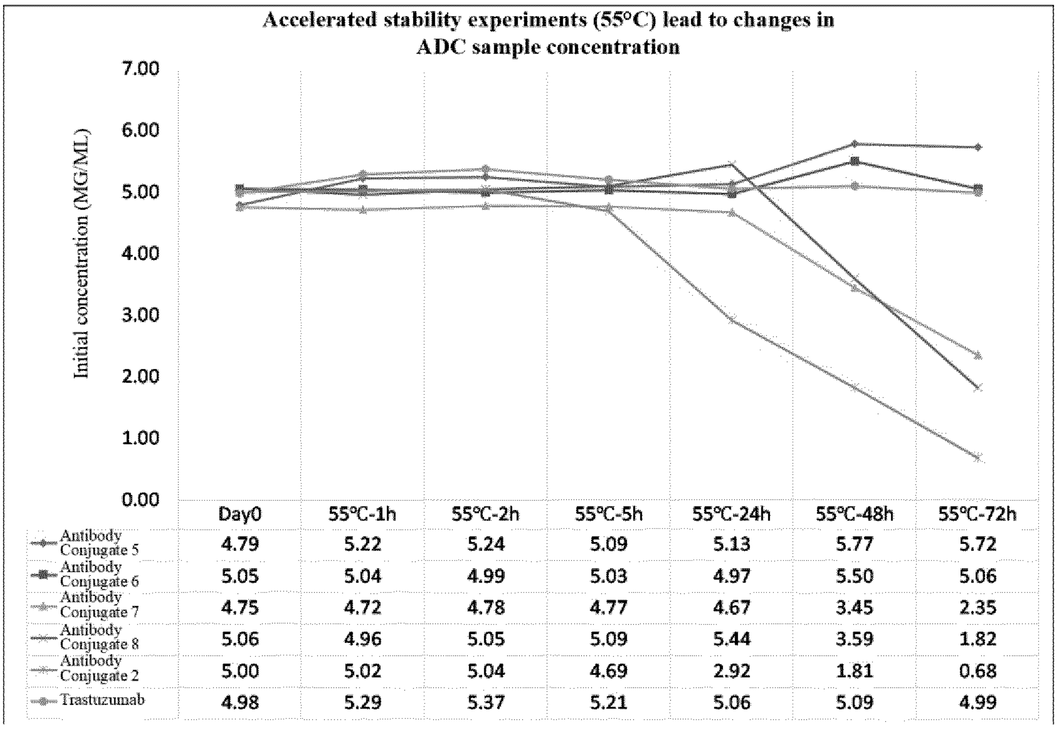


| | Day0 | 55℃-1h | 55℃-2h | 55℃-5h | 55℃-24h | 55℃-48h | 55℃-72h |
|---|---|---|---|---|---|---|---|
| Antibody Conjugate 5 | 4.79 | 5.22 | 5.24 | 5.09 | 5.13 | 5.77 | 5.72 |
| Antibody Conjugate 6 | 5.05 | 5.04 | 4.99 | 5.03 | 4.97 | 5.50 | 5.06 |
| Antibody Conjugate 7 | 4.75 | 4.72 | 4.78 | 4.77 | 4.67 | 3.45 | 2.35 |
| Antibody Conjugate 8 | 5.06 | 4.96 | 5.05 | 5.09 | 5.44 | 3.59 | 1.82 |
| Antibody Conjugate 2 | 5.00 | 5.02 | 5.04 | 4.69 | 2.92 | 1.81 | 0.68 |
| Trastuzumab | 4.98 | 5.29 | 5.37 | 5.21 | 5.06 | 5.09 | 4.99 |

FIG. 23

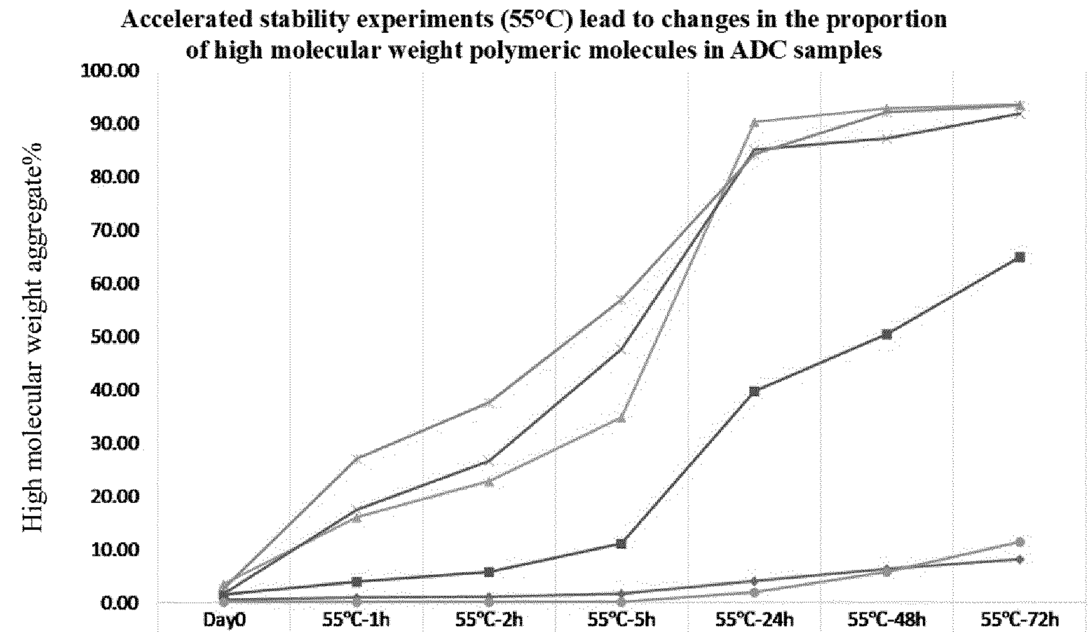


| | Day0 | 55℃-1h | 55℃-2h | 55℃-5h | 55℃-24h | 55℃-48h | 55℃-72h |
|---|---|---|---|---|---|---|---|
| Antibody Conjugate 5 | 0.68 | 0.98 | 1.24 | 1.71 | 4.10 | 6.38 | 8.22 |
| Antibody Conjugate 6 | 1.60 | 3.98 | 5.89 | 11.23 | 39.80 | 50.45 | 64.94 |
| Antibody Conjugate 7 | 3.63 | 16.11 | 22.80 | 34.80 | 90.25 | 92.87 | 93.58 |
| Antibody Conjugate 8 | 1.79 | 17.45 | 26.65 | 47.67 | 85.07 | 87.20 | 91.83 |
| Antibody Conjugate 2 | 2.67 | 27.02 | 37.65 | 57.00 | 84.22 | 92.18 | 93.44 |
| Trastuzumab | 0.23 | 0.24 | 0.22 | 0.21 | 1.95 | 5.88 | 11.48 |

FIG. 24

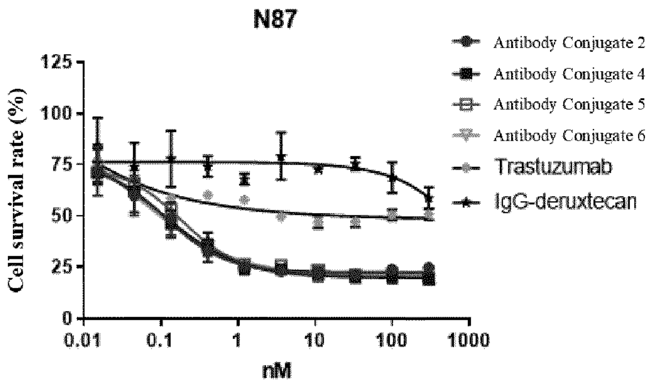


| Conjugate No. | NCI-N87 (HER2+++) |
|---|---|
| | $IC_{50}$ (nM) |
| Antibody Conjugate 2 | 0.117 |
| Antibody Conjugate 4 | 0.113 |
| Antibody Conjugate 5 | 0.187 |
| Antibody Conjugate 6 | 0.049 |
| Trastuzumab monoclonal antibody | > 500 |
| IgG-deruxtecan | > 500 |

FIG. 25

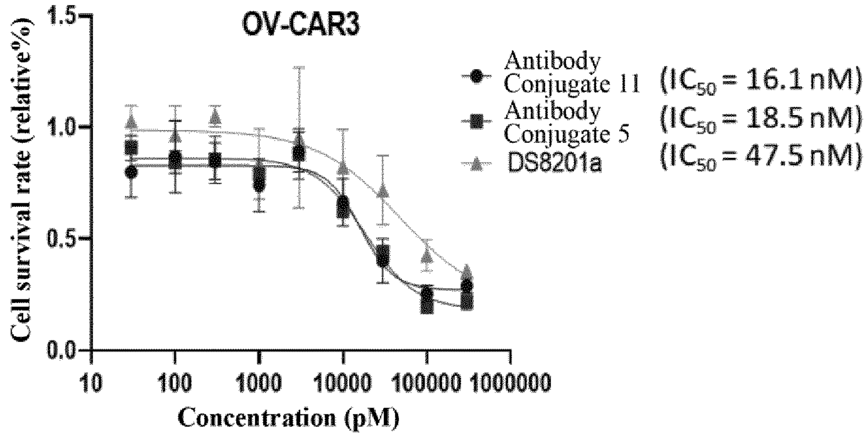


FIG. 26

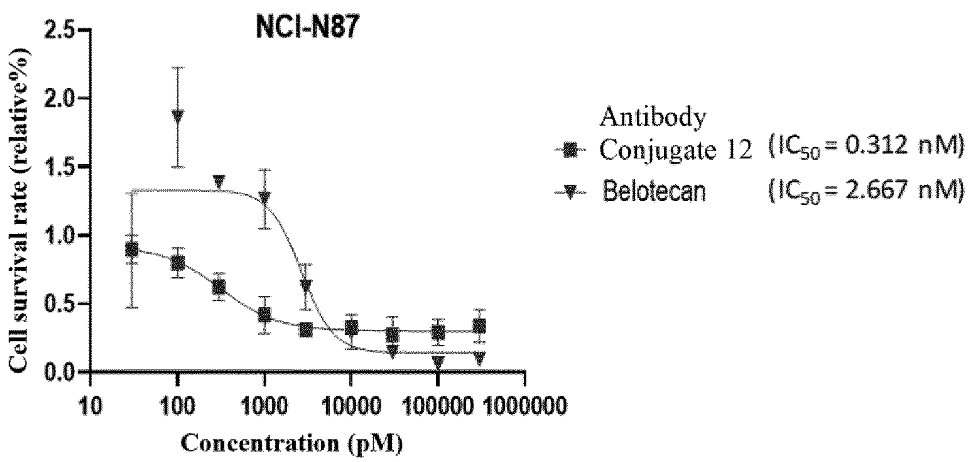


FIG. 27

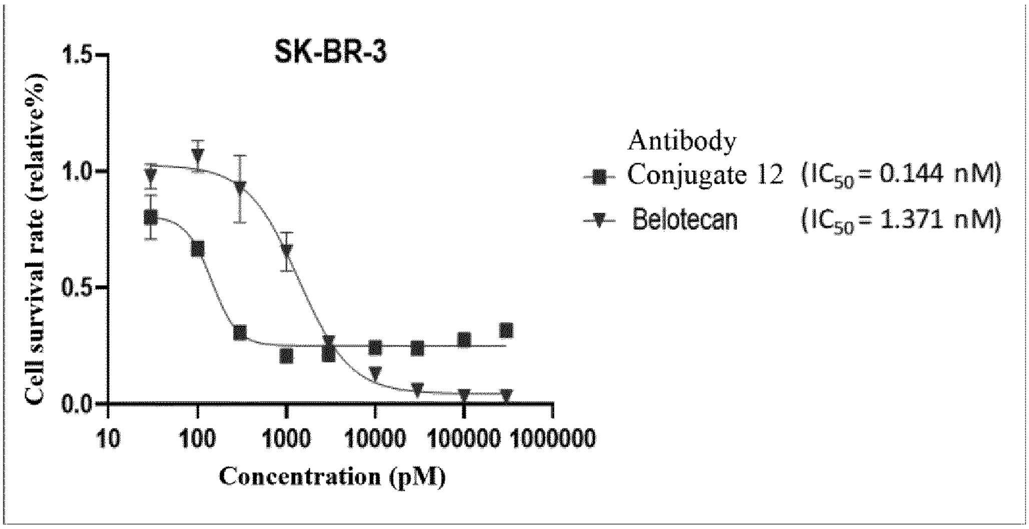
SK-BR-3
Cell survival rate (relative%)
1.5
1.0
0.5
0.0
10
100
1000
10000
100000
1000000
Concentration (pM)
Antibody
Conjugate 12 ($IC_{50}$ = 0.144 nM)
Belotecan ($IC_{50}$ = 1.371 nM)

FIG. 28

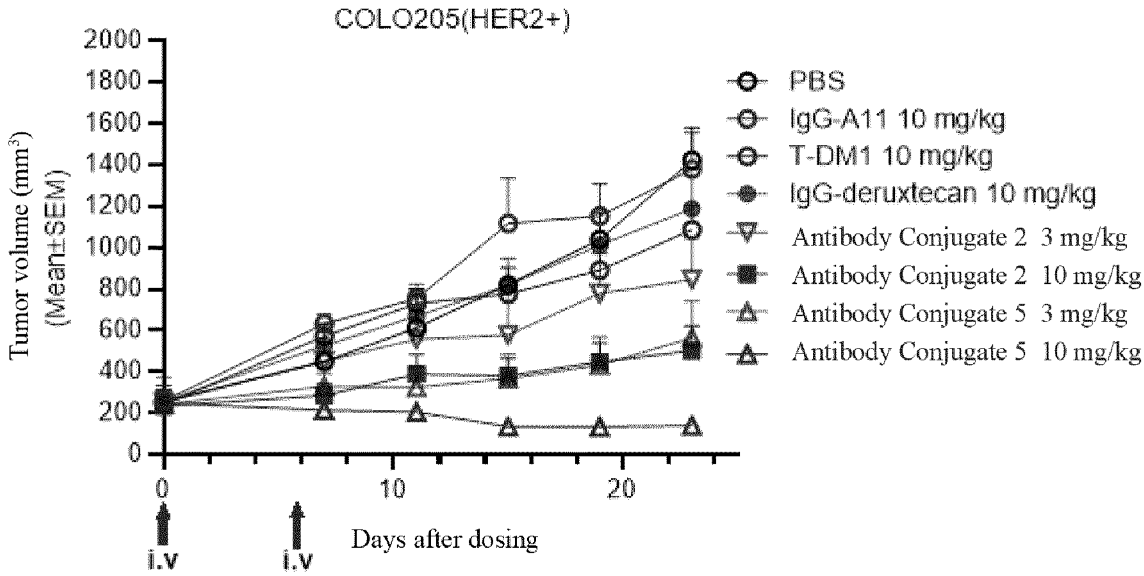
COLO205(HER2+)
Tumor volume ($mm^3$)
(Mean±SEM)
2000
1800
1600
1400
1200
1000
800
600
400
200
0
0
10
20
i.v
i.v
Days after dosing
PBS
IgG-A11 10 mg/kg
T-DM1 10 mg/kg
IgG-deruxtecan 10 mg/kg
Antibody Conjugate 2 3 mg/kg
Antibody Conjugate 2 10 mg/kg
Antibody Conjugate 5 3 mg/kg
Antibody Conjugate 5 10 mg/kg

FIG. 29

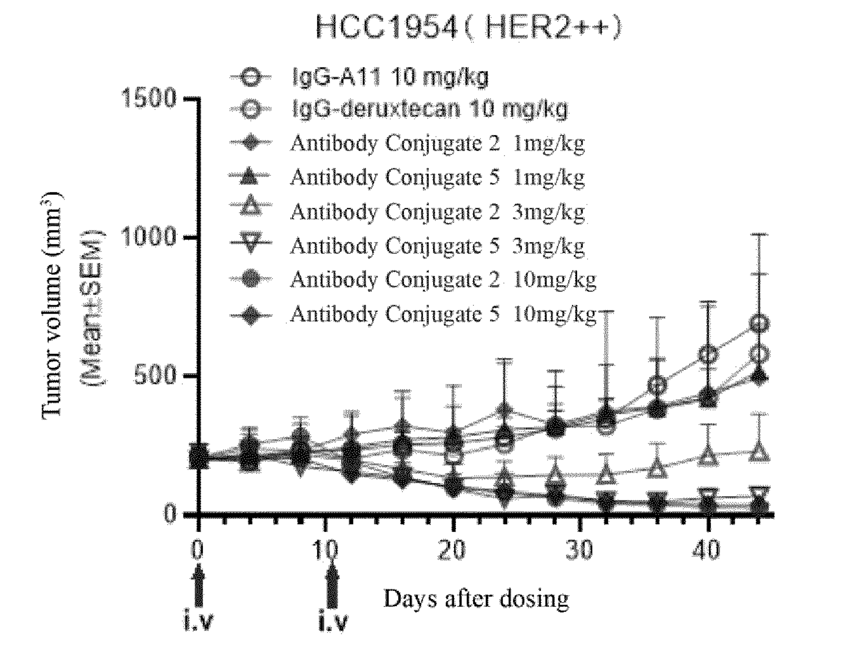


FIG. 30

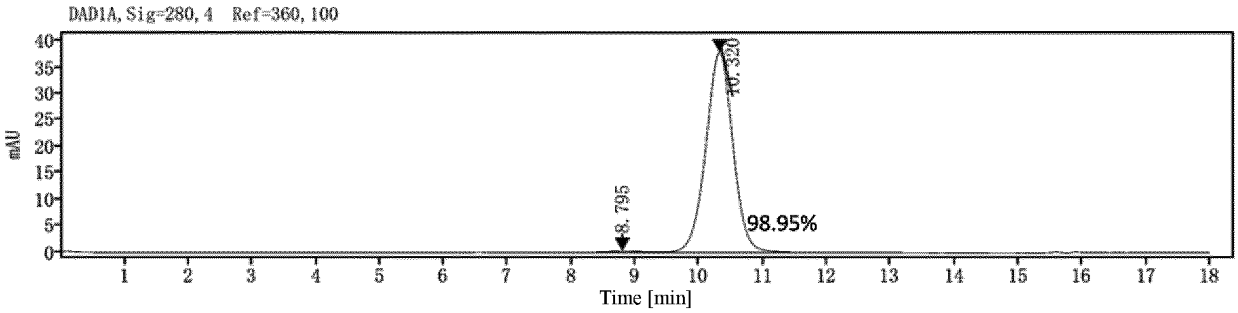


FIG. 31

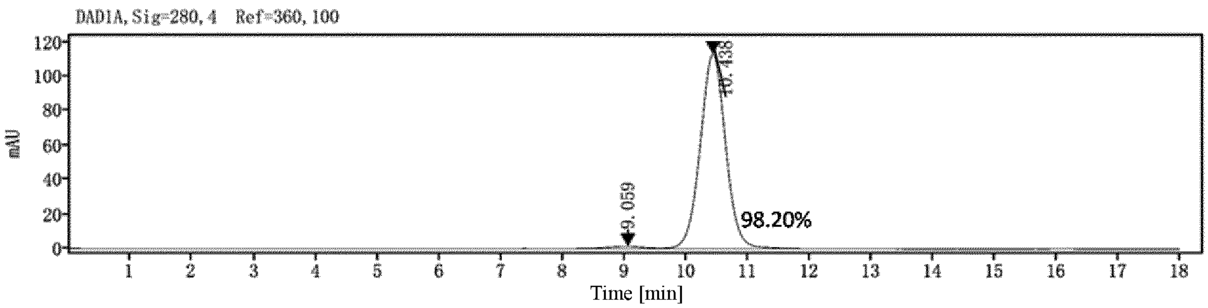


FIG. 32

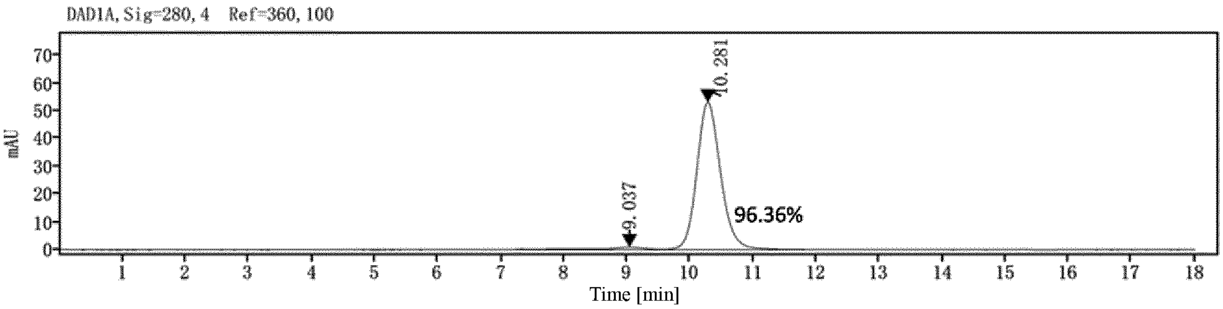
DAD1A, Sig=280, 4 Ref=360, 100
mAU
70
60
50
40
30
20
10
0
10.281
9.037
96.36%
1 2 3 4 5 6 7 8 9 10 11 12 13 14 15 16 17 18
Time [min]

FIG. 33

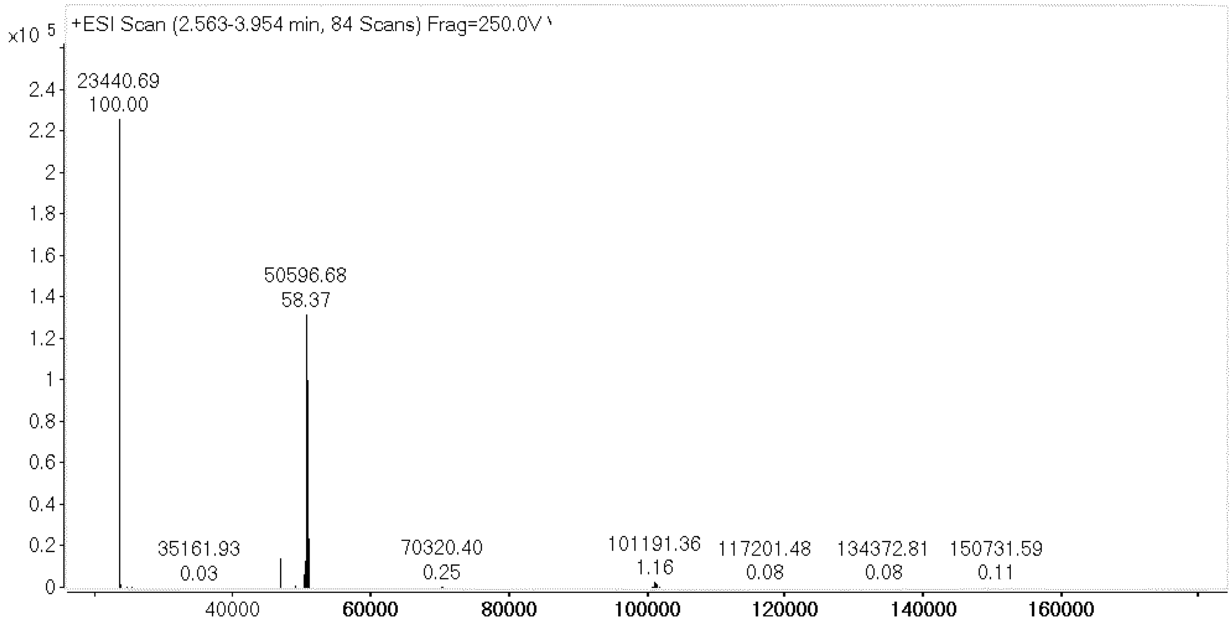
x10 5
+ESI Scan (2.563-3.954 min, 84 Scans) Frag=250.0V
2.4
2.2
2
1.8
1.6
1.4
1.2
1
0.8
0.6
0.4
0.2
0
23440.69
100.00
50596.68
58.37
35161.93
0.03
70320.40
0.25
101191.36
1.16
117201.48
0.08
134372.81
0.08
150731.59
0.11
40000 60000 80000 100000 120000 140000 160000

FIG. 34

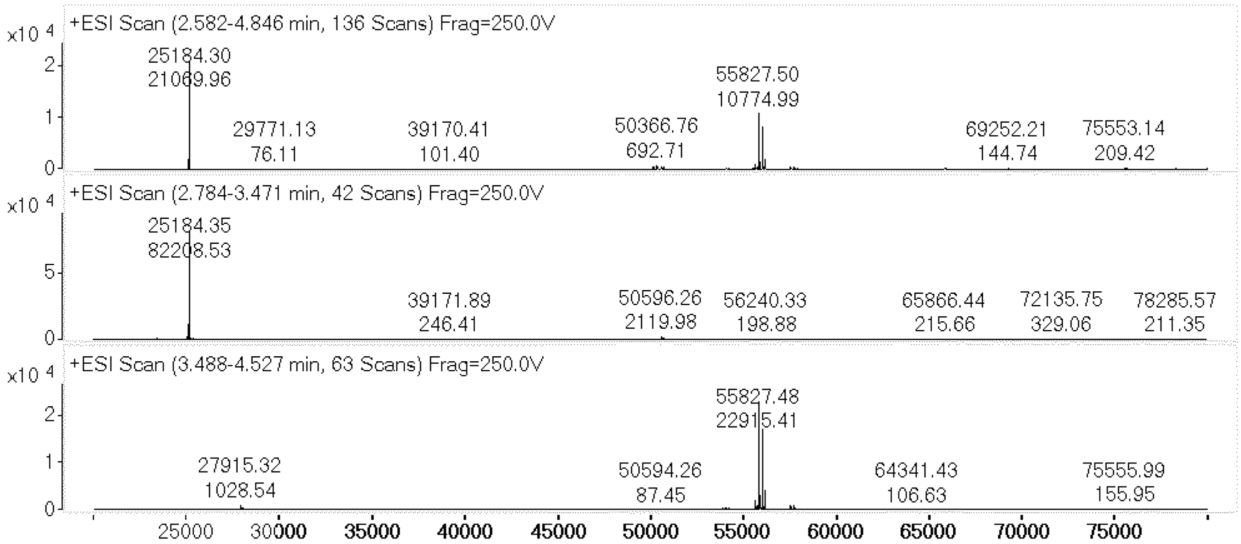
+ESI Scan (2.582-4.846 min, 136 Scans) Frag=250.0V
25184.30
21069.96
55827.50
10774.99
29771.13
76.11
39170.41
101.40
50366.76
692.71
69252.21
144.74
75553.14
209.42
+ESI Scan (2.784-3.471 min, 42 Scans) Frag=250.0V
25184.35
82208.53
39171.89
246.41
50596.26
2119.98
56240.33
198.88
65866.44
215.66
72135.75
329.06
78285.57
211.35
+ESI Scan (3.488-4.527 min, 63 Scans) Frag=250.0V
55827.48
22915.41
27915.32
1028.54
50594.26
87.45
64341.43
106.63
75555.99
155.95
25000 30000 35000 40000 45000 50000 55000 60000 65000 70000 75000

FIG. 35

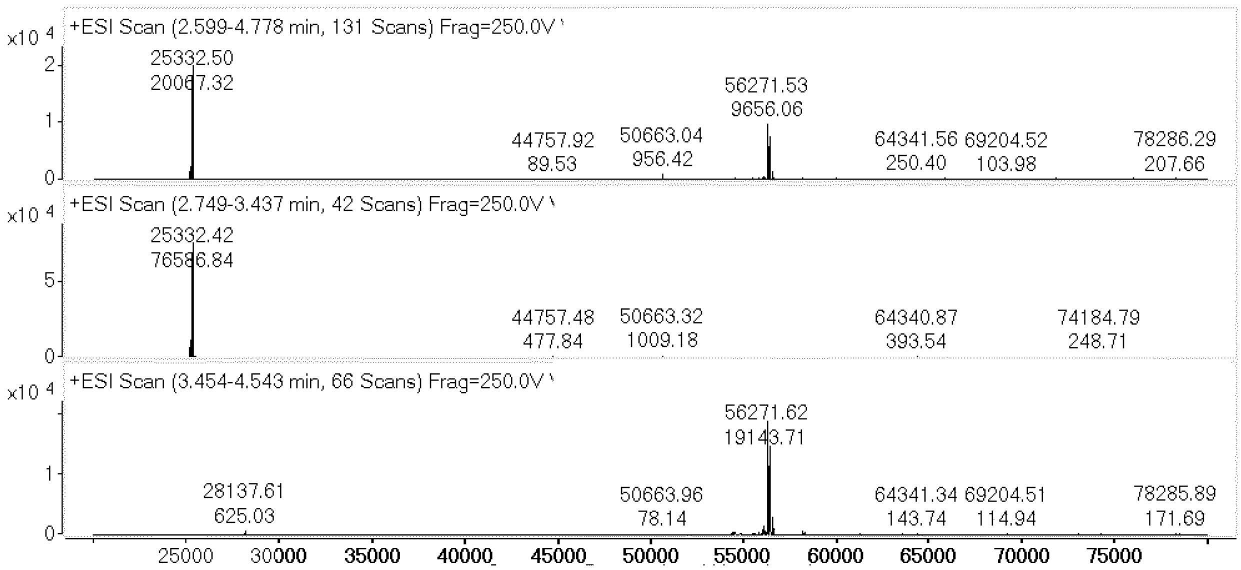
+ESI Scan (2.599-4.778 min, 131 Scans) Frag=250.0V
25332.50
20067.32
56271.53
9656.06
44757.92
89.53
50663.04
956.42
64341.56
250.40
69204.52
103.98
78286.29
207.66
+ESI Scan (2.749-3.437 min, 42 Scans) Frag=250.0V
25332.42
76586.84
44757.48
477.84
50663.32
1009.18
64340.87
393.54
74184.79
248.71
+ESI Scan (3.454-4.543 min, 66 Scans) Frag=250.0V
56271.62
19143.71
28137.61
625.03
50663.96
78.14
64341.34
143.74
69204.51
114.94
78285.89
171.69
25000 30000 35000 40000 45000 50000 55000 60000 65000 70000 75000

FIG. 36

**INTERNATIONAL SEARCH REPORT**

International application No.
**PCT/CN2022/089725**

**A. CLASSIFICATION OF SUBJECT MATTER**

A61K 47/68(2017.01)i; A61K 47/54(2017.01)i; C07K 5/062(2006.01)i; A61P 35/00(2006.01)i

According to International Patent Classification (IPC) or to both national classification and IPC

**B. FIELDS SEARCHED**

Minimum documentation searched (classification system followed by classification symbols)

A61K; C07K; A61P

Documentation searched other than minimum documentation to the extent that such documents are included in the fields searched

Electronic data base consulted during the international search (name of data base and, where practicable, search terms used)

CNTXT; CNABS; DWPI; SIPOABS; STNext caplus/reg; CNKI: 上海汇连生物; 张惠; 抗体偶联药物; 聚肌氨酸; 结构检索, structure search; ADC; antibody drug conjugate; Polysarcosine; Her2

**C. DOCUMENTS CONSIDERED TO BE RELEVANT**

| Category* | Citation of document, with indication, where appropriate, of the relevant passages | Relevant to claim No. |
|---|---|---|
| X | WO 2020236841 A2 (NOVARTIS AG) 26 November 2020 (2020-11-26) claims 1-5 and 10-22; description, page 205, table 4A, page 9, paragraph 3, page 150, last paragraph, and page 225; | 1-43 |
| Y | WO 2020236841 A2 (NOVARTIS AG) 26 November 2020 (2020-11-26) claims 1-5 and 10-22; description, page 205, table 4A, page 9, paragraph 3, page 150, last paragraph, and page 225; | 1-43 |
| Y | WARREN, V. et al. "Monodisperse polysarcosine-based highly-loaded antibody-drug conjugates;" *Chemical Science;*, Vol. 10, No. 14, 06 March 2019 (2019-03-06), abstract, figure 1, and page 4050, left-hand column, paragraph 4; pages 4048-4053 | 1-43 |
| Y | CONILH, L.e et al. "Exatecan Antibody Drug Conjugates Based on a Hydrophilic Polysarcosine Drug-Linker Platform;" *Pharmaceuticals;*, Vol. 14, No. 3, 09 March 2021 (2021-03-09), abstract, and figure 1; pages 1-17 | 1-43 |
| X | WO 2020233174 A1 (MABPLEX INTERNATIONAL LTD.) 26 November 2020 (2020-11-26) claims 1-9 | 1-8, 12, 20-43 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2022** | **28 June 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2022/089725

C. DOCUMENTS CONSIDERED TO BE RELEVANT

| Category* | Citation of document, with indication, where appropriate, of the relevant passages | Relevant to claim No. |
|---|---|---|
| A | CN 108066772 A (SHANGHAI INSTITUTE OF MATERIA MEDICA, CHINESE ACADEMY OF SCIENCES) 25 May 2018 (2018-05-25) entire document | 1-43 |
| A | CN 111542344 A (MABLINK BIOSCIENCE) 14 August 2020 (2020-08-14) entire document | 1-43 |
| A | CN 105102455 A (DONG-A ST CO., LTD. et al.) 25 November 2015 (2015-11-25) entire document | 1-43 |

Form PCT/ISA/210 (second sheet) (January 2015)

**INTERNATIONAL SEARCH REPORT**

International application No.
**PCT/CN2022/089725**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2022/089725**

| Patent document cited in search report | | | Publication date (day/month/year) | Patent family member(s) | | | Publication date (day/month/year) |
|---|---|---|---|---|---|---|---|
| WO | 2020236841 | A2 | 26 November 2020 | IL | 287596 | D0 | 01 December 2021 |
| | | | | KR | 20220010527 | A | 25 January 2022 |
| | | | | AU | 2020279731 | A1 | 06 January 2022 |
| | | | | CA | 3140063 | A1 | 26 November 2020 |
| | | | | CN | 113853219 | A | 28 December 2021 |
| | | | | EP | 3972650 | A2 | 30 March 2022 |
| WO | 2020233174 | A1 | 26 November 2020 | US | 2021085799 | A1 | 25 March 2021 |
| | | | | AU | 2020204250 | A1 | 10 December 2020 |
| | | | | RU | 2745738 | C1 | 31 March 2021 |
| | | | | JP | 2022512519 | A | 07 February 2022 |
| | | | | EP | 3760233 | A1 | 06 January 2021 |
| | | | | KR | 20200135284 | A | 02 December 2020 |
| CN | 108066772 | A | 25 May 2018 | WO | 2018086239 | A1 | 17 May 2018 |
| CN | 111542344 | A | 14 August 2020 | EP | 3700577 | A1 | 02 September 2020 |
| | | | | US | 2020345863 | A1 | 05 November 2020 |
| | | | | JP | 2021500412 | A | 07 January 2021 |
| | | | | WO | 2019081455 | A1 | 02 May 2019 |
| CN | 105102455 | A | 25 November 2015 | SG | 11201504887 T | A | 30 July 2015 |
| | | | | ES | 2714951 | T3 | 30 May 2019 |
| | | | | US | 2021113710 | A1 | 22 April 2021 |
| | | | | SG | 10201705150 R | A | 28 July 2017 |
| | | | | US | 2018264131 | A1 | 20 September 2018 |
| | | | | ZA | 201504941 | B | 29 November 2017 |
| | | | | MY | 169117 | A | 18 February 2019 |
| | | | | US | 2014193437 | A1 | 10 July 2014 |
| | | | | KR | 20210132742 | A | 04 November 2021 |
| | | | | CN | 108546283 | A | 18 September 2018 |
| | | | | PH | 12015501422 | B1 | 21 September 2015 |
| | | | | RU | 2015129800 | A | 30 January 2017 |
| | | | | CA | 2896690 | A1 | 26 June 2014 |
| | | | | AR | 094280 | A1 | 22 July 2015 |
| | | | | TW | 201427694 | A | 16 July 2014 |
| | | | | US | 2015352222 | A1 | 10 December 2015 |
| | | | | BR | 112015014712 | A2 | 11 July 2017 |
| | | | | JP | 2016508136 | A | 17 March 2016 |
| | | | | MX | 2015007918 | A | 21 June 2016 |
| | | | | HK | 1216640 | A1 | 25 November 2016 |
| | | | | KR | 20150119848 | A | 26 October 2015 |
| | | | | AU | 2013364065 | A1 | 23 July 2015 |
| | | | | US | 2016015830 | A1 | 21 January 2016 |
| | | | | RU | 2019105549 | A | 28 March 2019 |
| | | | | IL | 239506 | D0 | 31 August 2015 |
| | | | | DK | 2935259 | T3 | 18 March 2019 |
| | | | | NZ | 630601 | A | 26 May 2017 |
| | | | | PT | 2935259 | T | 04 April 2019 |
| | | | | WO | 2014100762 | A1 | 26 June 2014 |
| | | | | EP | 2935259 | A1 | 28 October 2015 |
| | | | | CL | 2015001735 | A1 | 19 August 2016 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020233174 A1
- WO 2017042210 A1
- WO 2014172371 A2
- US 20060275305 A1
- WO 2003074566 A
- CN 102174105 B
- WO 2018212136 A
- US 20200171163 A1
- WO 2016038383 A

### Non-patent literature cited in the description

- *CHEMICAL ABSTRACTS,* 7689-03-4
- *CHEMICAL ABSTRACTS,* 171335-80-1
- *CHEMICAL ABSTRACTS,* 256411-32-2
- *CHEMICAL ABSTRACTS,* 529488-28-6
- *Protein Science,* 1995, vol. 4, 2411-2423